(19) Europäisches Patentamt
European Patent Office
Office européen des brevets

(11) Numéro de publication: **0 499 521 B1**

(12) # FASCICULE DE BREVET EUROPEEN

(45) Date de publication de fascicule du brevet: **14.06.95**

(51) Int. Cl.[6]: **C07D 207/16**, C07D 233/54, C07D 471/04, C07C 233/47, C07C 235/06, C07F 9/38, C07F 9/40, C07F 9/30

(21) Numéro de dépôt: **92400341.1**

(22) Date de dépôt: **11.02.92**

Le dossier contient des informations techniques présentées postérieurement au dépôt de la demande et ne figurant pas dans le présent fascicule.

(54) **Nouveaux inhibiteurs de N-myristoyltransférase, leur procédé de préparation et les compositions pharmaceutiques qui les contiennent.**

(30) Priorité: **11.02.91 FR 9101502**

(43) Date de publication de la demande:
**19.08.92 Bulletin 92/34**

(45) Mention de la délivrance du brevet:
**14.06.95 Bulletin 95/24**

(84) Etats contractants désignés:
**AT BE CH DE DK ES FR GB GR IT LI LU NL PT SE**

(56) Documents cités:
**EP-A- 0 039 804**
**EP-A- 0 201 742**
**EP-A- 0 201 743**
**EP-A- 0 275 522**
**EP-A- 0 339 498**
**EP-A- 0 348 942**
**EP-A- 0 351 382**
**CA-A- 1 261 855**

**PATENT ABSTRACTS OF JAPAN vol. 12, no. 410 (C-540)(3257) 28 Octobre 1988**

(73) Titulaire: **ADIR ET COMPAGNIE**
**1 rue Carle Hébert**
**F-92415 Courbevoie Cédex (FR)**

(72) Inventeur: **Vincent, Michel**
**8 allée du Prunier Hardy**
**F-92220 Bagneux (FR)**
Inventeur: **Remond, Georges**
**9 avenue des Etats-Unis**
**F-78000 Versailles (FR)**
Inventeur: **Portevin, Bernard**
**6 rue Frédéric Passy**
**F-78990 Elancourt (FR)**
Inventeur: **Boutin, Jean-Albert**
**22 esplanade des Courtieux**
**F-92150 Suresnes (FR)**
Inventeur: **Atassi, Ghanem**
**4 rue Joséphine**
**F-92400 Saint-Cloud (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention a pour objet de nouveaux inhibiteurs de N-myristoyltransférase, leur procédé de préparation et les compositions pharmaceutiques qui les contiennent.

Il est connu de l'art antérieur, que le groupement amino N-terminal des protéines est bloqué par des groupements acétyle, pyroglutamyle et formyle. Or, SHOJI et Coll. ont mis en évidence que l'acide myristique était lié par une liaison covalente au groupement N-terminal des sous-unités catalytiques de la protéine kinase AMP cyclique dépendante (Proc. Natl. Acad. Sci. USA, (1982), 79, 6123-6131).

L'existence de ce groupement myristoyl terminal a depuis lors été montré dans diverses autres protéines comme la calcineurine B (AITKEN et Coll, Febs Letters, (1982), 150, n° 2, 314-318) ou la tyrosine protéine kinase (TPK) (BUSS et SEFTON, J. Virol, (1985),53, 7-12).

Dans le domaine des oncogènes également, BISHOP a mis en évidence qu'une protéine transformante subissait une myristoylation lors de la maturation. Il a d'ailleurs été montré depuis que cette étape de maturation passant par une myristoylation était essentielle au pouvoir transformant de cette protéine (KAMPS, BUSS et SEFTON, Proc. Natl. Acad. Sci. USA, (1985), 82, 4625-4628). Ce concept a dès lors été généralisé à de nombreuses autres protéines transformantes d'origine virale (RHEE et HUNTER, J. Virol., (1987), 61, 1045-1053). Cette maturation est catalysée par une enzyme appelée N-myristoyltransférase (NMT) mise en évidence dans la levure par TOWLER et GLASER (Proc. Natl. Acad. Sci. USA, (1986), 83, 2812-2816).

Or, la NMT ne reconnait pratiquement comme cosubstrat que l'acide myristique d'une part et d'autre part, comme substrat, les protéines comportant une glycine comme dernier acide aminé du côté N-terminal, avec participation de la séquence peptidique jouxtant cette glycine (participation de 7 amino-acides).

Ainsi, la myristoylation du résidu glycine N-terminal de certaines protéines joue un rôle très important sur certains mécanismes intervenant dans la transformation des cellules et le contrôle de leur prolifération. Il a d'ailleurs été montré par SHOJI et Coll. (brevets japonais JP 63-146851, JP 62-255810 et JP 62-126384) que la myristoylglycine ou des dérivés oligopeptidiques possédait un effet inhibiteur contre la transformation ou la prolifération cellulaire ou la multiplication de rétrovirus.

L'invention concerne plus spécifiquement les composés de formule (I)

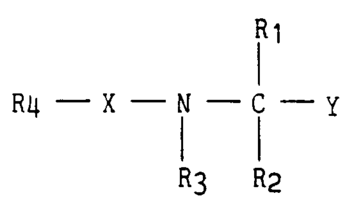

(I)

dans laquelle :

$R_1$ représente

- un atome d'hydrogène,
- un groupement alkyle ($C_1$-$C_6$) linéaire ou ramifié non substitué ou substitué par un ou plusieurs groupements hydroxy, amino, carboxy, carbamoyle, benzylthio, méthylthio, mercapto ou phényle (non substitué ou substitué par un ou plusieurs atomes d'halogène ou groupements hydroxy, alkyle ($C_1$-$C_6$) linéaire ou ramifié, alkoxy ($C_1$-$C_6$) linéaire ou ramifié ou $(CH_3\text{-}CH_2\text{-}O)_2PO\text{-}CH_2\text{-}$),
- un groupement phényle non substitué ou substitué par un ou plusieurs atomes d'halogène ou groupements hydroxy ou alkyle ($C_1$-$C_6$) linéaire ou ramifié,
- un groupement cycloalkyle ($C_3$-$C_7$) méthyle,
- un groupement (imidazol-2-yl) méthyle ou un groupement (indol-3-yl) méthyle non substitué ou substitué sur l'hétérocycle par un groupement benzyle, benzhydryle, trityle, benzyloxyméthyle, tosyle, alkyle ($C_1$-$C_6$) linéaire ou ramifié ou phényle,
- un groupement (1-azaindolizin-2-yl) méthyle de formule :

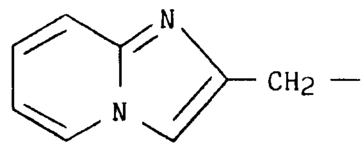

$R_2$ représente un atome d'hydrogène ou un groupement alkyle ($C_1$-$C_6$) linéaire ou ramifié,

$R_3$ représente un atome d'hydrogène ou un groupement alkyle ($C_1$-$C_6$) linéaire ou ramifié,

ou bien

lorsque $R_1$ représente un atome d'hydrogène,

$R_2$ et $R_3$ peuvent former avec les atomes de carbone et d'azote auxquels ils sont respectivement attachés, l'un quelconque des hétérocyles suivants :

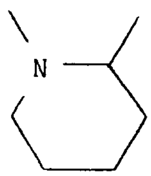 , 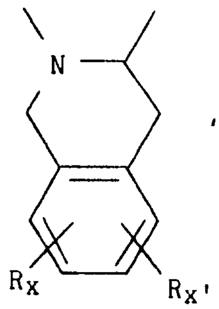 , 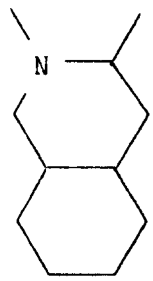 , 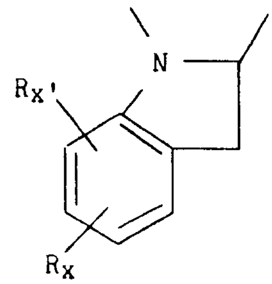 ,

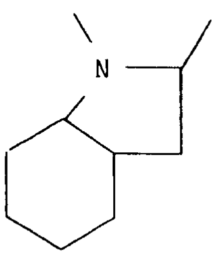 , 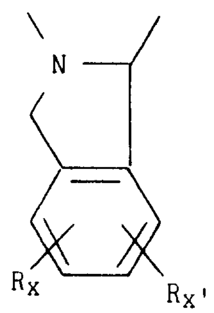 , 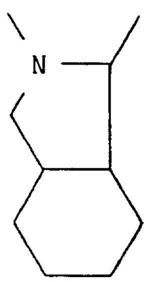 , 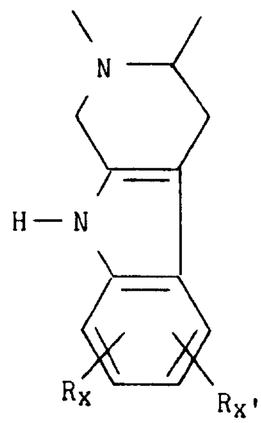

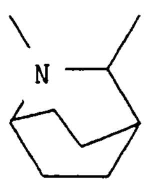 , 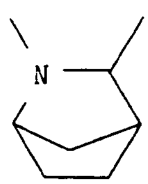 , 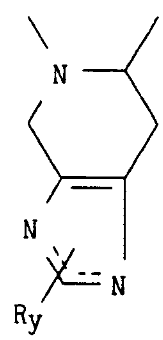

$R_x$ et $R_{x'}$ identiques ou différents représentent un atome d'hydrogène, un groupement alkyle ($C_1$-$C_6$) linéaire ou ramifié, un groupement hydroxy, un groupement alkoxy ($C_1$-$C_6$) linéaire ou ramifié, un atome d'halogène,

ou bien $R_x$ et $R_{x'}$, lorsqu'ils sont situés sur deux carbones adjacents, forment un

groupement méthylènedioxy ou éthylènedioxy,

Ry représente un atome d'hydrogène, un groupement alkyle ($C_1$-$C_6$) linéaire ou ramifié, un groupement aryle, un groupement aralkyle, un groupement aroyle, un groupement arylsulfonyl,

X représente l'un quelconque des groupements suivants :

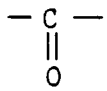 , $— SO_2 —$ , 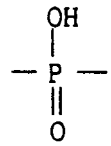 ,

Y représente un groupement — CO — $R_5$ ou

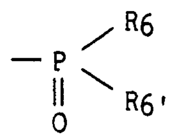

$R_5$ représente un groupement hydroxy, alkoxy ($C_1$-$C_6$) linéaire ou ramifié, $H_2N$-CO-$CH_2$-O-, HO-$CH_2$-CHOH-$CH_2$-O-,

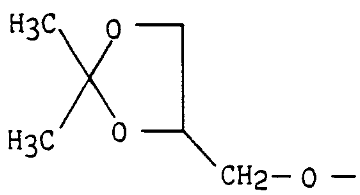 , 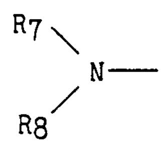 ,

$R_7$ et $R_8$ identiques ou différents représentent un atome d'hydrogène, un groupement alkyle ($C_1$-$C_6$) linéaire ou ramifié ou forment avec l'atome d'azote auxquels ils sont attachés un cycle pyrrolidine, pipéridine, morpholine ou pipérazine.

$R_6$ et $R_{6'}$ identiques ou différents représentent un atome d'hydrogène, un groupement hydroxy ou alkoxy ($C_1$-$C_6$) linéaire ou ramifié,

$R_4$ représente :

1 un groupement alkyle linéaire ou ramifié comportant de 6 à 21 atomes de carbone non substitué ou substitué sur le groupement méthyle terminal par un groupement hydroxy, mercapto, phényle ou éthynyle et dans lequel l'un au moins des groupements méthylènes est remplacé par un atome d'oxygène, de soufre ou un noyau p-phénylène,

dans le cas où :

$R_1$ représente

- un atome d'hydrogène,
- un groupement alkyle ($C_1$-$C_6$) linéaire ou ramifié non substitué ou substitué par un ou plusieurs groupements hydroxy, amino, carboxy, carbamoyle, benzylthio, méthylthio, mercapto ou phényle (non substitué ou substitué par un groupement hydroxy ou alkoxy ($C_1$-$C_6$) linéaire ou ramifié),
- un groupement phényle non substitué,
- un groupement (imidazol-2-yl) méthyle ou un groupement (indol-3-yl) méthyle non substitué ou substitué sur l'hétérocycle par un groupement méthyle

  et

  $R_2$ = H, $R_3$ = H,

X = 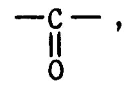

$Y = CO\ R_{5'}$ ($R_{5'}$ = OH, alkoxy)

2 Dans les autres cas :

un groupement alkyle linéaire ou ramifié comportant de 6 à 21 atomes de carbone non substitué ou substitué sur le groupement méthyle terminal par un groupement hydroxy, mercapto, phényle ou éthynyle et dans lequel un ou plusieurs groupements méthylènes peuvent être remplacés par un atome d'oxygène, de soufre ou un noyau p-phénylène, (à la condition que lorsque $R_2$ ou $R_3$ forment avec les atomes d'azote ou de carbone auxquels ils sont attachés un cycle 1,2,3,4-tétrahydroisoquinoléine, indoline ou isoindoline alors $R_4$ est différent du groupement : alk-S-($CH_2$)n- dans lequel : n est égal à 1,2,3,4 ou 5 et alk représente un groupement alkyle ($C_4$-$C_{19}$) linéaire ou ramifié),

leurs isomères, diastéréoisomères et épimères ainsi que leurs sels d'addition à un acide ou à une base pharmaceutiquement acceptable.

Parmi les acides pharmaceutiquement acceptables, on peut citer à titre non limitatif les acides chlorhydrique, sulfurique, tartrique, maléique, fumarique, oxalique, méthane sulfonique, camphorique, etc . . .

Parmi les bases pharmaceutiquement acceptables, on peut citer à titre non limitatif l'hydroxyde de sodium, l'hydroxyde de potassium, la terbutylamine. . .

L'invention s'étend aussi au procédé de préparation des composés de formule (I) caractérisé :

1/ dans le cas où les dérivés de formule (I) que l'on souhaite obtenir possèdent un radical $R_4 = R_{4'}$ dans lequel aucun groupement méthylène n'est remplacé par un atome de soufre ou d'oxygène,

en ce que l'on condense un composé de formule (II) :

$R_{4'}$ - X' - Z (II)

dans laquelle :

X' représente un groupement

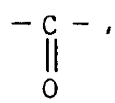

$-SO_2-$ ,

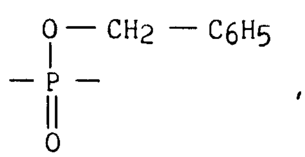

$R_{4'}$ représente un groupement alkyle linéaire ou ramifié comportant de 6 à 21 atomes de carbone, non substitué ou substitué sur le groupement méthyle terminal par un groupement hydroxy, mercapto, phényle ou éthynyle, et dans lequel un ou plusieurs groupements méthylène peuvent être remplacés par un groupement p.phénylène,

Z représente un atome d'halogène, un groupement hydroxy, un groupement alkoxy ($C_1$-$C_6$) linéaire ou ramifié, un groupement aralkoxy,

ou un groupement

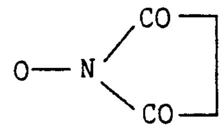 ,

sur une amine de formule (III) (sous forme racémique ou isomérique) protégée le cas échéant :

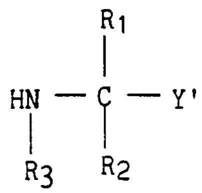 (III)

dans laquelle $R_2$, $R_3$ et $R_4$ ont la même signification que dans la formule I et Y' représente un groupement $-CO_2H$, -CO(alkoxy) ou $-PO(alkoxy)_2$,
pour conduire après déprotection éventuelle à un composé de formule (I/a), cas particulier des composés de formule (I)

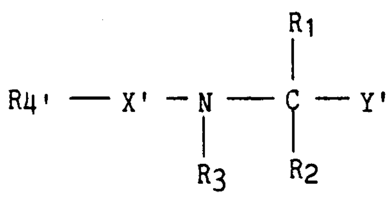 (I/a)

dans laquelle $R_1$, $R_2$, $R_3$, $R_{4'}$, X' et Y' ont la même signification que précédemment,
2/ dans le cas où les dérivés de formule (I) que l'on souhaite obtenir possèdent un radical $R_4 = R_{4''} = CH_3-(CH_2)n-$ dans lequel l'un au moins des groupements méthylène est remplacé par un atome de soufre ou d'oxygène,
en ce que l'on condense :
un composé de formule (IV)

$A - (CH_2)_m — X' — Z$ (IV)

dans laquelle :

X' et Z ont la même signification que précédemment,
A représente un atome d'halogène, un groupement mésyloxy ou un groupement tosyloxy,
m est inférieur ou égal à n-1,

et l'un des groupements méthylène peut être remplacé par un atome d'oxygène, de soufre, un groupement p.phénylène, un groupement $-CH(CH_3)-$ou un groupement $-C(CH_3)_2-$,
sur une amine de formule (III) (sous forme racémique ou isomérique) protégée le cas échéant telle que définie précédemment,
pour conduire à un composé de formule (V)

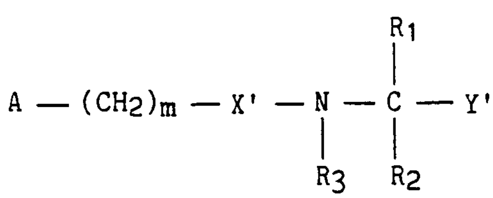 (V)

dans laquelle A, m, X', $R_1$, $R_2$, $R_3$ et Y' sont définis comme précédemment,
qui est mis en réaction avec un dérivé de formule (VI) :

$R_9 — B — M$ (VI)

dans laquelle :

- $R_9$ représente un groupement $CH_3$-$(CH_2)_p$- non substitué ou substitué sur le groupement méthyle terminal par un groupement hydroxy, mercapto, phényle ou éthynyle et dans lequel l'un des groupements méthylène peut être remplacé par un atome d'oxygène, de soufre, un groupement p.phénylène, un groupement -$CH(CH_3)$- ou un groupement -$C(CH_3)_2$-,
- B représente un atome d'oxygène ou de soufre,
- M représente un métal choisi parmi sodium, potassium ou césium,
- p est tel que la somme m + p est inférieure ou égale à n-1,

pour conduire, après déprotection éventuelle à un composé de formule (I/b), cas particulier des composés de formule (I) :

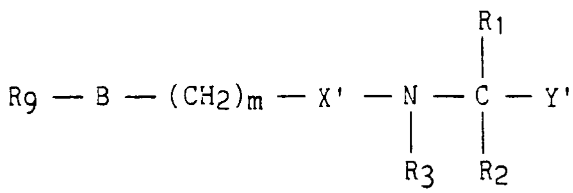

(I/b)

dans laquelle $R_9$, B, m, X', $R_1$, $R_2$, $R_3$ et Y' sont définis comme précédemment,
dérivés de formule (I/a) et (I/b), que l'on peut écrire de manière simplifié :

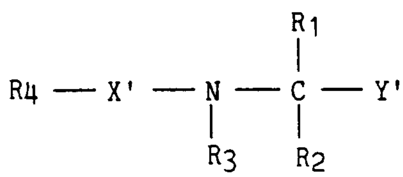

qui

a dans le cas où X' représente

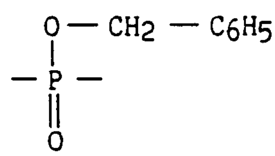

peuvent être transformés par hydrogénation catalytique, en composés de formule (I/c), cas particulier des composés de formule (I) :

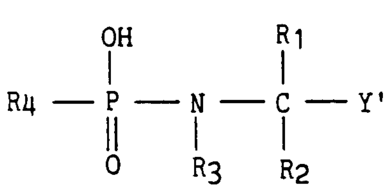

(I/c)

dans laquelle $R_1$, $R_2$, $R_3$, $R_4$ et Y' ont la même signification que précédemment,

b dans le cas où Y' représente un groupement -CO(alkoxy) ou -PO-$(alkoxy)_2$ peuvent être saponifiés de manière totale ou partielle pour conduire respectivement aux composés de formules (I/d), (I/e) et (I/f), cas particulier des composés de formule (I) :

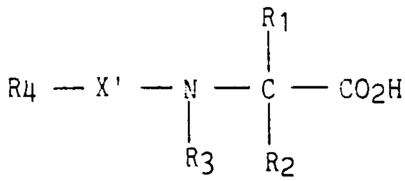

(I/d)

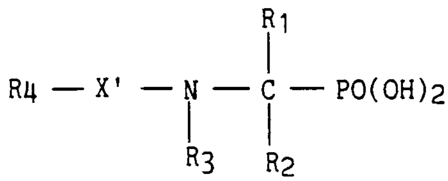

(I/e)

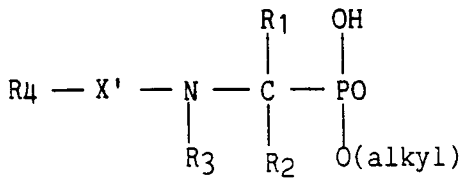

(I/f)

dans lesquelles $R_1$, $R_2$, $R_3$, $R_4$ et X' ont la même signification que précédemment,

c dans le cas où Y' représente un groupement $-CO_2H$ peuvent être transformés, en présence de carbonate de césium à l'aide de chloroacétamide, en composé de formule (I/g), cas particulier des composés de formule (I) :

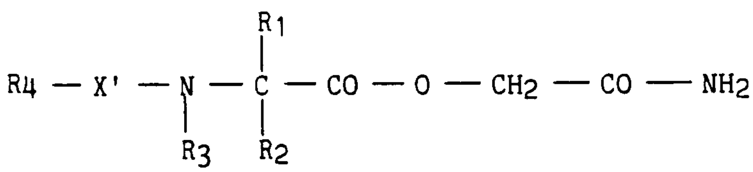

(I/g)

dans laquelle $R_1$, $R_2$, $R_3$, $R_4$ et X' ont la même signification que précédemment,

d dans le cas où Y' représente un groupement $-CO_2H$, peuvent être estérifiés à l'aide d'isopropylidène glycérol, en composé de formule (I/h), cas particulier des composés de formule (I),

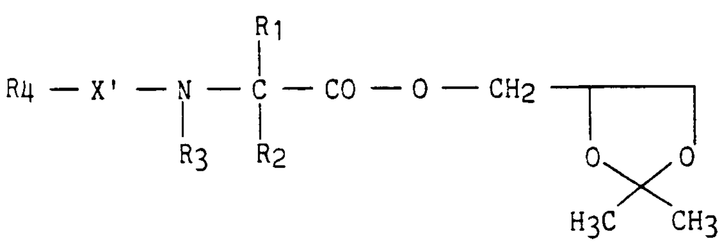

(I/h)

dans laquelle $R_1$, $R_2$, $R_3$ et $R_4$ ont la même signification que précédemment,

dérivé de formule (I/h) qui peut être hydrolysé en milieu acide pour conduire au composé de formule (I/i), cas particulier des composés de formule (I)

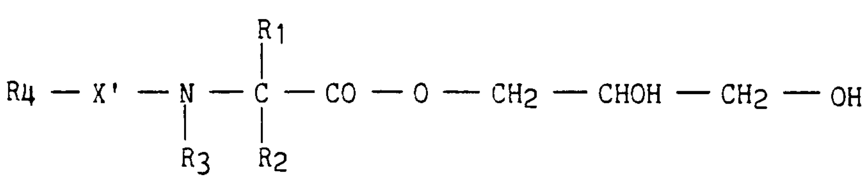

(I/i)

dans laquelle $R_1$, $R_2$, $R_3$ et $R_4$ ont la même signification que précédemment,
composés de formules (I/a) à (I/i) qui peuvent être purifiés, le cas échéant, par une technique classique de purification, dont les isomères peuvent être séparés selon une technique classique de séparation, et que l'on peut transformer en leurs sels d'addition à un acide ou à une base pharmaceutiquement acceptable.

Les composés de formule (I), outre le fait qu'ils soient nouveaux possèdent des propriétés pharmacologiques très intéressantes.

Ce sont de puissants inhibiteurs de la myristoylation de protéines telles que gag par le biais de l'enzyme responsable de cette myristoylation, c'est-à-dire la N-myristoyltransférase (NMT).

Or la NMT se trouve localisée dans de nombreuses sources biologiques ; elle peut être soit d'origine cytosolique soit d'origine microsomale comme l'ont montré J.A. BOUTIN et coll. (BIOCHEMICAL Journal, 1990, soumis). L'enzyme microsomale reconnait de nombreuses protéines endogènes, produits d'oncogènes ou protéines structurales de virus. L'enzyme cytosolique, quant à elle, reconnait plus difficilement les protéines endogènes.

Or les composés de l'invention sont non seulement reconnus par l'enzyme microsomale mais également par l'enzyme cytosolique. Ils inhibent à la fois et de manière surprenante les activités microsomales et cytosoliques.

L'utilisation des composés de l'invention en tant qu'inhibiteurs de NMT conduit à une inhibition de l'activité de cette enzyme nettement supérieure à celle de composés décrits dans l'art antérieur comme inhibiteurs de la prolifération de cellules cancéreuses et de rétrovirus.

En effet, une étude approfondie de l'influence excercée par les composés de l'invention sur la prolifération et la transformation cellulaire a été réalisée à partir de cellules cancéreuses d'origine murine (L1210) ou d'origine humaine (HL60). Après extraction de l'enzyme de ce milieu biologique et mesure de son activité, il apparait que l'addition de composés de l'invention inhibe fortement son activité.

De plus, les composés de l'invention présentent une activité cytoxique sur des cellules cancéreuses en culture telles que L1210 (d'origine murine) ou HL60 (d'origine humaine). Cette cytotoxicité s'est révélée être nettement supérieure à celle due à la N-myristoylglycine sur ces cellules.

D'autre part, les composés de l'invention protègent, par le biais de cette inhibition d'activité de la NMT, des cellules de lymphocytes T humains (CEM) en culture de l'infection par le virus HIV-1.

Ainsi, cette inhibition d'activité est d'autant plus intéressante que cette enzyme joue un rôle prépondérant, en particulier dans la maturation soit de protéines transformantes impliqués dans certains cancers, soit de protéines elles-mêmes impliquées dans la maturation de virus.

Les composés de l'invention ont donc potentiellement des applications dans le traitement du cancer et/ou de maladies virales dont la maturation implique une myristoylation comme le SIDA, l'herpes, l'hépatite B, l'influenza, la polyomyélite ou les leucémies.

La présente invention a également pour objet les compositions pharmaceutiques renfermant comme principe actif un composé de formule générale (I) ou un de ses sels d'addition à une base pharmaceutiquement acceptable, seul ou en combinaison avec un ou plusieurs excipients ou véhicules inertes non toxiques.

Parmi les compositions pharmaceutiques selon l'invention, on pourra citer celles qui conviennent pour l'administration orale, parentérale, nasale, les comprimés simples ou dragéifiés, les comprimés sublinguaux, les sachets, les paquets, les gélules, les glossettes, les tablettes, les suppositoires, etc. . .

La posologie varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,1 et 100 mg pour un traitement en 1 à 3 prises par 24 heures.

Les exemples suivants illustrent l'invention mais ne la limitent en aucune façon.

Les positions des substituants de l'histidine et de la spinacine des exemples sont notées de la manière suivante :

(N'τ-R)histidine

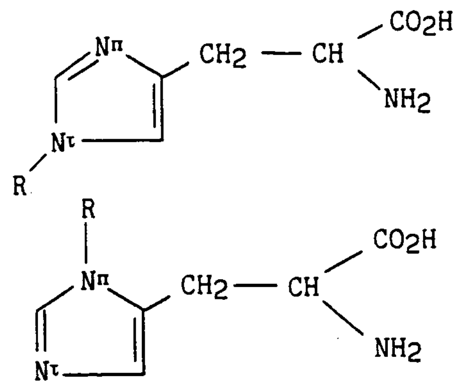

(N'π-R)histidine

(N'τ-R)spinacine

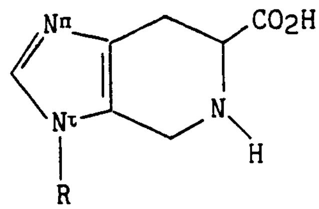

(N'π-R)spinacine

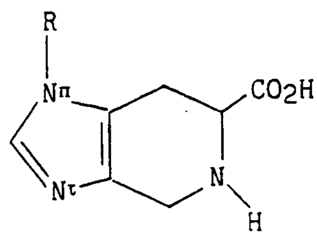

La préparation indiquée ci-dessous ne permet pas d'obtenir les composés de l'invention. En revanche, elle conduit à un produit de départ utile dans la synthèse de produits de l'invention.

Préparation A : (Imidazo[1,2-a]pyridin-2-yl)alanine

STADE 1 : 2-Hydroxyméthylimidazo[1,2-a]pyridine

Dans un ballon sous atmosphère d'azote, on introduit 200 ml de tétrahydrofurane, 120 mmoles d'hydrure de lithium-aluminium puis en 40 minutes, sous agitation, 120 mmoles de 2-carbethoxyimidazo-[1,2-a]pyridine préparé selon la méthode décrite par J.G. LOMBARDINO (J. Org. Chem., 30, 2403-2407, 1965) en solution dans 150 ml de tétrahydrofurane. L'agitation est maintenue 20 heures à température ambiante. Le mélange est hydrolysé par 50 ml d'isopropanol puis par 50 ml d'une solution saturée de chlorure de sodium. Après évaporation à sec, le produit attendu est obtenu après purification sur colonne de silice (solvant d'élution : dichlorométhane/méthanol : 95/5).
Rendement : 31 %

STADE 2 : Chlorhydrate de 2-chlorométhylimidazo[1,2-a]pyridine

Dans un ballon, on introduit 40 ml de chlorure de thionyle puis, par fractions, 34 mmoles du produit obtenu au stade précédent. L'ensemble est porté à reflux 20 minutes puis évaporé à sec. Le résidu est repris par 100 ml de toluène et évaporé, puis à nouveau repris par 50 ml d'isopropanol. Le précipité est alors filtré puis lavé par du dichlorométhane.
Rendement : 61 %

STADE 3 : (Imidazo[1,2-a[pyridin-2-yl)alanine

Dans un ballon, on introduit 100 ml d'éthanol puis, par fractions, 0,056 at.g. de sodium. Quand le sodium a réagi, 8,05 g d'acétamidomalonate d'éthyle sont ajoutés et l'ensemble est agité en laissant revenir la température à 20 °C. On ajoute alors, en 10 minutes, par fractions, 19 mmoles du produit obtenu au

stade précédent. L'agitation est maintenue 18 heures à température ambiante. Après évaporation de l'éthanol, 200 ml d'acide chlorhydrique N sont ajoutés. Cette phase aqueuse est lavée par de l'acétate d'éthyle puis alcalinisée par du carbonate de sodium et extraite par de l'acétate d'éthyle. La phase organique est alors lavée à l'eau puis évaporée. Le produit attendu, obtenu sous forme de base, est hydrolysé sous forme de chlorhydrate par reflux pendant 6 heures dans de l'acide chlorhydrique 6N, évaporation à sec, reprise par de l'eau et fixation sur résine. Il est élué par de l'ammoniaque à 10 % qui est alors évaporé.
Rendement : 74 %

**Exemple 1 : N-Myristoyl-3-(S)-carboxy-(1,2,3,4)-tétrahydroisoquinoléine**

Stade 1 : Myristate de N-hydroxysuccinimide

60 mmoles de N-hydroxysuccinimide sont dissoutes sous agitation dans 200 ml d'acétate d'éthyle. On ajoute alors une solution de 60 mmoles d'acide myristique dans 100 ml d'acétate d'éthyle puis 60 mmoles de cyclohexylcarbodiimine. Après 20 heures d'agitation à température ambiante, la dicyclohexylurée formée est filtrée et le filtrat est évaporé. Le produit attendu est obtenu après recristallisation dans l'éthanol.
Rendement : 75 %
Point de fusion : 83 °C

Stade 2 : N-Myristoyl-3-(S)-carboxy-(1,2,3,4)-tétrahydroisoquinoléine

En utilisant la méthode décrite par Y. LAPIDOT et S. RAPPOPORT (J. of Lipid. Research, 8, 142-145, 1967), on obtient à partir du produit décrit au stade précédent et de la 3-(S)-carboxy-(1,2,3,4)-tétrahydroisoquinoléine, le produit attendu qui est purifié par chromatographie sur gel de silice (solvant d'élution : $CH_2Cl_2$/MeOH : 95/5).
Rendement : 50 %
Infrarouge (nujol) : $\nu_{OH}$ entre 3000 et 2300 $cm^{-1}$
$\nu_{CO}$ acide : 1720 $cm^{-1}$
$\nu_{CO}$ amide : 1640 $cm^{-1}$

| Microanalyse élémentaire : | | | |
|---|---|---|---|
| | C % | H % | N % |
| calculé | 74,38 | 9,62 | 3,61 |
| trouvé | 74,47 | 9,72 | 3,61 |

Les exemples 2 à 14, 17, 18, 20, 22, 28 à 34, 56 à 59 ont été synthétisés à partir de matières premières décrites dans la littérature selon le même mode opératoire que celui décrit dans l'exemple 1.

**Exemple 2 : N-Myristoyl-3-(S)-carboxy-2-azabicyclo [2.2.2] octane**

Rendement : 16 %
Infrarouge (nujol) : $\nu_{OH}$ entre 3700 et 2000 $cm^{-1}$
$\nu_{CO}$ acide : 1740 $cm^{-1}$
$\nu_{CO}$ amide : 1650 $cm^{-1}$

**Exemple 3 : N-Myristoyl-2-carboxy-(2S, 3aS, 7aS)-perhydroindole**

Rendement : 44 %
Infrarouge (nujol) : $\nu_{OH}$ entre 3700 et 2250 $cm^{-1}$
$\nu_{CO}$ acide : 1740 $cm^{-1}$
$\nu_{CO}$ amide I : 1640 $cm^{-1}$
$\nu_{CO}$ amide II : 1600 $cm^{-1}$

**Exemple 4 : N-Myristoyl-1-carboxyisoindoline**

Rendement : 54 %
Infrarouge (nujol) : $\nu_{OH}$ entre 3500 et 2200 $cm^{-1}$
$\nu_{CO}$ acide : 1743 $cm^{-1}$
$\nu_{CO}$ amide : 1603 $cm^{-1}$

| Microanalyse élémentaire : | | | |
|---|---|---|---|
| | C % | H % | N % |
| calculé | 73,96 | 9,44 | 3,75 |
| trouvé | 73,60 | 9,43 | 3,76 |

**Exemple 5 : N-Myristoyl-($N^{\prime\prime\tau}$-benzyl)-(S)-histidine**

Rendement : 28 %
Infrarouge (nujol) : $\nu_{NH}$ : 3300 $cm^{-1}$
$\nu_{OH}$ entre 3200 et 1800 $cm^{-1}$
$\nu_{CO}$ acide : 1700 $cm^{-1}$
$\nu_{CO}$ amide : 1640 $cm^{-1}$

| Microanalyse élémentaire : | | | |
|---|---|---|---|
| | C % | H % | N % |
| calculé | 71,17 | 9,07 | 9,22 |
| trouvé | 70,74 | 9,35 | 9,25 |

**Exemple 6 : N-Myristoyl-($N^{\prime\prime\tau}$-tosyl)-(S)-histidine**

Rendement : 55 %
Infrarouge (nujol) : $\nu_{NH}$ entre 3600 et 2400 $cm^{-1}$
$\nu_{CO}$ carboxylate et amide ≃ 1640 $cm^{-1}$

**Exemple 7 : N-Myristoyl-($N^{\prime\prime\tau}$-trityl)-(S)-histidine**

Rendement : 65 %
Infrarouge (nujol) : $\nu_{OH}$ et $\nu_{NH}$ : vers 3300 $cm^{-1}$
$\nu_{CO}$ amide : 1651 $cm^{-1}$

**Exemple 8 : N-Myristoyl-($N^{\prime\pi}$-bensyloxyméthyl)-(S)-histidine**

Rendement : 49 %
Infrarouge (nujol) : $\nu_{NH}$ : 3317 $cm^{-1}$
$\nu_{OH}$ entre 2500 et 2000 $cm^{-1}$
$\nu_{CO}$ acide : 1714 $cm^{-1}$
$\nu_{CO}$ amide : 1645 $cm^{-1}$
Point de fusion : 128 °C

| Microanalyse élémentaire : | | | |
|---|---|---|---|
| | C % | H % | N % |
| calculé<br>trouvé | 69,25<br>68,72 | 8,92<br>8,88 | 8,65<br>8,53 |

**Exemple 9 : N-Myristoyl-(4-hydroxy-3,5-diterbutylphényl)alanine**

Rendement : 36 %
Infrarouge (nujol) : $\nu_{OH}$ phénol : 3630 $cm^{-1}$
$\nu_{OH}$ entre 3600 et 2300 $cm^{-1}$
$\nu_{CO}$ acide : 1730 $cm^{-1}$

**Exemple 10 : N-Myristoyl-α-éthylphénylglycine**

Rendement : 31 %
Point de fusion : 128 °C
Infrarouge (nujol) : $\nu_{NH}$ : 3400 $cm^{-1}$
$\nu_{OH}$ entre 3200 et 1800 $cm^{-1}$
$\nu_{CO}$ acide : 1690 $cm^{-1}$
$\nu_{CO}$ amide I : 1620 $cm^{-1}$
$\nu_{CO}$ amide II : 1520 $cm^{-1}$

| Microanalyse élémentaire : | | | |
|---|---|---|---|
| | C % | H % | N % |
| calculé<br>trouvé | 73,99<br>73,54 | 10,09<br>10,26 | 3,60<br>3,62 |

**Exemple 11 : N-Myristoyl-p.(diéthylphosphonométhyl)phénylalanine**

La p.(diéthylphosphonométhyl)phénylalanine utilisée est décrite par I. MARSEIGNE et B.P. ROQUES (J. Org. Chem., 53, 3621-3624, 1988).
Rendement : 11 %
Infrarouge (nujol) : $\nu_{NH}$ : 3600 $cm^{-1}$
$\nu_{OH}$ entre 3500 et 2000 $cm^{-1}$
$\nu_{CO}$ acide : 1720 $cm^{-1}$
$\nu_{CO}$ amide I : 1640 $cm^{-1}$
$\nu_{CO}$ amide II : 1520 $cm^{-1}$

| Microanalyse élémentaire : | | | |
|---|---|---|---|
| | C % | H % | N % |
| calculé<br>trouvé | 63,98<br>63,94 | 9,20<br>9,20 | 2,66<br>2,49 |

**Exemple 12 : N-Myristoyl-α-méthylphénylalanine**

Rendement : 62 %
Infrarouge : $\nu_{NH}$ : 3290 $cm^{-1}$
$\nu_{OH}$ entre 3300 et 2300 $cm^{-1}$
$\nu_{CO}$ acide : 1720 $cm^{-1}$

$\nu_{CO}$ amide : 1620 $cm^{-1}$

| Microanalyse élémentaire : | | | |
|---|---|---|---|
| | C % | H % | N % |
| calculé<br>trouvé | 73,99<br>73,87 | 10,09<br>10,18 | 3,60<br>3,42 |

**Exemple 13 : N-Myristoylspinacine**

Rendement : 22 %
Infrarouge (nujol) : $\nu_{OH}$ entre 3700 et 2300 $cm^{-1}$
$\nu_{CO}$ amide : 1614 $cm^{-1}$

**Exemple 14 : 1-Myristoylamino-2-phényléthanphosphonate de diéthyle**

Rendement : 51 %
Infrarouge (film liquide) :
$\nu_{NH}$ : 3260 $cm^{-1}$
$\nu_{CO}$ amide I ≃ 1670 $cm^{-1}$
$\nu_{CO}$ amide II : 1540 $cm^{-1}$

| Microanalyse élémentaire : | | | |
|---|---|---|---|
| | C % | H % | N % |
| calculé<br>trouvé | 64,21<br>63,99 | 9,31<br>9,12 | 3,40<br>3,36 |

**Exemple 15 : Acide 1-myristoylamino-2-phényléthanephosphonique**

Le produit attendu est obtenu par saponification totale de 37 mmoles du composé décrit dans l'exemple 14 en présence de 7,5 ml d'acide acétique et de 1,85 ml d'acide bromhydrique à 48 %, évaporation et recristallisation dans un mélange acétone/eau (3/1).
Rendement : 76 %
Point de fusion : 108°C
Infrarouge (nujol) : $\nu_{NH}$ : 3600 $cm^{-1}$
$\nu_{OH}$ entre 3400 et 2000 $cm^{-1}$

| Microanalyse élémentaire : | | | |
|---|---|---|---|
| | C % | H % | N % |
| calculé<br>trouvé | 64,21<br>63,99 | 9,31<br>9,84 | 3,40<br>3,36 |

**Exemple 16 : 1-Myristoylamino-2-phényléthanehydrogénophosphonate d'éthyle**

Le produit attendu est obtenu par saponification partielle de 11 mmoles du composé décrit dans l'exemple 14 en présence de 10 ml d'éthanol et de 1,1 ml de potasse 1N, et après 72 heures de reflux. L'éthanol est évaporé et 50 ml d'eau sont ajoutés. La phase aqueuse est lavée par du pentane, acidifiée à pH = 1 par de l'acide chlorhydrique concentré. Le produit est alors extrait par de l'acétate d'éthyle et purifié par chromatographie sur colonne de silice (éluant : dichlorométhane/méthanol : 80/20).
Rendement : 21 %

Infrarouge (nujol) : $\nu_{NH}$ : 3380 $cm^{-1}$
$\nu_{CO}$ amide : 1580 $cm^{-1}$

**Exemple 17 : Acide 1-myristoylamino-2-phényléthanephosphinique**

L'acide 1-amino-2-phényléthanephosphinique utilisé est décrit dans J. Chem. Soc., Perkin Trans, 1, 2845-2853, 1984.
Purification par recristallisation dans de l'acétate d'éthyle.
Rendement : 81 %
Point de fusion : 108 ° C
Infrarouge (nujol) : $\nu_{NH}$ : 3290 $cm^{-1}$
$\nu_{OH}$ entre 3100 et 2000 $cm^{-1}$
$\nu_{CO}$ amide ≃ 1650 $cm^{-1}$

| Microanalyse élémentaire : | | | |
|---|---|---|---|
| | C % | H % | N % |
| calculé | 66,81 | 9,68 | 3,64 |
| trouvé | 66,76 | 9,57 | 3,54 |

**Exemple 18 : Myristoylaminométhanephosphonte de diéthyle**

Rendement : 60 %
Infrarouge : $\nu_{NH}$ : 3280 $cm^{-1}$
$\nu_{CO}$ amide I : 1650 $cm^{-1}$
$\nu_{CO}$ amide II : 1550 $cm^{-1}$

**Exemple 19 : Acide myristoylaminométhanephosphonique**

En procédant comme dans l'exemple 15 mais en saponifiant le composé décrit dans l'exemple 18, on obtient le produit attendu.
Rendement : 58 %
Infrarouge (nujol) : $\nu_{NH}$ : 3260 $cm^{-1}$
$\nu_{OH}$ entre 3500 et 2000 $cm^{-1}$
$\nu_{CO}$ amide I : 1650 $cm^{-1}$
$\nu_{CO}$ amide II : 1550 $cm^{-1}$

| Microanalyse élémentaire : | | | |
|---|---|---|---|
| | C % | H % | N % |
| calculé | 56,06 | 10,03 | 4,35 |
| trouvé | 56,38 | 10,36 | 4,33 |

**Exemple 20 : 1-(S)-Myristoylaminoéthanephosphonate de diéthyle**

Rendement : 55 %

**Exemple 21 : Acide 1-(S)-myristoylaminoéthanephosphonique**

En procédant comme dans l'exemple 15 mais en saponifiant le composé décrit dans l'exemple 20, on obtient le produit attendu.
Rendement : 49 %
Infrarouge (nujol) : $\nu_{NH}$ : 3280 $cm^{-1}$
$\nu_{OH}$ entre 3600 et 2000 $cm^{-}1$

$\nu_{CO}$ amide I : 1650 $cm^{-1}$
$\nu_{CO}$ amide II : 1540 $cm^{-1}$

| Microanalyse élémentaire : | | | |
|---|---|---|---|
| | C % | H % | N % |
| calculé | 57,29 | 10,22 | 4,17 |
| trouvé | 57,50 | 10,13 | 4,08 |

**Exemple 22 : 1-(R)-Myristoylaminoéthanephosphonate de diéthyle**

Rendement : 52 %

**Exemple 23 : Acide 1-(R)-myristoylaminoéthanephosphonique**

En procédant comme dans l'exemple 15 mais en saponifiant le composé décrit dans l'exemple 22, on obtient le produit attendu.
Rendement : 41 %
Infrarouge (nujol) : $\nu_{NH}$ : 3280 $cm^{-1}$
$\nu_{OH}$ entre 3500 et 2000 $cm^{-1}$
$\nu_{CO}$ amide I : 1650 $cm^{-1}$
$\nu_{CO}$ amide II : 1540 $cm^{-1}$

| Microanalyse élémentaire : | | | |
|---|---|---|---|
| | C % | H % | N % |
| calculé | 57,29 | 10,22 | 4,17 |
| trouvé | 57,21 | 10,10 | 4,02 |

**Exemple 24 : N-Myristoyl-(S)-$\beta$-cyclohexylalanine**

La (S)-$\beta$-cyclohexylalanine est préparée selon le procédé décrit dans J. Med. Chem., 15 (8), 794, 1972.
Rendement : 60 %
Point de fusion : 88 ° C
Infrarouge (nujol) : $\nu_{NH}$ : 3340 $cm^{-1}$
$\nu_{OH}$ entre 3300 et 2000 $cm^{-1}$
$\nu_{CO}$ acide : 1720 $cm^{-1}$
$\nu_{CO}$ amide I : 1620 $cm^{-1}$
$\nu_{CO}$ amide II : 1560 $cm^{-1}$

| Microanalyse élémentaire : | | | |
|---|---|---|---|
| | C % | H % | N % |
| calculé | 72,39 | 11,36 | 3,67 |
| trouvé | 72,40 | 11,30 | 3,59 |

**Exemple 25 : N-Myristoyl-(S)-phénylalaninate de carboxamidométhyle**

4 mmoles de N-myristoyl-(S)-phénylalanine sont solubilisées dans 15 ml de méthanol et 1,5 ml d'eau. 4,6 ml d'une solution aqueuse à 20 % de carbonate de césium sont ajoutés à ce mélange et l'ensemble est agité 10 minutes. Après évaporation des solvants et séchage, le résidu est dissous dans 20 ml de diméthylformamide. 44 mmoles de chloroacétamide sont alors ajoutées et l'ensemble est agité 20 heures à

température ambiante. Après évaporation du solvant, le résidu est repris par de l'eau et extrait par de l'acétate d'éthyle. Le produit attendu est alors obtenu après évaporation et recristallisation dans de l'oxyde d'isopropyle.

Rendement : 18 %
Point de fusion : 104 °C
Infrarouge (nujol) : $\nu_{NH}$ : 3354, 3307 et 3159 $cm^{-1}$
$\nu_{CO}$ ester : 1757 $cm^{-1}$
$\nu_{CO}$ amide I : 1739 et 1707 $cm^{-1}$
$\nu_{CO}$ amide II : 1635 et 1620 $cm^{-1}$

| Microanalyse élémentaire : | | | |
|---|---|---|---|
| | C % | H % | N % |
| calculé | 69,41 | 9,32 | 6,47 |
| trouvé | 69,11 | 9,48 | 6,33 |

**Exemple 26 : N-Myristoyl-(S)-phénylalaninate de glycérolacétonide**

5 mmoles de N-myristoylphénylalanine sont dissoutes dans 50 ml de dichlorométhane, puis 5 mmoles de triéthylamine sont ajoutées à cette solution en refroidissant à 0 °C. Une solution contenant 55 mmoles de chloroformiate d'isobutyle dans 10 ml de dichlorométhane est additionnée lentement au mélange précédent puis 5 mmoles de 4-diméthylaminopyridine en maintenant l'ensemble à 0 °C. Enfin, après addition d'une solution contenant 5 mmoles d'isopropylidène glycérol dans 10 ml de dichlorométhane, l'ensemble est agité 18 heures à température ambiante. Le dichlorométhane est évaporé et le résidu repris par 50 ml d'acétate d'éthyle. La solution est lavée par une solution saturée de bicarbonate de sodium puis par de l'eau. Après séchage et évaporation, le produit attendu est obtenu après purification sur colonne de silice (solvant d'élution : dichlorométhane/acétate d'éthyle : 90/10).

Rendement : 52 %
Infrarouge (nujol) : $\nu_{NH}$ : 3300 $cm^{-1}$
$\nu_{CO}$ ester : 1740 $cm^{-1}$
$\nu_{CO}$ amide I : 1640 $cm^{-1}$
$\nu_{CO}$ amide II : 1540 $cm^{-1}$

| Microanalyse élémentaire : | | | |
|---|---|---|---|
| | C % | H % | N % |
| calculé | 71,13 | 9,67 | 2,86 |
| trouvé | 71,07 | 9,87 | 2,77 |

**Exemple 27 : N-Myristoyl-(S)-phénylalaninate de glycérol**

15 mmoles du produit obtenu dans l'exemple 26 sont dissoutes dans 5 ml de méthanol et 1,5 ml d'acide chlorhydrique N. La solution est abandonnée 48 heures à température ambiante. Après évaporation, le produit attendu est obtenu après purification sur colonne de silice (solvant d'élution : dichlorométhane/méthanol : 97/3).

Rendement : 51 %
Infrarouge (nujol) : $\nu_{NH}$ et $\nu_{OH}$ entre 3600 et 3100 $cm^{-1}$
$\nu_{CO}$ ester : 1736 $cm^{-1}$
$\nu_{CO}$ amide : 1647 $cm^{-1}$

| Microanalyse élémentaire : | | | |
|---|---|---|---|
| | C % | H % | N % |
| calculé<br>trouvé | 69,45<br>69,41 | 9,64<br>9,74 | 3,11<br>3,01 |

**Exemple 28 : N-Myristoyl-(imidazo[1,2-a]pyridin-2-yl)alanine**

La (imidazo[1,2-a]pyridin-2-yl)alanine utilisée est décrite dans la préparation A.
Rendement : 25 %
Infrarouge (nujol) : $\nu_{NH}$ : 3288 $cm^{-1}$
$\nu_{CO}$ carboxylate et amide : 1635 $cm^{-1}$
$\nu_{C=C}$ : 1591 $cm^{-1}$

**Exemple 29 : N-Myristoyl-($N^{\tau}$-benzyl)spinacine**

Rendement : 23 %
Infrarouge (nujol) : $\nu_{OH}$ entre 3600 et 2000 $cm^{-1}$
$\nu_{CO}$ : 1639 $cm^{-1}$
$\nu_{CO}$ carboxylate : 1600 $cm^{-1}$

**Exemple 30 : N-Myristoyl-($N^{\pi}$-benzyl)spinacine**

Rendement : 25 %
Infrarouge (nujol) : $\nu_{OH}$ entre 3600 et 2000 $cm^{-1}$
$\nu_{CO}$ acide : 1720 $cm^{-1}$
$\nu_{CO}$ amide : 1641 $cm^{-1}$

**Exemple 31 : N-Palmitoyl-($N^{\tau}$-benzyl)histidine**

Rendement : 50 %
Infrarouge (nujol) : $\nu_{NH}$ : 3300 $cm^{-1}$
$\nu_{OH}$ entre 3400 et 2300 $cm^{-1}$
$\nu_{CO}$ amide I : 1640 $cm^{-1}$
$\nu_{CO}$ amide II : 1550 $cm^{-1}$

| Microanalyse élémentaire : | | | |
|---|---|---|---|
| | C % | H % | N % |
| calculé<br>trouvé | 72,01<br>72,14 | 9,38<br>9,33 | 8,69<br>8,62 |

**Exemple 32 : N-Lauroyl-($N^{\tau}$-benzyl)histidine**

Rendement : 48 %
Infrarouge (nujol) : $\nu_{NH}$ : 3200 $cm^{-1}$
$\nu_{OH}$ entre 3400 et 2000 $cm^{-1}$
$\nu_{CO}$ acide : 1710 $cm^{-1}$
$\nu_{CO}$ amide I : 1640 $cm^{-1}$
$\nu_{CO}$ amide II : 1550 $cm^{-1}$

| Microanalyse élémentaire : | | | |
|---|---|---|---|
| | C % | H % | N % |
| calculé<br>trouvé | 70,33<br>69,22 | 8,72<br>8,81 | 9,83<br>9,63 |

**Exemple 33 : N-Palmitoyl-p.(diéthylphosphonométhyl)phénylalanine**

Rendement : 76 %
Point de fusion : 73 ° C
Infrarouge (nujol) : $\nu_{NH}$ : 3280 $cm^{-1}$
$\nu_{OH}$ entre 2800 et 2500 $cm^{-1}$
$\nu_{CO}$ acide : 1745 $cm^{-1}$
$\nu_{CO}$ amide I : 1650 $cm^{-1}$
$\nu_{CO}$ amide II : 1540 $cm^{-1}$

**Exemple 34 : N-Lauroyl-p.(diéthylphosphoflométhyl)phénylalanine**

Rendement : 68 %
Point de fusion : 52 ° C
Infrarouge (nujol) : $\nu_{NH}$ : 3270 $cm^{-1}$
$\nu_{OH}$ entre 3000 et 2400 $cm^{-1}$
$\nu_{CO}$ acide : 1745 $cm^{-1}$
$\nu_{CO}$ amide I : 1650 $cm^{-1}$
$\nu_{CO}$ amide II : 1535 $cm^{-1}$

**Exemple 35 : N-(Tridécanephosphonyl)-(S)-phénylalaninate de méthyle, sel monosodique**

STADE 1 :

2,2 mmoles de chlorure de tridécanephosphonate de benzyle obtenu selon la méthode décrite par K.A. PETNOV (J. GEN. CHEM. USSR, 29, 1465-1467, 1959) sont condensées avec 2,2 mmoles de (S)-phénylalaninate de méthyle dans 10 ml de dichlorométhane en présence de 0,65 ml triéthylamine. Le produit attendu est obtenu après évaporation des solvants et purification sur colonne de silice (solvant d'élution : dichlorométhane/acétate d'éthyle : 9/1).
Rendement : 70 %

STADE 2 : N-(Tridécanephosphonyl)-(S)-phénylalaninate de méthyle, sel monosodique

1,5 mmoles du produit obtenu au stade précédent sont hydrogénés dans 100 ml de méthanol en présence de palladium sur charbon à 10 % et de bicarbonate de sodium. Après élimination du catalyseur et des solvants, le produit attendu est obtenu après lyophilisation.
Rendement : 79 %

**Exemple 36 : N-(12-Méthylthiolauroyl)-(S)-phénylalanine**

STADE 1 : N-(12-Bromolauroyl)-(S)-phénylalanine

En procédant comme dans l'exemple 1 stade 2 mais en remplaçant au stade 2 le myristate de N-hydroxysuccinimide par le 12-bromolauroate de N-hydroxy-succinimide et la 3-(S)-carboxytétrahydro-[1,2,3,4]isoquinoléine par la (S)-phénylalanine, on obtient le produit attendu.
Rendement : 61 %

STADE 2 : N-(12-Méthylthiolauroyl)-(S)-phénylalanine

3 mmoles du produit obtenu au stade précédent sont dissoutes dans 25 ml d'éthanol contenant 6 mmoles de potasse à 85 %. Un barbottage de méthylmercaptan est alors réalisé pendant 45 minutes et l'ensemble est chauffé à 60 °C pendant 5 heures. Après refroidissement et acidification, les solvants sont évaporés. Le résidu est repris par 30 ml d'acétate d'éthyle et la solution est lavée par de l'eau puis par une solution saturée de chlorure de sodium. Le produit attendu est obtenu après évaporation des solvants et purification sur colonne de silice (solvant d'élution : dichlorométhane/éthanol : 97/3).
Rendement : 20 %
Infrarouge (nujol) : $\nu_{NH}$ : 3303 $cm^{-1}$
$\nu_{OH}$ entre 3500 et 2400 $cm^{-1}$
$\nu_{CO}$ acide : 1730 $cm^{-1}$
$\nu_{CO}$ amide : 1643 $cm^{-1}$

| Microanalyse élémentaire : | | | | |
|---|---|---|---|---|
| | C % | H % | N % | S % |
| calculé | 67,14 | 8,96 | 3,56 | 8,15 |
| trouvé | 67,05 | 9,07 | 3,91 | 7,95 |

Les exemples 37 à 48, 50, 52 à 55 et 60 ont été préparés selon le même mode opératoire que celui décrit pour l'exemple 36, à partir de matières premières décrites dans la littérature.

**Exemple 37 : N-(11-Ethylthioundéoanoyl)-(S)-phénylalanine**

Rendement : 20 %
Point de fusion : 57 °C
Infrarouge (nujol) : $\nu_{NH}$ : 3300 $cm^{-1}$
$\nu_{OH}$ entre 3400 et 2000 $cm^{-1}$
$\nu_{CO}$ acide : 1700 $cm^{-1}$
$\nu_{CO}$ amide I : 1600 $cm^{-1}$
$\nu_{CO}$ amide II : 1550 $cm^{-1}$

| Microanalyse élémentaire : | | | | |
|---|---|---|---|---|
| | C % | H % | N % | S % |
| calculé | 67,13 | 8,96 | 3,56 | 8,14 |
| trouvé | 67,40 | 9,09 | 3,45 | 7,92 |

**Exemple 38 : N-(11-Ethylthioundécanoyl)-(S)-leucine**

Rendement : 25 %
Infrarouge (nujol) : $\nu_{NH}$ : 3327 $cm^{-1}$
$\nu_{OH}$ entre 3000 et 2500 $cm^{-1}$
$\nu_{CO}$ acide : 1697 $cm^{-1}$
$\nu_{CO}$ amide I : 1622 $cm^{-1}$
$\nu_{CO}$ amide II : 1522 $cm^{-1}$

| Microanalyse élémentaire : | | | | |
|---|---|---|---|---|
| | C % | H % | N % | S % |
| calculé | 63,47 | 10,37 | 3,90 | 8,92 |
| trouvé | 63,81 | 10,48 | 3,85 | 8,56 |

**Exemple 39 : 1-(11-Ethylthioundécanoylamino)-2-phényléthanephosphonate de diéthyle**

Rendement : 23 %
Infrarouge (film liquide) :
$\nu_{NH}$ : 3267 $cm^{-1}$
$\nu_{CO}$ : 1660 $cm^{-1}$

| Microanalyse élémentaire : | | | | |
|---|---|---|---|---|
| | C % | H % | N % | S % |
| calculé | 61,83 | 9,13 | 2,88 | 6,60 |
| trouvé | 61,84 | 9,33 | 2,96 | 6,66 |

**Exemple 40 : N-(11-Ethylthioundécanoyl)-(S)-phénylalaninate de carboxamidométhyle**

Rendement : 32 %
Infrarouge (chloroforme) : $\nu_{NH}$ : 3400 $cm^{-1}$
$\nu_{CO}$ ester : 1739 $cm^{-1}$
$\nu_{CO}$ amide : 1637 et 1620 $cm^{-1}$

| Microanalyse élémentaire : | | | | |
|---|---|---|---|---|
| | C % | H % | N % | S % |
| calculé | 63,97 | 8,50 | 6,22 | 7,12 |
| trouvé | 64,03 | 8,68 | 6,18 | 6,73 |

**Exemple 41 : N-(11-Ethylthioundécanoyl)-($N^{\tau}$-benzyl)-(S)-histidine**

Rendement : 24 %
Infrarouge (nujol) : $\nu_{NH}$ : 3315 $cm^{-1}$
$\nu_{CO}$ acide : 1705 $cm^{-1}$
$\nu_{CO}$ amide : 1643 $cm^{-1}$

| Microanalyse élémentaire : | | | | |
|---|---|---|---|---|
| | C % | H % | N % | S % |
| calculé | 65,93 | 8,30 | 8,87 | 6,77 |
| trouvé | 66,00 | 8,57 | 8,58 | 6,17 |

**Exemple 42 : N-(11-Ethylthioundécanoyl)-α-éthyl-α-phénylglycine**

Rendement : 35 %
Infrarouge (nujol) : $\nu_{NH}$ et $\nu_{OH}$ entre 3700 et 2000 $cm^{-1}$
$\nu_{CO}$ acide : 1743 $cm^{-1}$
$\nu_{CO}$ amide : 1637 $cm^{-1}$

| Microanalyse élémentaire : | | | | |
|---|---|---|---|---|
| | C % | H % | N % | S % |
| calculé<br>trouvé | 67,77<br>67,61 | 9,15<br>9,29 | 3,44<br>3,55 | 7,87<br>7,70 |

**Exemple 43 : N-(11-Ethylthioundécanoyl)-4-hydroxy-3,5-diterbutylphénylalanine**

Rendement : 19 %
Infrarouge : $\nu_{OH}$ : 3643 $cm^{-1}$
$\nu_{CO}$ amide I : 1650 $cm^{-1}$
$\nu_{CO}$ amide II : 1568 $cm^{-1}$

**Exemple 44 : N-(11-Ethylthioundécanoyl)-(S)-$\beta$-cyclohexylalanine**

Rendement : 32 %
Infrarouge (nujol) : $\nu_{NH}$ : 3332 $cm^{-1}$
$\nu_{OH}$ entre 3600 et 1800 $cm^{-1}$
$\nu_{CO}$ acide : 1697 $cm^{-1}$
$\nu_{CO}$ amide : 1622 $cm^{-1}$

| Microanalyse élémentaire : | | | | |
|---|---|---|---|---|
| | C % | H % | N % | S % |
| calculé<br>trouvé | 66,12<br>65,67 | 10,34<br>10,40 | 3,50<br>3,66 | 8,02<br>8,23 |

**Exemple 45 : N-(11-Ethylthioundécanoyl)-(S)-proline**

Rendement : 40 %
Infrarouge (film liquide) : $\nu_{OH}$ : 2800 $cm^{-1}$
$\nu_{CO}$ acide : 1743 $cm^{-1}$
$\nu_{CO}$ amide : 1651 $cm^{-1}$

| Microanalyse élémentaire : | | | | |
|---|---|---|---|---|
| | C % | H % | N % | S % |
| calculé<br>trouvé | 62,93<br>62,80 | 9,68<br>9,32 | 4,08<br>4,23 | 9,33<br>9,24 |

**Exemple 46 : N-(11-Ethylthioundécanoyl)-3-(S)-carboxy-(1,2,3,4)-tétrahydroisoquinoléine**

Rendement : 32 %
Infrarouge (chloroforme) : $\nu_{OH}$ entre 3600 et 2000 $cm^{-1}$
$\nu_{CO}$ acide : 1720 $cm^{-1}$
$\nu_{CO}$ amide : 1610 $cm^{-1}$

**Exemple 47 : N-(5-Octylthiopentanoyl)-(S)-phénylalanine**

Rendement : 40 %
Infrarouge (nujol) : $\nu_{NH}$ : 3302 $cm^{-1}$
$\nu_{OH}$ entre 3400 et 1850 $cm^{-1}$

$\nu_{CO}$ acide : 1709 $cm^{-1}$
$\nu_{CO}$ amide : 1616 $cm^{-1}$

| Microanalyse élémentaire : | | | | |
|---|---|---|---|---|
| | C % | H % | N % | S % |
| calculé<br>trouvé | 67,14<br>66,20 | 8,96<br>8,87 | 3,56<br>3,30 | 8,15<br>7,60 |

**Exemple 48 : N-(12-Méthoxylauroyl)-(S)-phénylalaninate d'éthyle**

Le produit attendu est obtenu selon le même mode opératoire que celui décrit dans l'exemple 36 mais au stade 2 le méthylmercaptan est remplacé par 2 équivalents de méthylate de sodium.
Rendement : 50 %
Infrarouge (nujol) : $\nu_{NH}$ : 3300 $cm^{-1}$
$\nu_{CO}$ ester : 1732 $cm^{-1}$
$\nu_{CO}$ amide : 1645 $cm^{-1}$

| Microanalyse élémentaire : | | | |
|---|---|---|---|
| | C % | H % | N % |
| calculé<br>trouvé | 71,07<br>71,30 | 9,69<br>9,75 | 3,45<br>3,44 |

**Exemple 49 : N-(12-Méthoxylauroyl)(S)-phénylalanine**

Le produit attendu est obtenu par saponification dans de la potasse N en milieu méthanolique du composé décrit dans l'exemple 48.
Rendement : 88 %
Infrarouge (nujol) : $\nu_{NH}$ : 3352 $cm^{-1}$
$\nu_{OH}$ entre 3500 et 2200 $cm^{-1}$
$\nu_{CO}$ acide : 1701 $cm^{-1}$
$\nu_{CO}$ amide I : 1676 $cm^{-1}$
$\nu_{CO}$ amide II : 1525 $cm^{-1}$

| Microanalyse élémentaire : | | | |
|---|---|---|---|
| | C % | H % | N % |
| calculé<br>trouvé | 69,99<br>69,31 | 9,34<br>9,69 | 3,71<br>3,48 |

**Exemple 50 : 1-(12-Méthoxylauroylamino)-2-phényléthanephosphonate de diéthyle**

Le produit attendu est obtenu selon le même mode opératoire que celui décrit dans l'exemple 48.
Rendement : 25 %
Infrarouge (nujol) : vNH : 3269 $cm^{-1}$
vCO amide I : 1678 $cm^{-1}$
vCO amide II : 1541 $cm^{-1}$

| Microanalyse élémentaire : | | | |
|---|---|---|---|
| | C % | H % | N % |
| calculé<br>trouvé | 63,94<br>63,83 | 9,44<br>9,51 | 2,98<br>2,86 |

**Exemple 51 : Acide 1-(12-méthoxylauroylamino)-2-phényléthanephosphonique**

Le produit attendu est obtenu par saponification du composé décrit dans l'exemple 50 dans de la potasse en milieu éthanolique en présence de bromure de tétrabutylammonium, pendant 2 heures à 60 ° C.
Rendement : 20 %
Infrarouge (nujol) : $\nu_{NH}$ : 3282 $cm^{-1}$
$\nu_{CO}$ amide : 1645 $cm^{-1}$

**Exemple 52 : N-(10-propyloxydécanoyl)-(S)-phénylalanine**

Le produit attendu est obtenu selon le même mode opératoire que celui décrit dans l'exemple 36 mais, au stade 2, le méthylmercaptan est remplacé par 2 équivalents de propanolate de sodium.
Rendement : 15 %
Infrarouge (film liquide) : $\nu_{NH}$ et $\nu_{OH}$ entre 3600 et 1900 $cm^{-1}$
$\nu_{CO}$ acide : 1738 $cm^{-1}$
$\nu_{CO}$ amide I : 1649 $cm^{-1}$
$\nu_{CO}$ amide II : 1543 $cm^{-1}$

| Microanalyse élémentaire : | | | |
|---|---|---|---|
| | C % | H % | N % |
| calculé<br>trouvé | 69,99<br>69,71 | 9,34<br>9,45 | 3,71<br>3,79 |

**Exemple 53 : N-(10-Propargyloxydéoanoyl)-(S)-phénylalanine**

Le produit attendu est obtenu selon le même mode opératoire que celui décrit dans l'exemple 36 mais, au stade 2, le méthylmercaptan est remplacé par 2 équivalents de propargylate de sodium.
Rendement : 25 %
Infrarouge (nujol) : $\nu_{NH}$ et $\nu_{OH}$ entre 3600 et 1800 $cm^{-1}$
$\nu_{CO}$ acide : 1734 $cm^{-1}$
$\nu_{CO}$ amide I : 1649 $cm^{-1}$
$\nu_{CO}$ amide II : 1535 $cm^{-1}$

| Microanalyse élémentaire : | | | |
|---|---|---|---|
| | C % | H % | N % |
| calculé<br>trouvé | 70,75<br>70,35 | 8,37<br>8,25 | 3,75<br>3,99 |

**Exemple 54 : N-(p.décylbenzoyl)-(S)-phénylalanine**

Le produit attendu est obtenu selon le même mode opératoire que celui décrit pour l'exemple 1 mais, au stade 1, l'acide myristique est remplacé par l'acide p.décylbenzoïque.
Pendement : 90 %
Point de fusion : 109 ° C

Infrarouge (nujol) : $\nu_{NH}$ : 3320 $cm^{-1}$
$\nu_{OH}$ entre 3000 et 2300 $cm^{-1}$
$\nu_{CO}$ acide : 1725 $cm^{-1}$
$\nu_{CO}$ amide : 1635 $cm^{-1}$

| Microanalyse élémentaire : | | | |
|---|---|---|---|
| | C % | H % | N % |
| calculé | 76,25 | 8,61 | 3,42 |
| trouvé | 75,50 | 8,64 | 3,39 |

**Exemple 55 : N-(p.décylbenzoyl)glycine**

Le mode opératoire est le même que celui décrit dans l'exemple 54.
Rendement : 59 %
Point de fusion : 134 °C
Infrarouge (nujol) : $\nu_{NH}$ : 3320 $cm^{-1}$
$\nu_{OH}$ entre 3100 et 2300 $cm^{-1}$
$\nu_{CO}$ acide : 1740 $cm^{-1}$
$\nu_{CO}$ amide : 1620 $cm^{-1}$

| Microanalyse élémentaire : | | | |
|---|---|---|---|
| | C % | H % | N % |
| calculé | 71,44 | 9,15 | 4,38 |
| trouvé | 71,25 | 9,05 | 4,23 |

**Exemple 56 : N-(12-Hydroxylauroyl)(S)-phénylalanine**

Le produit attendu est obtenu selon le même mode opératoire que celui décrit pour l'exemple 1 mais, au stade 1, l'acide myristique est remplacé par l'acide 12-hydroxylaurique.
Rendement : 85 %
Point de fusion : 90 °C
Infrarouge (nujol) : $\nu_{NH}$ et $\nu_{OH}$ entre 3583 et 1800 $cm^{-1}$
$\nu_{CO}$ acide : 1720 $cm^{-1}$
$\nu_{CO}$ amide I : 1647 $cm^{-1}$
$\nu_{CO}$ amide II : 1539 $cm^{-1}$

| Microanalyse élémentaire : | | | |
|---|---|---|---|
| | C % | H % | N % |
| calculé | 69,39 | 9,15 | 3,85 |
| trouvé | 68,73 | 9,18 | 3,51 |

**Exemple 57 : N-Myristoyl-2-(S)-carboxy indoline**

| Microanalyse élémentaire : | | | |
|---|---|---|---|
| | C % | H % | N % |
| calculé<br>trouvé | 73,96<br>74,00 | 9,44<br>9,29 | 3,75<br>3,99 |

**Exemple 58 : N-Myristoyl-3-(S)-carboxy-1,2,3,4-tétrahydro-β-carboline**

| Microanalyse élémentaire : | | | |
|---|---|---|---|
| | C % | H % | N % |
| calculé<br>trouvé | 73,20<br>72,85 | 8,98<br>9,04 | 6,57<br>6,58 |

**Exemple 59 : N-Myristoyl-2-carboxy pipéridine**

| Microanalyse élémentaire : | | | |
|---|---|---|---|
| | C % | H % | N % |
| calculé<br>trouvé | 70,75<br>70,78 | 10,98<br>11,00 | 4,13<br>4,44 |

**Exemple 60 : N-(10-Propylthiodécanoyl)-(S)-phénylalanine**

| Microanalyse élémentaire : | | | | |
|---|---|---|---|---|
| | C % | H % | N % | S % |
| calculé<br>trouvé | 67,14<br>67,11 | 8,96<br>8,95 | 3,56<br>3,59 | 8,15<br>7,92 |

**Exemple 61 : N-(12-Méthoxylauroyl)glycine**

Le produit attendu est obtenu selon le même mode opératoire que celui décrit dans l'exemple 48.
Infrarouge : $\nu_{CO}$ acide : 1703 $cm^{-1}$
$\nu_{CO}$ amide : 1645 $cm^{-1}$

**Exemple 62 : N-(9-Méthoxyéthoxy-nonanoyl)-(S) Phénylalanine**

Le produit attendu est obtenu selon le même mode opératoire que celui décrit dans l'exemple 48.

| Microanalyse élémentaire : | | | |
|---|---|---|---|
| | C % | H % | N % |
| calculé | 66,46 | 8,76 | 3,69 |
| trouvé | 65,88 | 8,86 | 3,81 |

ETUDE PHARMACOLOGIQUE DES DERIVES DE L'INVENTION

**Exemple 63 : Inhibition de l'activité NMT cytosolique de cellules cancéreuses L1210 par les composés de l'invention**

Les cellules L1210 (leucémie murine) en culture en masse sont utilisées comme source de NMT.

Elles sont cultivées dans du RPMI 1640 supplémenté avec 50 U/ml de pénicilline, 50 $\mu$M de streptomycine, 2 mM de glutamine, 10 mM HEPES et 10 % de sérum de veau foetal, maintenues en atmosphère 5 % $CO_2$/95 % air et à 37°C.

Elles sont récoltées puis lavées dans du PBS par centrifugation à basse vitesse. Le culot cellulaire final est resuspendu dans un tampon HEPES 50 mM, pH 7,4 EGTA 2 mM, DTT 1 mM et PMSF 1 mM. Les cellules sont éclatées par sonication à 4°C et homogénéisées par aller-retour dans un potter verre/verre. L'homogénat cellulaire est ensuite centrifugé à basse vitesse (10000 rpm, 10 minutes) pour précipiter les débris cellulaires et le surnageant soumis à ultra centrifugation à 105000 x g pendant 1 heure.

Le surnageant est alors utilisé comme source de NMT. La mesure de l'activité se fait selon la méthode décrite par Towler et Glazer (PNAS, 1986, 83, 2812) en utilisant comme peptide substrat soit le peptide dérivé de $NH_2$ terminal du produit d'oncogène pp60src : GSSKSKPKDP (DP), soit le peptide dérivé de $NH_2$ terminal du produit de gag, protéine structurale du virus leucémique murin de Moloney : GQTVTTPL (T3) à la concentration finale de 0,3 mM.

Dans ces conditions tous les dérivés de l'invention présentent une activité nettement supérieure à celle de la N-myristoylglycine.

Plus particulièrement, le composé de l'exemple 1 présente une $IC_{50}$ égale à $1,8.10^{-7}$ M, celle du composé de l'exemple 5 est égale à $6.10^{-7}$ M, enfin celle du composé de l'exemple 16 est égale à $2.10^{-6}$ M.

**Exemple 64 : Inhibition de la myristoylation d'un peptide dérivé de gag de HIV-1 (sous type BRU) par les composés de l'invention**

Des lymphocytes T immortalisés (CEM) sont cultivés en masse, comme décrit dans l'exemple 57, puis récoltés par centrifugation et lavés dans du PBS.

Ces cellules sont soniquées (3 brèves périodes de 10 secondes) puis homogénéisées dans un potter verre/verre.

Cet homogénat, dans lequel toutes les cellules ont été éclatées (vérification sous microscope), est soumis à une centrifugation pendant 1 h 10 ) à 105000 x g. Le culot (microsomes) et le surnageant (cytosol) sont recueillis indépendamment puis congelés.

L'activité NMT est ensuite mesurée en suivant le protocole de Towler et Glaser mais en stoppant la réaction par 200 $\mu$l d'acétonitrile pour éviter la précipitation du peptide myristoylé. Ce peptide, de 20 AA : $NH_2$GARASVLSGGELDRWEKIRLL$_{COOH}$, est dérivé de p18 du virus HIV-1 (sous type BRU).

Ces expérimentations sont conduites avec une source cytosolique (40 $\mu$l) ou microsomale (20 $\mu$l traitée par 4 $\mu$l de Triton 770, 10 %) de cellules CEM, en présence de concentrations croissantes des composés de l'invention (1 à 1000 $\mu$M, final). Les échantillons, incubés pendant 30 minutes à 37°C et stoppés par 200 $\mu$l d'acétonitrile, sont ensuites analysés par HPLC et la myristoylation de p18 quantifiée.

Dans ces conditions, le composé de l'exemple 1 présente une $IC_{50}$ égale à $10^{-4}$ M lors de l'incubation avec la fraction microsomale, et une $IC_{50}$ égale à $8.10^{-6}$ M lors de l'incubation dans le cytosol de ces cellules CEM.

**Exemple 65 : Effet protecteur des composés de l'invention des cellules CEM vis-à-vis de l'infection par le virus HIV**

La méthode utilisée est décrite par WEISLOW et coll. (J. Natl. Cancer Inst., 1989, 81, 577).

Dans ces conditions, le composé de l'exemple 49 montre une activité protectrice ($EC_{50}$) à la concentration de $10^{-5}$ M et une toxicité ($IC_{50}$) à la concentration de $2.10^{-4}$ M, l'index thérapeutique est donc égal à 20.

Ces résultats sont présentés dans le tableau I en annexe.

**Exemple 66 : Différentiation des cellules HL60 en culture par les composés de l'invention**

Des cellules HL60 en culture sont traitées pendant 6 jours par les composés dissous dans un minimum de méthanol, à 9 concentrations différentes. Les doses choisies encadrent l'$IC_{50}$ déterminée au préalable en utilisant l'essai au MTT (Carmichael et al., Cancer Res., 1986, 47, 936).

Les cellules sont lavées (450 $\mu$l), remises en suspension dans un même volume de milieu complet et incubées 1 heure à 37 °C en présence de 7 $\mu$g/ml fluorochrome spécifique de l'ADN.

Les mesures réalisées à l'aide d'un cytomètre à laser permettent d'estimer le pourcentage de cellules accumulées en phase G0 et G1 du cycle cellulaire, et donc différenciées.

Dans ces conditions, les composés présentent un effet différenciateur marqué qui peut aller jusqu'à des doses inférieures à 50 $\mu$g/ml, comme c'est le cas pour les composés des exemples 1, 16 ou 49.

**Exemple 67 : Composition pharmaceutique**

Comprimé : formule de préparation pour 1000 comprimés dosés à 2 mg de principe actif.

| | |
|---|---|
| N-Myristoyl-3-(S)-carboxy-(1,2,3,4)-tétrahydroisoquinoléine | 2 g |
| Hydroxypropylcellulose | 2 g |
| Amidon de blé | 10 g |
| Lactose | 100 g |
| Stéarate de magnésium | 3 g |
| Talc | 3 g |

TABLEAU 1

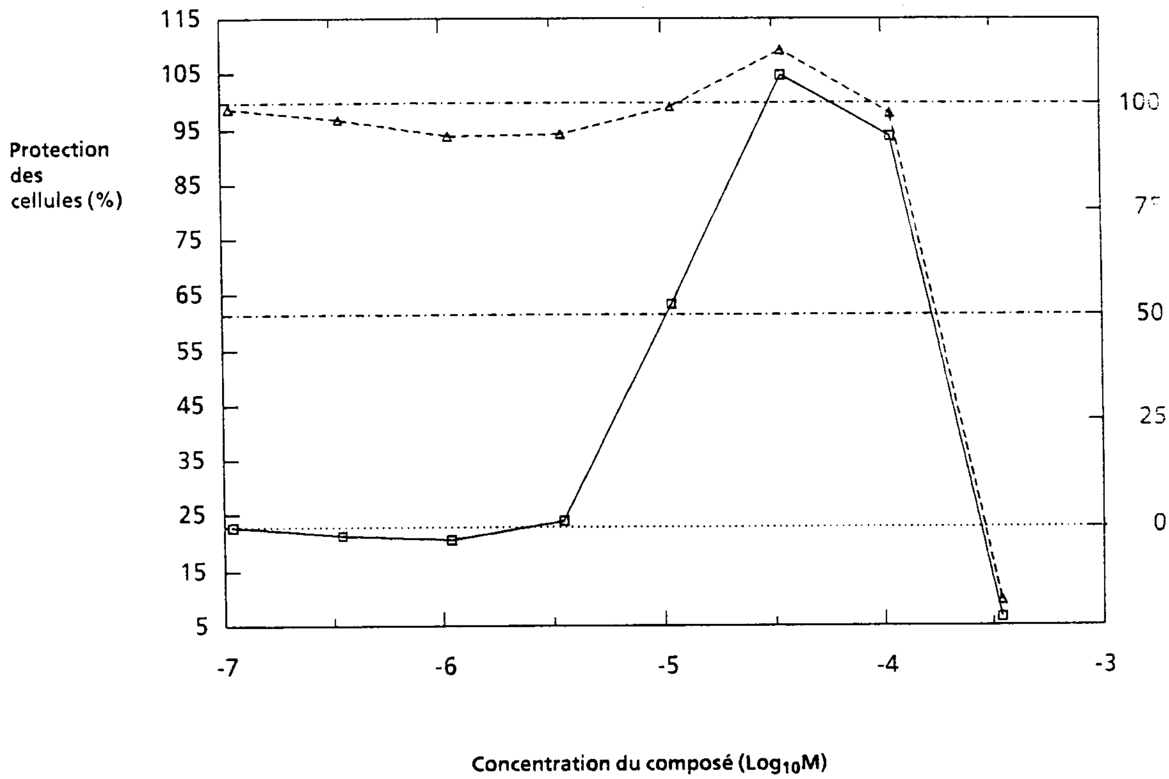


---------- **Cellules non infectées traitées par le composé de l'exemple 49**

———— **Cellules infectées traitées par le composé de l'exemple 49**

## Revendications

**Revendications pour les Etats contractants suivants : AT, BE, CH, LI, DE, DK, FR, GB, IT, LU, NL, PT, SE**

**1.** Composés de formule générale (I)

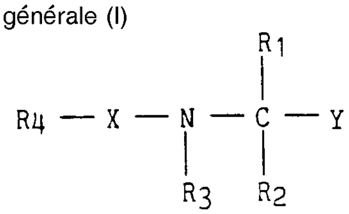

(I)

dans laquelle :

$R_1$ représente

- un atome d'hydrogène,
- un groupement alkyle ($C_1$-$C_6$) linéaire ou ramifié non substitué ou substitué par un ou plusieurs groupements hydroxy, amino, carboxy, carbamoyle, benzylthio, méthylthio, mercapto ou phényle (non substitué ou substitué par un ou plusieurs atomes d'halogène ou groupements hydroxy, alkyle ($C_1$-$C_6$) linéaire ou ramifié, alkoxy ($C_1$-$C_6$) linéaire ou ramifié ou $(CH_3\text{-}CH_2\text{-}O)_2PO\text{-}CH_2\text{-}$),
- un groupement phényle non substitué ou substitué par un ou plusieurs atomes d'halogène ou groupements hydroxy ou alkyle ($C_1$-$C_6$) linéaire ou ramifié,
- un groupement cycloalkyle ($C_3$-$C_7$) méthyle,
- un groupement (imidazol-2-yl) méthyle ou un groupement (indol-3-yl) méthyle non substitué ou substitué sur l'hétérocycle par un groupement benzyle, benzhydryle, trityle, benzyloxyméthyle, tosyle, alkyle ($C_1$-$C_6$) linéaire ou ramifié ou phényle,
- un groupement (1-azaindolizin-2-yl) méthyle de formule :

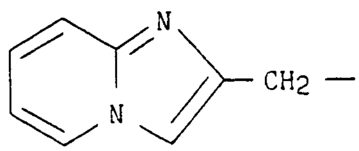

$R_2$ représente un atome d'hydrogène ou un groupement alkyle ($C_1$-$C_6$) linéaire ou ramifié,

$R_3$ représente un atome d'hydrogène ou un groupement alkyle ($C_1$-$C_6$) linéaire ou ramifié,

ou bien

lorsque $R_1$ représente un atome d'hydrogène,

$R_2$ et $R_3$ peuvent former avec les atomes de carbone et d'azote auxquels ils sont respectivement attachés, l'un quelconque des hétérocyles suivants :

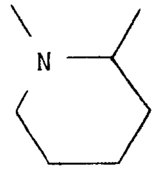 , 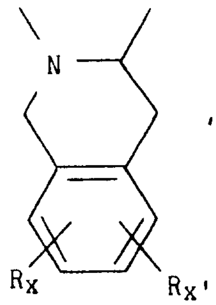 , 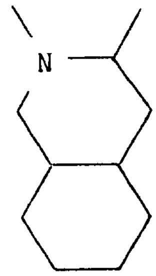 , 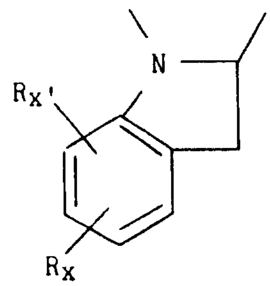 ,

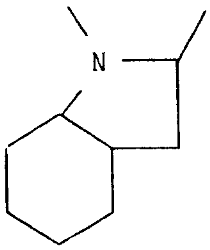 , 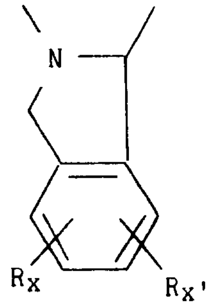 , 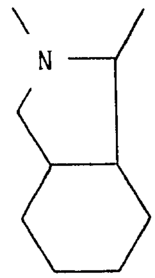 , 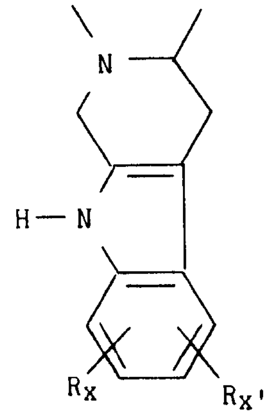

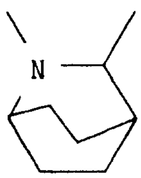 , 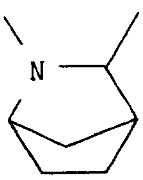 , 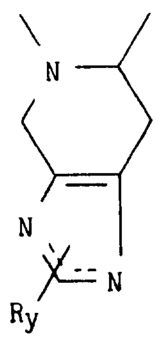

$R_x$ et $R_{x'}$ identiques ou différents représentent un atome d'hydrogène, un groupement alkyle ($C_1$-$C_6$) linéaire ou ramifié, un groupement hydroxy, un groupement alkoxy ($C_1$-$C_6$) linéaire ou ramifié, un atome d'halogène,

ou bien $R_x$ et $R_{x'}$, lorsqu'ils sont situés sur deux carbones adjacents, forment un groupement méthylènedioxy ou éthylènedioxy,

Ry représente un atome d'hydrogène, un groupement alkyle ($C_1$-$C_6$) linéaire ou ramifié, un groupement aryle, un groupement aralkyle, un groupement aroyle, un groupement arylsulfonyl,

X représente l'un quelconque des groupements suivants :

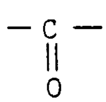 ,

$-SO_2-$ ,

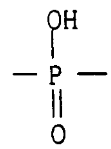 ,

Y représente un groupement — CO — $R_5$ ou

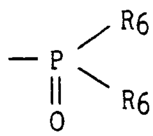

$R_5$ représente un groupement hydroxy, alkoxy ($C_1$-$C_6$) linéaire ou ramifié, $H_2N$-CO-$CH_2$-O-, HO-$CH_2$-CHOH-$CH_2$-O-,

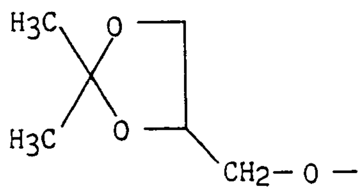 , 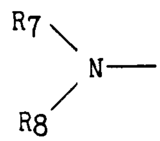 ,

$R_7$ et $R_8$ identiques ou différents représentent un atome d'hydrogène, un groupement alkyle ($C_1$-$C_6$) linéaire ou ramifié ou forment avec l'atome d'azote auxquels ils sont attachés un cycle pyrrolidine, pipéridine, morpholine ou pipérazine.

$R_6$ et $R_{6'}$ identiques ou différents représentent un atome d'hydrogène, un groupement hydroxy ou alkoxy ($C_1$-$C_6$) linéaire ou ramifié,

$R_4$ représente :

1 un groupement alkyle linéaire ou ramifié comportant de 6 à 21 atomes de carbone non substitué ou substitué sur le groupement méthyle terminal par un groupement hydroxy, mercapto, phényle ou éthynyle et dans lequel l'un au moins des groupements méthylènes est remplacé par un atome d'oxygène, de soufre ou un noyau p-phénylène,

dans le cas où :

$R_1$ représente

- un atome d'hydrogène,
- un groupement alkyle ($C_1$-$C_6$) linéaire ou ramifié non substitué ou substitué par un ou plusieurs groupements hydroxy, amino, carboxy, carbamoyle, benzylthio, méthylthio, mercapto ou phényle (non substitué ou substitué par un groupement hydroxy ou alkoxy ($C_1$-$C_6$) linéaire ou ramifié),
- un groupement phényle non substitué,
- un groupement (imidazol-2-yl) méthyle ou un groupement (indol-3-yl) méthyle non substitué ou substitué sur l'hétérocycle par un groupement méthyle

et

$R_2$ = H, $R_3$ = H,

X = 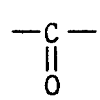 ,

Y = CO $R_{5'}$ ($R_{5'}$ = OH, alkoxy)

2 Dans les autres cas :

un groupement alkyle linéaire ou ramifié comportant de 6 à 21 atomes de carbone non substitué ou substitué sur le groupement méthyle terminal par un groupement hydroxy, mercapto, phényle ou éthynyle et dans lequel un ou plusieurs groupements méthylènes peuvent être remplacés par un atome d'oxygène, de

soufre ou un noyau p-phénylène, (à la condition que lorsque $R_2$ ou $R_3$ forment avec les atomes d'azote ou de carbone auxquels ils sont attachés un cycle 1,2,3,4-tétrahydroisoquinoléine, indoline ou isoindoline alors $R_4$ est différent du groupement : alk-S-$(CH_2)$n- dans lequel : n est égal à 1,2,3,4 ou 5 et alk représente un groupement alkyle ($C_4$-$C_{19}$) linéaire ou ramifié),

leurs isomères, diastéréoisomères et épimères ainsi que leurs sels d'addition à un acide ou à une base pharmaceutiquement acceptable.

**2.** Composé selon la revendication 1 qui est la N-myristoyl-3-carboxy-(1,2,3,4)-tétrahydroiscquinoléine, ses énantiomères ainsi que ses sels d'addition à une base pharmaceutiquement acceptable.

**3.** Composé selon la revendication 1 qui est la N-myristoyl-(N'$^{\tau}$-benzyl)-histidine, ses énantiomères ainsi que ses sels d'addition à un acide ou à une base pharmaceutiquement acceptable.

**4.** Composé selon la revendication 1 qui est le 1-myristoylamino-2-phényléthanehydrogénophosphonate d'éthyle, ses énantiomères ainsi que ses sels d'addition à une base pharmaceutiquement acceptable.

**5.** Composé selon la revendication 1 qui est la N-(12-méthoxylauroyl)-phénylalanine, ses énantiomères ainsi que ses sels d'addition à une base pharmaceutiquement acceptable.

**6.** Procédé de préparation des dérivés de formule (I) selon la revendication 1 caractérisé :

1- dans le cas où les dérivés de formule (I) que l'on souhaite obtenir possèdent un radical $R_4$ = $R_{4'}$ dans lequel aucun groupement méthylène n'est remplacé par un atome de soufre ou d'oxygène, en ce que l'on condense un composé de formule (II) :

$R_{4'}$ - X' - Z (II)

dans laquelle :

X' représente un groupement

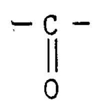,

— $SO_2$ — ,

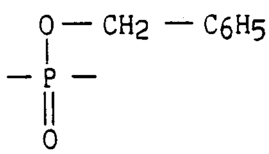,

$R_{4'}$ représente un groupement alkyle linéaire ou ramifié comportant de 6 à 21 atomes de carbone, non substitué ou substitué sur le groupement méthyle terminal par un groupement hydroxy, mercapto, phényle ou éthynyle, et dans lequel un ou plusieurs groupements méthylène peuvent être remplacés par un groupement p.phénylène,

Z représente un atome d'halogène, un groupement hydroxy, un groupement alkoxy ($C_1$-$C_6$) linéaire ou ramifié, un groupement aralkoxy, ou un groupement

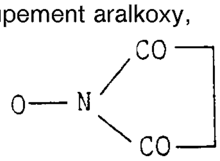,

sur une amine de formule (III) (sous forme racémique ou isomérique) protégée le cas échéant :

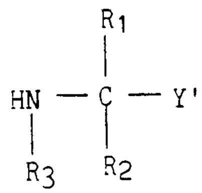

(III)

dans laquelle $R_2$, $R_3$ et $R_4$ ont la même signification que dans la formule I et Y' représente un groupement $-CO_2H$, -CO(alkoxy) ou $-PO(alkoxy)_2$,

pour conduire après déprotection éventuelle à un composé de formule (I/a), cas particulier des composés de formule (I)

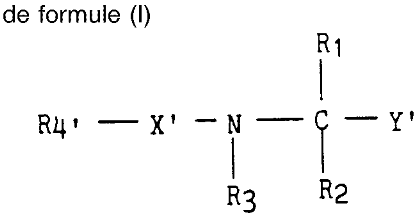

(I/a)

dans laquelle $R_1$, $R_2$, $R_3$, $R_{4'}$, X' et Y' ont la même signification que précédemment,

2- dans le cas où les dérivés de formule (I) que l'on souhaite obtenir possèdent un radical $R_4 = R_{4''} = CH_3-(CH_2)_n-$ dans lequel l'un au moins des groupements méthylène est remplacé par un atome de soufre ou d'oxygène,

en ce que l'on condense :

un composé de formule (IV)

$A - (CH_2)_m - X' - Z$ (IV)

dans laquelle :

- X' et Z ont la même signification que précédemment,
- A représente un atome d'halogène, un groupement mésyloxy ou un groupement tosyloxy,
- m est inférieur ou égal à n-1,

et l'un des groupements méthylène peut être remplacé par un atome d'oxygène, de soufre, un groupement p.phénylène, un groupement $-CH(CH_3)-$ou un groupement $-C(CH_3)_2-$,

sur une amine de formule (III) (sous forme racémique ou isomérique) protégée le cas échéant, telle que définie précédemment,

pour conduire à un composé de formule (V)

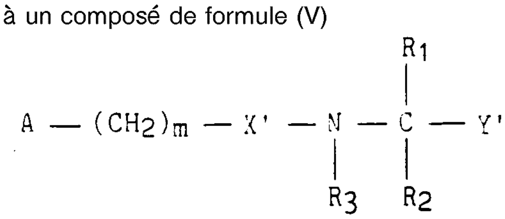

(V)

dans laquelle A, m, X', $R_1$, $R_2$, $R_3$ et Y' sont définis comme précédemment,

qui est mis en réaction avec un dérivé de formule (VI) :

$R_9 - B - M$ (VI)

dans laquelle :

- $R_9$ représente un groupement $CH_3(CH_2)_p-$ non substitué ou substitué sur le groupement méthyle terminal par un groupement hydroxy, mercapto, phényle ou éthynyle et dans lequel l'un des groupements méthylène peut être remplacé par un atome d'oxygène, de soufre, un groupement p.phénylène, un groupement $-CH(CH_3)-$ ou un groupement $-C(CH_3)_2-$,
- B représente un atome d'oxygène ou de soufre,
- M représente un métal choisi parmi sodium, potassium ou césium,
- p est tel que la somme m + p est inférieure ou égale à n-1,

pour conduire, après déprotection éventuelle à un composé de formule (I/b), cas particulier des

composés de formule (I) :

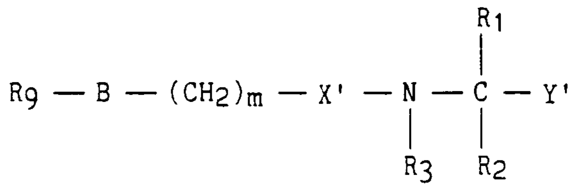 (I/b)

dans laquelle $R_9$, B, m, X', $R_1$, $R_2$, $R_3$ et Y' sont définis comme précédemment, dérivés de formule (I/a) et (I/b), que l'on peut écrire de manière simplifié :

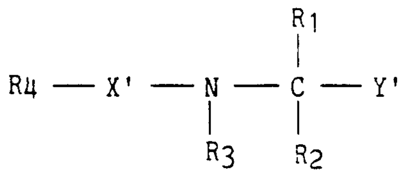

qui

a dans le cas où X' représente

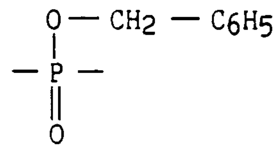

peuvent être transformés par hydrogénation catalytique, en composés de formule (I/c), cas particulier des composés de formule (I) :

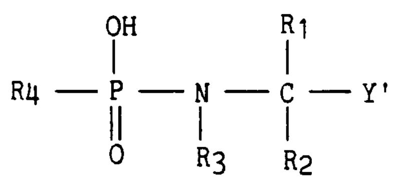 (I/c)

dans laquelle $R_1$, $R_2$, $R_3$, $R_4$ et Y' ont la même signification que précédemment,

b dans le cas où Y' représente un groupement -CO(alkoxy) ou -PO-(alkoxy)$_2$ peuvent être saponifiés de manière totale ou partielle pour conduire respectivement aux composés de formules (I/d), (I/e) et (I/f), cas particulier des composés de formule (I) :

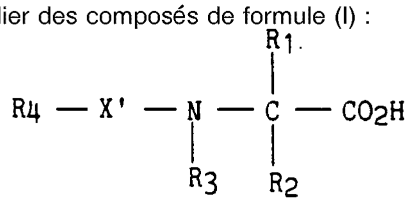 (I/d)

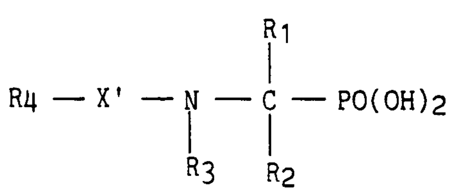 (I/e)

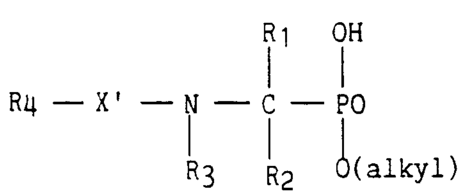 (I/f)

dans lesquelles $R_1$, $R_2$, $R_3$, $R_4$ et X' ont la même signification que précédemment,
c dans le cas où Y' représente un groupement $-CO_2H$ peuvent être transformés, en présence de carbonate de césium à l'aide de chloroacétamide, en composé de formule (I/g), cas particulier des composés de formule (I) :

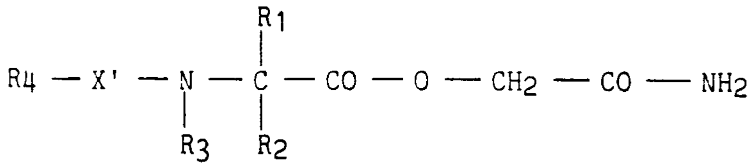
(I/g)

dans laquelle $R_1$, $R_2$, $R_3$, $R_4$ et X' ont la même signification que précédemment,
d dans le cas où Y' représente un groupement $-CO_2H$,
peuvent être estérifiés à l'aide d'isopropylidène glycérol, en composé de formule (I/h), cas particulier des composés de formule (I),

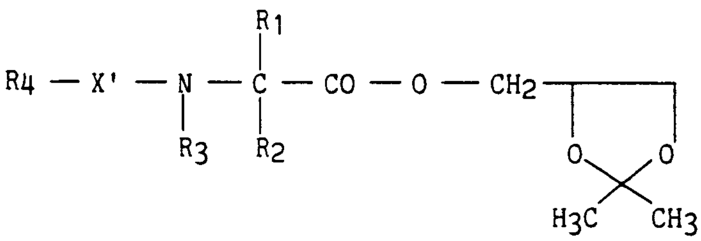
(I/h)

dans laquelle $R_1$, $R_2$, $R_3$ et $R_4$ ont la même signification que précédemment,
dérivé de formule (I/h) qui peut être hydrolysé en milieu acide pour conduire au composé de formule (I/i), cas particulier des composés de formule (I)

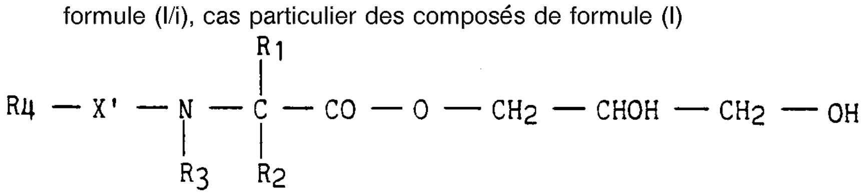
(I/i)

dans laquelle $R_1$, $R_2$, $R_3$ et $R_4$ ont la même signification que précédemment,
composés de formules (I/a) à (I/i) qui peuvent être purifiés, le cas échéant, par une technique classique de purification, dont les isomères peuvent être séparés selon une technique classique de séparation, et que l'on peut transformer en leurs sels d'addition à un acide ou à une base pharmaceutiquement acceptable.

**7.** Compositions pharmaceutiques contenant comme principe actif au moins un composé selon l'une quelconque des revendications 1 à 5, seul ou en combinaison avec un ou plusieurs véhicules inertes, non toxiques pharmaceutiquement acceptables.

**8.** Compositions pharmaceutiques selon la revendication 7 utile pour le traitement du cancer et/ou des maladies virales dont la maturation implique une myristoylation comme le SIDA, l'herpes, l'hépatite B, l'influenza, la polyomyélite ou les leucémies.

**Revendications pour l'Etat contractant suivant : ES**

**1.** Procédé de préparation des composés de formule générale (I)

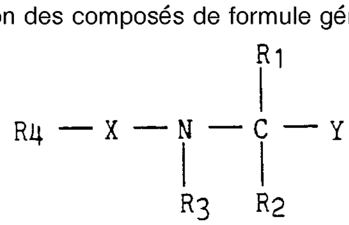
(I)

dans laquelle :

$R_1$ représente

- un atome d'hydrogène,
- un groupement alkyle ($C_1$-$C_6$) linéaire ou ramifié non substitué ou substitué par un ou plusieurs groupements hydroxy, amino, carboxy, carbamoyle, benzylthio, méthylthio, mercapto ou phényle (non substitué ou substitué par un ou plusieurs atomes d'halogène ou groupements hydroxy, alkyle ($C_1$-$C_6$) linéaire ou ramifié, alkoxy ($C_1$-$C_6$) linéaire ou ramifié ou $(CH_3\text{-}CH_2\text{-}O)_2PO\text{-}CH_2\text{-}$),
- un groupement phényle non substitué ou substitué par un ou plusieurs atomes d'halogène ou groupements hydroxy ou alkyle ($C_1$-$C_6$) linéaire ou ramifié,
- un groupement cycloalkyle ($C_3$-$C_7$) méthyle,
- un groupement (imidazol-2-yl) méthyle ou un groupement (indol-3-yl) méthyle non substitué ou substitué sur l'hétérocycle par un groupement benzyle, benzhydryle, trityle, benzyloxyméthyle, tosyle, alkyle ($C_1$-$C_6$) linéaire ou ramifié ou phényle,
- un groupement (1-azaindolizin-2-yl) méthyle de formule :

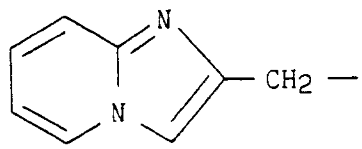

$R_2$ représente un atome d'hydrogène ou un groupement alkyle ($C_1$-$C_6$) linéaire ou ramifié,

$R_3$ représente un atome d'hydrogène ou un groupement alkyle ($C_1$-$C_6$) linéaire ou ramifié,

ou bien

lorsque $R_1$ représente un atome d'hydrogène,

$R_2$ et $R_3$ peuvent former avec les atomes de carbone et d'azote auxquels ils sont respectivement attachés, l'un quelconque des hétérocyles suivants :

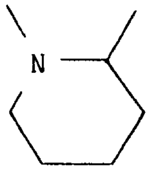 , 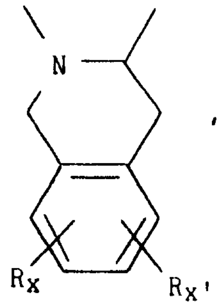 , 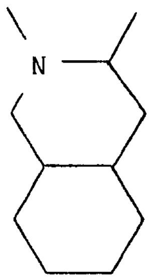 , 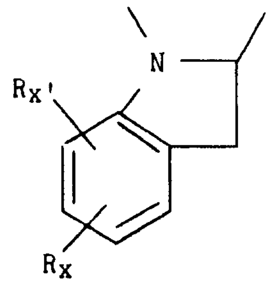 ,

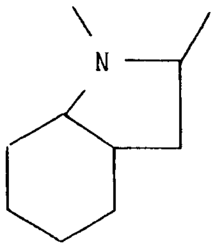 , 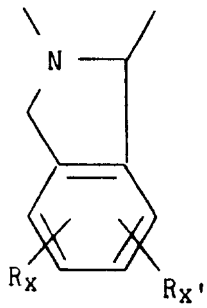 , 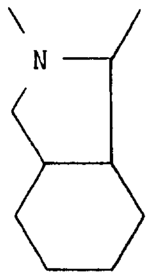 , 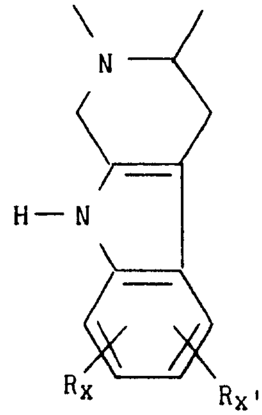

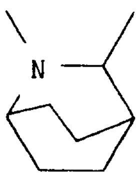 , 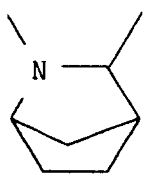 , 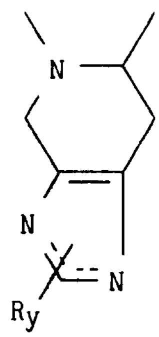

$R_x$ et $R_{x'}$ identiques ou différents représentent un atome d'hydrogène, un groupement alkyle ($C_1$-$C_6$) linéaire ou ramifié, un groupement hydroxy, un groupement alkoxy ($C_1$-$C_6$) linéaire ou ramifié, un atome d'halogène,

ou bien $R_x$ et $R_{x'}$, lorsqu'ils sont situés sur deux carbones adjacents, forment un groupement méthylènedioxy ou éthylènedioxy,

Ry représente un atome d'hydrogène, un groupement alkyle ($C_1$-$C_6$) linéaire ou ramifié, un groupement aryle, un groupement aralkyle, un groupement aroyle, un groupement arylsulfonyl,

X représente l'un quelconque des groupements suivants :

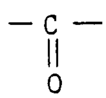 ,

$-SO_2-$ ,

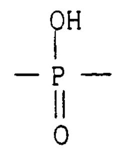 ,

Y représente un groupement — CO — $R_5$ ou

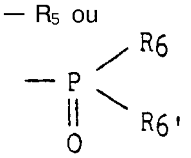

$R_5$ représente un groupement hydroxy, alkoxy ($C_1$-$C_6$) linéaire ou ramifié, $H_2N$-CO-$CH_2$-O-, HO-$CH_2$-CHOH-$CH_2$-O-,

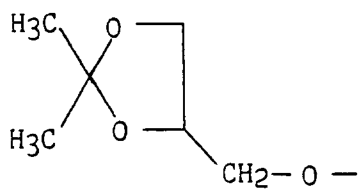 ,

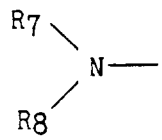 ,

$R_7$ et $R_8$ identiques ou différents représentent un atome d'hydrogène, un groupement alkyle ($C_1$-$C_6$) linéaire ou ramifié ou forment avec l'atome d'azote auxquels ils sont attachés un cycle pyrrolidine, pipéridine, morpholine ou pipérazine.

$R_6$ et $R_{6'}$ identiques ou différents représentent un atome d'hydrogène, un groupement hydroxy ou alkoxy ($C_1$-$C_6$) linéaire ou ramifié,

$R_4$ représente :

1 un groupement alkyle linéaire ou ramifié comportant de 6 à 21 atomes de carbone non substitué ou substitué sur le groupement méthyle terminal par un groupement hydroxy, mercapto, phényle ou éthynyle et dans lequel l'un au moins des groupements méthylènes est remplacé par un atome d'oxygène, de soufre ou un noyau p-phénylène,

dans le cas où :

$R_1$ représente

- un atome d'hydrogène,
- un groupement alkyle ($C_1$-$C_6$) linéaire ou ramifié non substitué ou substitué par un ou plusieurs groupements hydroxy, amino, carboxy, carbamoyle, benzylthio, méthylthio, mercapto ou phényle (non substitué ou substitué par un groupement hydroxy, ou alkoxy ($C_1$-$C_6$) linéaire ou ramifié),
- un groupement phényle non substitué,
- un groupement (imidazol-2-yl) méthyle ou un groupement (indol-3-yl) méthyle non substitué ou substitué sur l'hétérocycle par un groupement méthyle

et

$R_2$ = H, $R_3$ = H,

X = 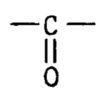 ,

Y = CO $R_{5'}$ ($R_{5'}$ = OH, alkoxy)

2 Dans les autres cas :

un groupement alkyle linéaire ou ramifié comportant de 6 à 21 atomes de carbone non substitué ou substitué sur le groupement méthyle terminal par un groupement hydroxy, mercapto, phényle ou éthynyle et dans lequel un ou plusieurs

groupements méthylènes peuvent être remplacés par un atome d'oxygène, de soufre ou un noyau p-phénylène,

(à la condition que lorsque $R_2$ ou $R_3$ forment avec les atomes d'azote ou de carbone auxquels ils sont attachés un cycle 1,2,3,4-tétrahydroisoquinoléine, indoline ou isoindoline alors $R_4$ est différent du groupement : alk-S-$(CH_2)_n$- dans lequel : n est égal à 1,2,3,4 ou 5 et alk représente un groupement alkyle ($C_4$-$C_{19}$) linéaire ou ramifié),

caractérisé :

1- dans le cas où les dérivés de formule (I) que l'on souhaite obtenir possèdent un radical $R_4$ = $R_{4'}$ dans lequel aucun groupement méthylène n'est remplacé par un atome de soufre ou d'oxygène, en ce que l'on condense un composé de formule (II) :

$R_{4'}$ - X' - Z (II)

dans laquelle :

X' représente un groupement

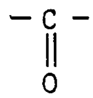 ,

— $SO_2$ — ,

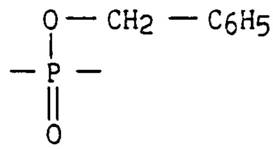 ,

$R_{4'}$ représente un groupement alkyle linéaire ou ramifié comportant de 6 à 21 atomes de carbone, non substitué ou substitué sur le groupement méthyle terminal par un groupement hydroxy, mercapto, phényle ou éthynyle, et dans lequel un ou plusieurs groupements méthylène peuvent être remplacés par un groupement p.phénylène,

Z représente un atome d'halogène, un groupement hydroxy, un groupement alkoxy ($C_1$-$C_6$) linéaire ou ramifié, un groupement aralkoxy, ou un groupement

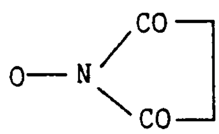 ,

sur une amine de formule (III) (sous forme racémique ou isomérique) protégée le cas échéant :

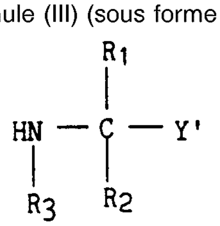

(III)

dans laquelle $R_2$, $R_3$ et $R_4$ ont la même signification que dans la formule I et Y' représente un groupement $-CO_2H$, -CO(alkoxy) ou $-PO(alkoxy)_2$,

pour conduire après déprotection éventuelle à un composé de formule (I/a), cas particulier des composés de formule (I)

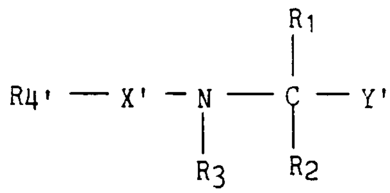

(I/a)

dans laquelle $R_1$, $R_2$, $R_3$, $R_{4'}$, X' et Y' ont la même signification que précédemment,

2- dans le cas où les dérivés de formule (I) que l'on souhaite obtenir possèdent un radical $R_4 = R_{4''} = CH_3-(CH_2)_n-$ dans lequel l'un au moins des groupements méthylène est remplacé par un atome de soufre ou d'oxygène,

en ce que l'on condense :

un composé de formule (IV)

$A — (CH_2)_m — X' — Z$ (IV)

dans laquelle :

- X' et Z : ont la même signification que précédemment,
- A : représente un atome d'halogène, un groupement mésyloxy ou un groupement tosyloxy,
- m : est inférieur ou égal à n-1,

et l'un des groupements méthylène peut être remplacé par un atome d'oxygène, de soufre, un groupement p.phénylène, un groupement $-CH(CH_3)-$ou un groupement $-C(CH_3)_2-$,

sur une amine de formule (III) (sous forme racémique ou isomérique) protégée le cas échéant, telle que définie précédemment,

pour conduire à un composé de formule (V)

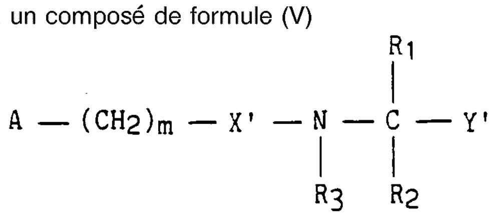

(V)

dans laquelle A, m, X', $R_1$, $R_2$, $R_3$ et Y' sont définis comme précédemment,

qui est mis en réaction avec un dérivé de formule (VI) : $R_9 — B — M$ (VI)

dans laquelle :

- $R_9$ : représente un groupement $CH_3-(CH_2)_p-$ non substitué ou substitué sur le groupement méthyle terminal par un groupement hydroxy, mercapto, phényle ou éthynyle et dans lequel l'un des groupements méthylène peut être remplacé par un atome d'oxygène, de soufre, un groupement p.phénylène, un groupement $-CH(CH_3)-$ ou un groupement $-C(CH_3)_2-$,
- B : représente un atome d'oxygène ou de soufre,
- M : représente un métal choisi parmi sodium, potassium ou césium,
- p : est tel que la somme m + p est inférieure ou égale à n-1,

pour conduire, après déprotection éventuelle à un composé de formule (I/b), cas particulier des composés de formule (I) :

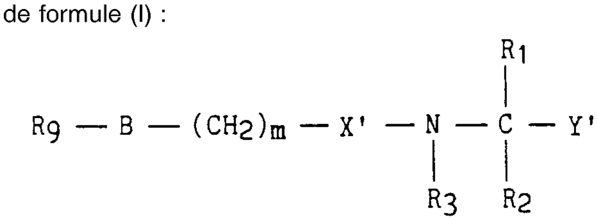

(I/b)

dans laquelle $R_9$, B, m, X', $R_1$, $R_2$, $R_3$ et Y' sont définis comme précédemment,

dérivés de formule (I/a) et (I/b), que l'on peut écrire de manière simplifié :

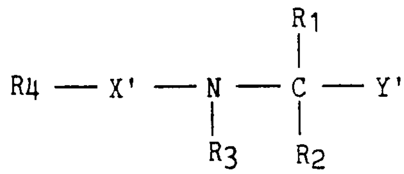

qui

a dans le cas où X' représente

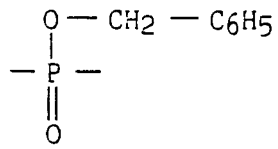

peuvent être transformés par hydrogénation catalytique, en composés de formule (I/c), cas particulier des composés de formule (I) :

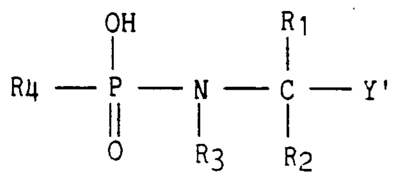

(I/c)

dans laquelle $R_1$, $R_2$, $R_3$, $R_4$ et Y' ont la même signification que précédemment,

b dans le cas où Y' représente un groupement -CO(alkoxy) ou -PO-(alkoxy)$_2$ peuvent être saponifiés de manière totale ou partielle pour conduire respectivement aux composés de formules (I/d), (I/e) et (I/f), cas particulier des composés de formule (I) :

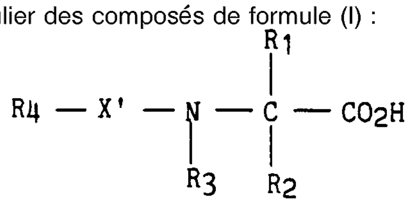

(I/d)

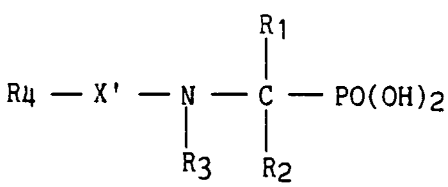

(I/e)

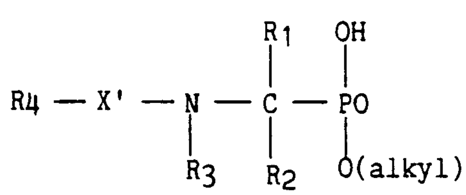

(I/f)

dans lesquelles $R_1$, $R_2$, $R_3$, $R_4$ et X' ont la même signification que précédemment,

c dans le cas où Y' représente un groupement -$CO_2H$ peuvent être transformés, en présence de carbonate de césium à l'aide de chloroacétamide, en composé de formule (I/g), cas particulier des composés de formule (I) :

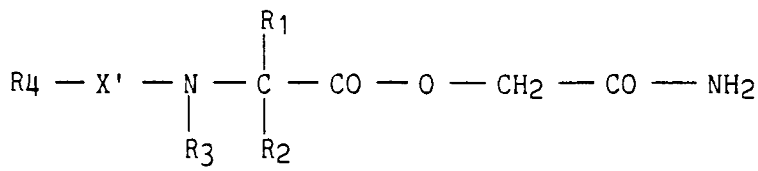

(I/g)

dans laquelle $R_1$, $R_2$, $R_3$, $R_4$ et X' ont la même signification que précédemment,

d dans le cas où Y' représente un groupement $-CO_2H$,

peuvent être estérifiés à l'aide d'isopropylidène glycérol, en composé de formule (I/h), cas particulier des composés de formule (I)

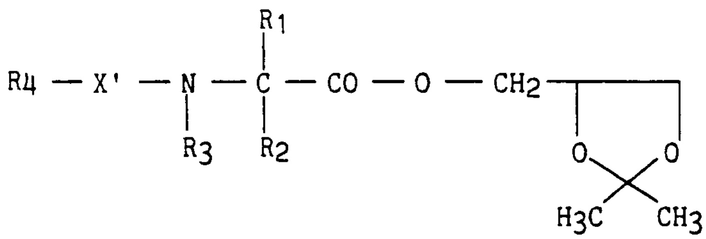

(I/h)

dans laquelle $R_1$, $R_2$, $R_3$ et $R_4$ ont la même signification que précédemment,

dérivé de formule (I/h) qui peut être hydrolysé en milieu acide pour conduire au composé de formule (I/i), cas particulier des composés de formule (I)

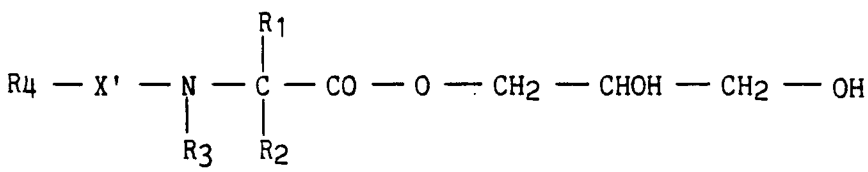

(I/i)

dans laquelle $R_1$, $R_2$, $R_3$ et $R_4$ ont la même signification que précédemment,

composés de formules (I/a) à (I/i) qui peuvent être purifiés, le cas échéant, par une technique classique de purification, dont les isomères peuvent être séparés selon une technique classique de séparation, et que l'on peut transformer en leurs sels d'addition à un acide ou à une base pharmaceutiquement acceptable.

**2.** Procédé de préparation selon la revendication 1 de la N-myristoyl-3-carboxy-(1,2,3,4)-tétrahydroisoquinoléine, de ses énantiomères ainsi que ses sels d'addition à une base pharmaceutiquement acceptable.

**3.** Procédé de préparation selon la revendication 1 de la N-myristoyl-($N^{\tau}$-benzyl)-histidine, de ses énantiomères ainsi que ses sels d'addition à un acide ou à une base pharmaceutiquement acceptable.

**4.** Procédé de préparation selon la revendication 1 du 1-myristoylamino-2-phényléthanehydrogénophosphonate d'éthyle, de ses énantiomères ainsi que ses sels d'addition à une base pharmaceutiquement acceptable.

**5.** Composé selon la revendication 1 qui est la N-(12-méthoxylauroyl)-phénylalanine, ses énantiomères ainsi que ses sels d'addition à une base pharmaceutiquement acceptable.

**Revendications pour l'Etat contractant suivant : GR**

**1.** Procédé de préparation des composés de formule générale (I)

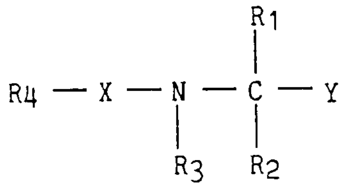

(I)

dans laquelle :

$R_1$ représente

- un atome d'hydrogène,
- un groupement alkyle ($C_1$-$C_6$) linéaire ou ramifié non substitué ou substitué par un ou plusieurs groupements hydroxy, amino, carboxy, carbamoyle, benzylthio, méthylthio, mercapto ou phényle (non substitué ou substitué par un ou plusieurs atomes d'halogène ou groupements hydroxy, alkyle ($C_1$-$C_6$) linéaire ou ramifié, alkoxy ($C_1$-$C_6$) linéaire ou ramifié ou $(CH_3$-$CH_2$-$O)_2PO$-$CH_2$-),
- un groupement phényle non substitué ou substitué par un ou plusieurs atomes d'halogène ou groupements hydroxy ou alkyle ($C_1$-$C_6$) linéaire ou ramifié,
- un groupement cycloalkyle ($C_3$-$C_7$) méthyle,
- un groupement (imidazol-2-yl) méthyle ou un groupement (indol-3-yl) méthyle non substitué ou substitué sur l'hétérocycle par un groupement benzyle, benzhydryle, trityle, benzyloxyméthyle, tosyle, alkyle ($C_1$-$C_6$) linéaire ou ramifié ou phényle,
- un groupement (1-azaindolizin-2-yl) méthyle de formule :

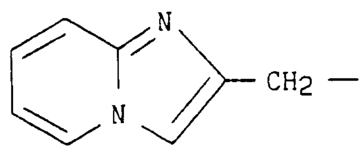

$R_2$ représente un atome d'hydrogène ou un groupement alkyle ($C_1$-$C_6$) linéaire ou ramifié,

$R_3$ représente un atome d'hydrogène ou un groupement alkyle ($C_1$-$C_6$) linéaire ou ramifié,

ou bien

lorsque $R_1$ représente un atome d'hydrogène,

$R_2$ et $R_3$ peuvent former avec les atomes de carbone et d'azote auxquels ils sont respectivement attachés, l'un quelconque des hétérocyles suivants :

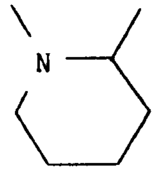 , 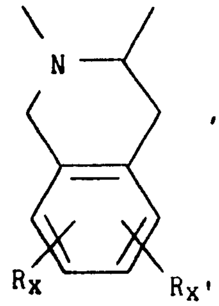 , 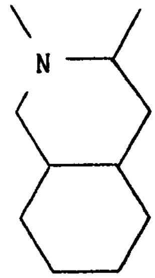 , 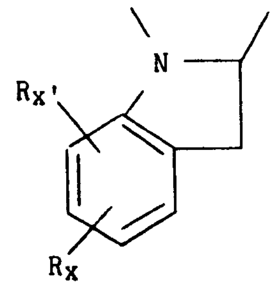 ,

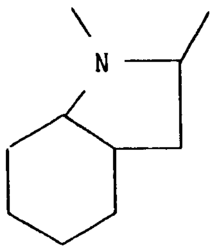 , 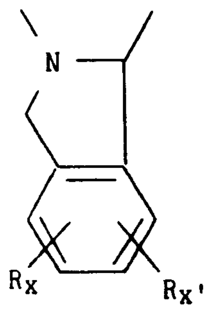 , 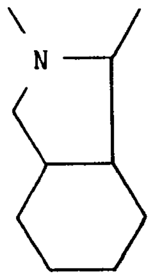 , 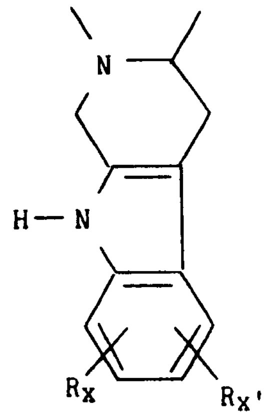

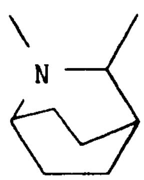 , 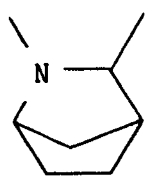 , 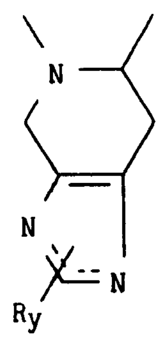

$R_x$ et $R_{x'}$ identiques ou différents représentent un atome d'hydrogène, un groupement alkyle ($C_1$-$C_6$) linéaire ou ramifié, un groupement hydroxy, un groupement alkoxy ($C_1$-$C_6$) linéaire ou ramifié, un atome d'halogène,

ou bien $R_x$ et $R_{x'}$, lorsqu'ils sont situés sur deux carbones adjacents, forment un groupement méthylènedioxy ou éthylènedioxy,

Ry représente un atome d'hydrogène, un groupement alkyle ($C_1$-$C_6$) linéaire ou ramifié, un groupement aryle, un groupement aralkyle, un groupement aroyle, un groupement arylsulfonyl,

X représente l'un quelconque des groupements suivants :

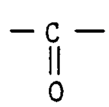 ,

$-SO_2-$ ,

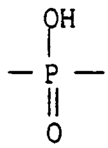,

Y représente un groupement — CO — $R_5$ ou

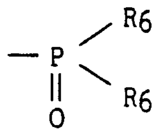

$R_5$ représente un groupement hydroxy, alkoxy ($C_1$-$C_6$) linéaire ou ramifié, $H_2N$-CO-$CH_2$-O-, HO-$CH_2$-CHOH-$CH_2$-O-,

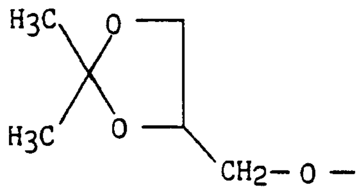, 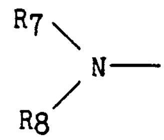,

$R_7$ et $R_8$ identiques ou différents représentent un atome d'hydrogène, un groupement alkyle ($C_1$-$C_6$) linéaire ou ramifié ou forment avec l'atome d'azote auxquels ils sont attachés un cycle pyrrolidine, pipéridine, morpholine ou pipérazine.

$R_6$ et $R_{6'}$ identiques ou différents représentent un atome d'hydrogène, un groupement hydroxy ou alkoxy ($C_1$-$C_6$) linéaire ou ramifié,

$R_4$ représente :

1 un groupement alkyle linéaire ou ramifié comportant de 6 à 21 atomes de carbone non substitué ou substitué sur le groupement méthyle terminal par un groupement hydroxy, mercapto, phényle ou éthynyle et dans lequel l'un au moins des groupements méthylènes est remplacé par un atome d'oxygène, de soufre ou un noyau p-phénylène,

dans le cas où :

$R_1$ représente

- un atome d'hydrogène,
- un groupement alkyle ($C_1$-$C_6$) linéaire ou ramifié non substitué ou substitué par un ou plusieurs groupements hydroxy, amino, carboxy, carbamoyle, benzylthio, méthylthio, mercapto ou phényle (non substitué ou substitué par un groupement hydroxy, ou alkoxy ($C_1$-$C_6$) linéaire ou ramifié),
- un groupement phényle non substitué,
- un groupement (imidazol-2-yl) méthyle ou un groupement (indol-3-yl) méthyle non substitué ou substitué sur l'hétérocycle par un groupement méthyle

et

$R_2$ = H, $R_3$ = H,

X = 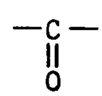,

Y = CO $R_{5'}$ ($R_{5'}$ = OH, alkoxy)

2 Dans les autres cas :

un groupement alkyle linéaire ou ramifié comportant de 6 à 21 atomes de carbone non substitué ou substitué sur le groupement méthyle terminal par un groupement hydroxy, mercapto, phényle ou èthynyle et dans lequel un ou plusieurs groupements méthylènes peuvent être remplacés par un atome d'oxygène, de soufre ou un noyau p-phénylène,

(à la condition que lorsque $R_2$ ou $R_3$ forment avec les atomes d'azote ou de carbone auxquels ils sont attachés un cycle 1,2,3,4-tétrahydroisoquinoléine, indoline ou isoindoline alors $R_4$ est différent du groupement : alk-S-$(CH_2)_n$- dans lequel : n est égal à 1,2,3,4 ou 5 et alk représente un groupement alkyle ($C_4$-$C_{19}$) linéaire ou ramifié),

caractérisé :

1- dans le cas où les dérivés de formule (I) que l'on souhaite obtenir possèdent un radical $R_4$ = $R_{4'}$ dans lequel aucun groupement méthylène n'est remplacé par un atome de soufre ou d'oxygène, en ce que l'on condense un composé de formule (II) :

$R_{4'}$ - X' - Z (II)

dans laquelle :

X' représente un groupé

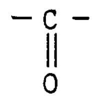 ,

— $SO_2$ — ,

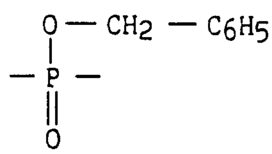 ,

$R_{4'}$ représente un groupement alkyle linéaire ou ramifié comportant de 6 à 21 atomes de carbone, non substitué ou substitué sur le groupement méthyle terminal par un groupement hydroxy, mercapto, phényle ou éthynyle, et dans lequel un ou plusieurs groupements méthylène peuvent être remplacés par un groupement p.phénylène,

Z représente un atome d'halogène, un groupement hydroxy, un groupement alkoxy ($C_1$-$C_6$) linéaire ou ramifié, un groupement aralkoxy, ou un groupement

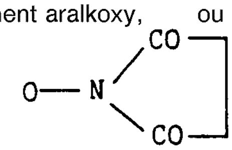 ,

sur une amine de formule (III) (sous forme racémique ou isomérique) protégée le cas échéant :

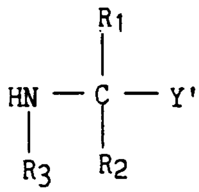

(III)

dans laquelle $R_2$, $R_3$ et $R_4$ ont la même signification que dans la formule I et Y' représente un groupement $-CO_2H$, -CO(alkoxy) ou $-PO(alkoxy)_2$,

pour conduire après déprotection éventuelle à un composé de formule (I/a), cas particulier des composés de formule (I)

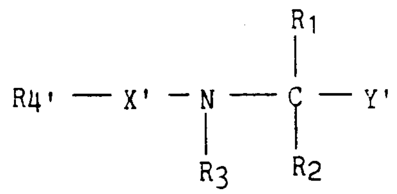

(I/a)

dans laquelle $R_1$, $R_2$, $R_3$, $R_{4'}$, X' et Y' ont la même signification que précédemment,

2- dans le cas où les dérivés de formule (I) que l'on souhaite obtenir possèdent un radical $R_4 = R_{4''} = CH_3$-$(CH_2)_n$- dans lequel l'un au moins des groupements méthylène est remplacé par un atome de soufre ou d'oxygène,

en ce que l'on condense :

un composé de formule (IV)

A — $(CH_2)_m$ — X' — Z  (IV)

dans laquelle :

- X' et Z  ont la même signification que précédemment,
- A  représente un atome d'halogène, un groupement mésyloxy ou un groupement tosyloxy,
- m  est inférieur ou égal à n-1,

et l'un des groupements méthylène peut être remplacé par un atome d'oxygène, de soufre, un groupement p.phénylène, un groupement -CH($CH_3$)-ou un groupement -C($CH_3$)$_2$-,

sur une amine de formule (III) (sous forme racémique ou isomérique) protégée le cas échéant, telle que définie précédemment,

pour conduire à un composé de formule (V)

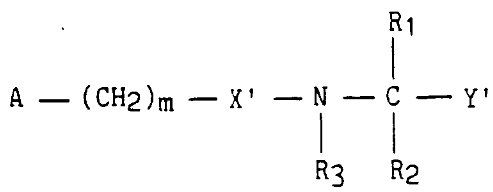

(V)

dans laquelle A, m, X' , $R_1$, $R_2$, $R_3$ et Y' sont définis comme précédemment,

qui est mis en réaction avec un dérivé de formule (VI) :

$R_9$ — B — M  (VI)

dans laquelle :

- $R_9$  représente un groupement $CH_3$-$(CH_2)_p$- non substitué ou substitué sur le groupement méthyle terminal par un groupement hydroxy, mercapto, phényle ou éthynyle et dans lequel l'un des groupements méthylène peut être remplacé par un atome d'oxygène, de soufre, un groupement p.phénylène, un groupement -CH($CH_3$)- ou un groupement -C-($CH_3$)$_2$-,
- B  représente un atome d'oxygène ou de soufre,
- M  représente un métal choisi parmi sodium, potassium ou césium,
- p  est tel que la somme m + p est inférieure ou égale à n-1,

pour conduire, après déprotection éventuelle à un composé de formule (I/b), cas particulier des composés de formule (I) :

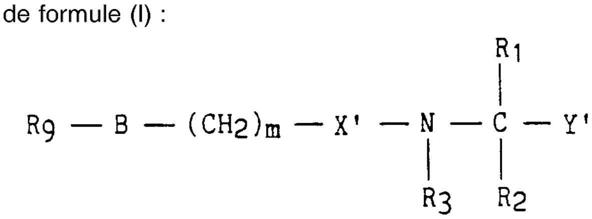

(I/b)

dans laquelle $R_9$, B, m, X', $R_1$, $R_2$, $R_3$ et Y' sont définis comme précédemment, dérivés de formule (I/a) et (I/b), que l'on peut écrire de manière simplifié :

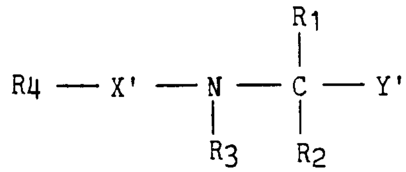

qui

a dans le cas où X' représente

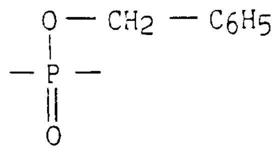

peuvent être transformés par hydrogénation catalytique, en composés de formule (I/c), cas particulier des composés de formule (I) :

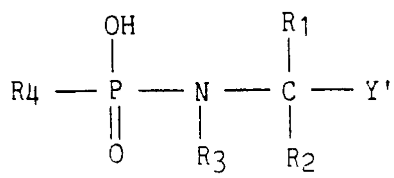
(I/c)

dans laquelle $R_1$, $R_2$, $R_3$, $R_4$ et Y' ont la même signification que précédemment,

b dans le cas où Y' représente un groupement -CO(alkoxy) ou $-PO-(alkoxy)_2$ peuvent être saponifiés de manière totale ou partielle pour conduire respectivement aux composés de formules (I/d), (I/e) et (I/f), cas particulier des composés de formule (I) :

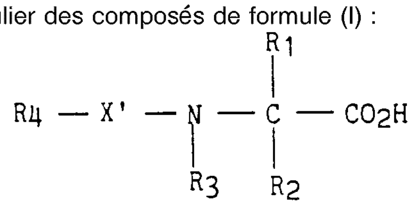
(I/d)

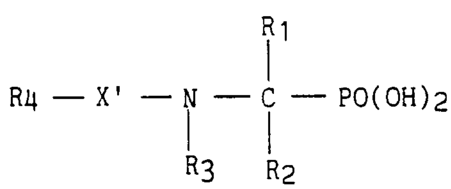
(I/e)

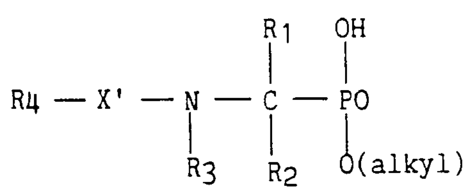
(I/f)

dans lesquelles $R_1$, $R_2$, $R_3$, $R_4$ et X' ont la même signification que précédemment,

c dans le cas où Y' représente un groupement $-CO_2H$ peuvent être transformés, en présence de carbonate de césium à l'aide de chloroacétamide, en composé de formule (I/g), cas particulier des composés de formule (I) :

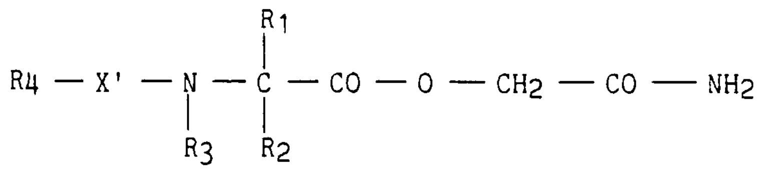

(I/g)

dans laquelle $R_1$, $R_2$, $R_3$, $R_4$ et X' ont la même signification que précédemment,

d dans le cas où Y' représente un groupement $-CO_2H$,

peuvent être estérifiés à l'aide d'isopropylidène glycérol, en composé de formule (I/h), cas particulier des composés de formule (I),

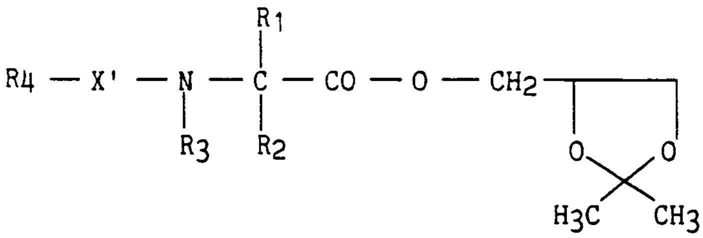

(I/h)

dans laquelle $R_1$, $R_2$, $R_3$ et $R_4$ ont la même signification que précédemment,

dérivé de formule (I/h) qui peut être hydrolysé en milieu acide pour conduire au composé de formule (I/i), cas particulier des composés de formule (I)

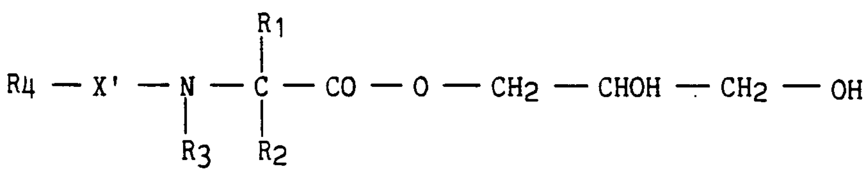

(I/i)

dans laquelle $R_1$, $R_2$, $R_3$ et $R_4$ ont la même signification que précédemment,

composés de formules (I/a) à (I/i) qui peuvent être purifiés, le cas échéant, par une technique classique de purification, dont les isomères peuvent être séparés selon une technique classique de séparation, et que l'on peut transformer en leurs sels d'addition à un acide ou à une base pharmaceutiquement acceptable.

2. Procédé de préparation selon la revendication 1 de la N-myristoyl-3-carboxy-(1,2,3,4)-tétrahydroisoquinoléine, de ses énantiomères ainsi que ses sels d'addition à une base pharmaceutiquement acceptable.

3. Procédé de préparation selon la revendication 1 de la N-myristoyl-(N$^{\tau}$-benzyl)-histidine, de ses énantiomères ainsi que ses sels d'addition à un acide ou à une base pharmaceutiquement acceptable.

4. Procédé de préparation selon la revendication 1 du 1-myristoylamino-2-phényléthanehydrogénophosphonate d'éthyle, de ses énantiomères ainsi que ses sels d'addition à une base pharmaceutiquement acceptable.

5. Composé selon la revendication 1 qui est la N-(12-méthoxylauroyl)-phénylalanine, ses énantiomères ainsi que ses sels d'addition à une base pharmaceutiquement acceptable.

## Claims

**Claims for the following Contracting States : AT, BE, CH, LI, DE, DK, FR, GB, IT, LU, NL, PT, SE**

1. Compounds of the general formula (I)

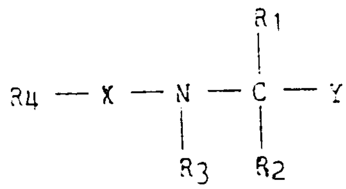

(I)

wherein :

$R_1$ represents

- a hydrogen atom,
- a straight-chain or branched ($C_1$-$C_6$) alkyl group that is unsubstituted or is substituted by one or more groups hydroxy, amino, carboxy, carbamoyl, benzylthio, methylthio, mercapto or phenyl (unsubstituted or substituted by one or more halogen atoms or hydroxy, straight-chain or branched ($C_1$-$C_6$) alkyl, straight-chain or branched ($C_1$-$C_6$) alkoxy or $(CH_3\text{-}CH_2\text{-}O)_2PO\text{-}CH_2$-groups),
- a phenyl group that is unsubstituted or is substituted by one or more halogen atoms or hydroxy or straight-chain or branched ($C_1$-$C_6$) alkyl groups,
- a ($C_3$-$C_7$) cycloalkyl-methyl group,
- an (imidazol-2-yl) methyl group or an (indol-3-yl)methyl group that is unsubstituted or is substituted on the heterocycle by a benzyl, benzhydryl, trityl, benzyloxymethyl, tosyl, straight-chain or branched ($C_1$-$C_6$) alkyl or phenyl group,
- a (1-azaindolizin-2-yl)methyl group of formula :

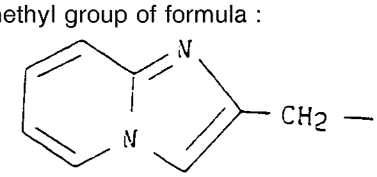

$R_2$ represents a hydrogen atom or a straight-chain or branched ($C_1$-$C_6$) alkyl group,

$R_3$ represents a hydrogen atom or a straight-chain or branched ($C_1$-$C_6$) alkyl group,

or

when $R_1$ represents a hydrogen atom,

$R_2$ and $R_3$ may form, with the carbon and nitrogen atoms to which they are respectively attached, any one of the following heterocycles:

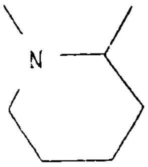 , 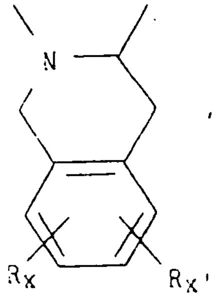 , 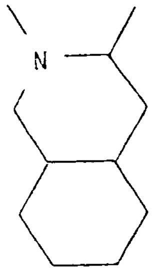 , 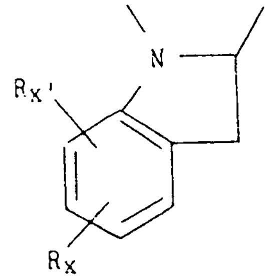 ,

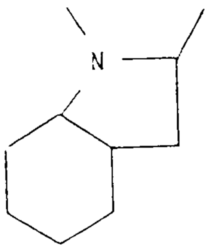 , 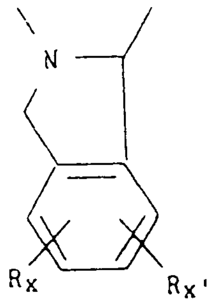 , 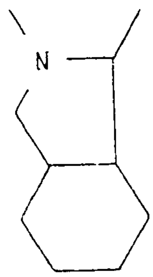 , 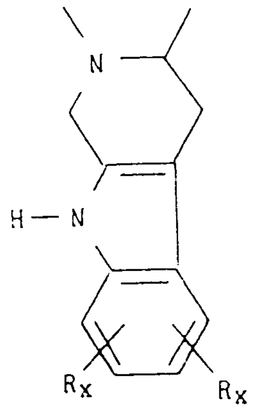

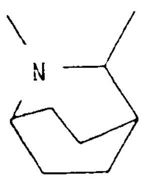 , 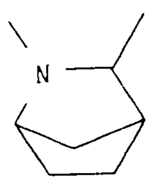 , 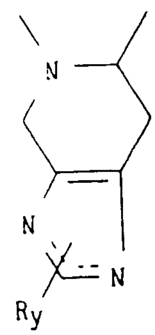

$R_x$ and $R_{x'}$, which are the same or different, each represents a hydrogen atom, a straight-chain or branched ($C_1$-$C_6$) alkyl group, a hydroxy group, a straight-chain or branched ($C_1$-$C_6$) alkoxy group or a halogen atom,

or $R_x$ and $R_{x'}$, when located at two adjacent carbon atoms, alternatively form a methylenedioxy or ethylenedioxy group,

Ry represents a hydrogen atom, a straight-chain or branched ($C_1$-$C_6$) alkyl group, an aryl group, an aralkyl group, an aroyl group or an arylsulphonyl group,

X represents any one of the following groups :

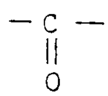 ,

$-SO_2-$ ,

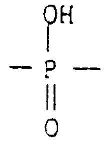 ,

Y represents a group $-CO-R_5$ or

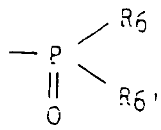 ,

$R_5$ represents a hydroxy group, a straight-chain or branched ($C_1$-$C_6$) alkoxy group, a $H_2N-CO-CH_2-O-$ group, a $HO-CH_2-CHOH-CH_2-O-$ group,

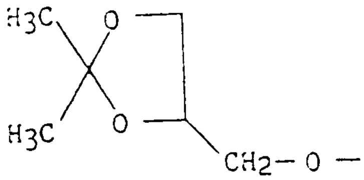 or 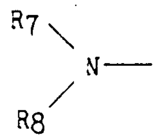 ,

$R_7$ and $R_8$, which are the same or different, each represents a hydrogen atom or a straight-chain or branched ($C_1$-$C_6$) alkyl group, or together with the nitrogen atom to which they are attached form a pyrrolidine, piperidine, morpholine or piperazine ring,

$R_6$ and $R_{6'}$, which are the same or different, each represents a hydrogen atom, a hydroxy group or a straight-chain or branched ($C_1$-$C_6$) alkoxy group,

$R_4$ represents :

1 a straight-chain or branched alkyl group containing from 6 to 21 carbon atoms that is unsubstituted or is substituted at the terminal methyl group by a hydroxy, mercapto, phenyl or ethynyl group and in which at least one of the methylene groups is replaced by an oxygen atom, a sulphur atom or a p-phenylene nucleus,

in the case where :

$R_1$ represents

- a hydrogen atom,
- a straight-chain or branched ($C_1$-$C_6$) alkyl group that is unsubstituted or is substituted by one or more groups hydroxy, amino, carboxy, carbamoyl, benzylthio, methylthio, mercapto or phenyl (unsubstituted or substituted by a hydroxy or straight-chain or branched ($C_1$-$C_6$)alkoxy group),
- an unsubstituted phenyl group,
- an (imidazol-2-yl) methyl group or an (indol-3-yl) methyl group that is unsubstituted or is substituted on the heterocycle by a methyl group

and

$R_2$ = H, $R_3$ = H,

X = 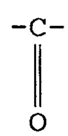 ,

Y = $COR_5$, ($R_5$, = OH, alkoxy),

2 in the other cases :

a straight-chain or branched alkyl group containing from 6 to 21 carbon atoms that is unsubstituted or is substituted at the terminal methyl group by a hydroxy, mercapto, phenyl or ethynyl group and in which one or more methylene groups may be replaced by an oxygen atom, a sulphur atom or a p-phenylene nucleus

(with the proviso that when $R_2$ or $R_3$, together with the nitrogen or carbon atoms to which they are attached, form a 1,2,3,4-tetrahydroisoquinoline, indoline or isoindoline ring then $R_4$ is other than the group alk-S-$(CH_2)_n$- in which n is 1, 2, 3, 4 or 5 and alk represents a straight-chain or branched ($C_4$-$C_{19}$) alkyl group),

their isomers, diastereoisomers and epimers, and also their addition salts with a pharmaceutically acceptable acid or base.

**2.** Compound according to claim 1, which is N-myristoyl-3-carboxy-(1,2,3,4)-tetrahydroisoquinoline, its enantiomers and also its addition salts with a pharmaceutically acceptable base.

**3.** Compound according to claim 1, which is N-myristoyl-($N'^{\tau}$-benzyl) histidine, its enantiomers and also its addition salts with a pharmaceutically acceptable acid or base.

**4.** Compound according to claim 1, which is ethyl 1-myristoylamino-2-phenylethanehydrogenphosphonate, its enantiomers and also its addition salts with a pharmaceutically acceptable base.

**5.** Compound according to claim 1, which is N-(12-methoxylauroyl)-phenylalanine, its enantiomers and also its addition salts with a pharmaceutically acceptable base.

**6.** Process for the preparation of compounds of formula (I) according to claim 1, characterised in that :

1- in the case where the compounds of formula (I) it is desired to obtain contain a radical $R_4 = R_{4'}$ in which no methylene group is replaced by a sulphur or oxygen atom,

a compound of formula (II) :

$R_{4'}$ - X' - Z (II)

wherein :

X' represents a group

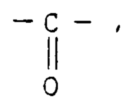

— $SO_2$ — ,

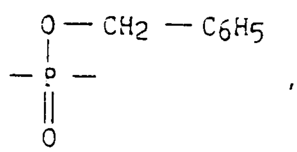

$R_{4'}$ represents a straight-chain or branched alkyl group containing from 6 to 21 carbon atoms that is unsubstituted or is substituted at the terminal methyl group by a hydroxy, mercapto, phenyl or ethynyl group, and in which one or more methylene groups may be replaced by a p-phenylene group,

Z represents a halogen atom, a hydroxy group, a straight-chain or branched ($C_1$-$C_6$) alkoxy group, an aralkoxy group or a group

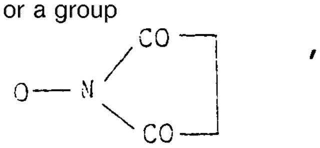

is condensed with an amine of formula (III) (in racemic or isomeric form), which amine is protected where appropriate :

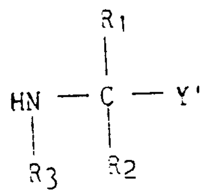

(III)

wherein $R_2$, $R_3$ and $R_4$ are as defined for formula I and Y' represents a group $-CO_2H$, -CO(alkoxy) or $-PO(alkoxy)_2$,
to yield, after deprotection where necessary, a compound of formula (I/a),
a particular instance of compounds of formula (I)

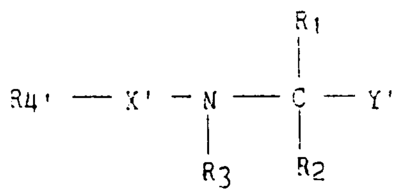

(Ia)

wherein $R_1$, $R_2$, $R_3$, $R_{4'}$, X' and Y' are as defined above,
2- in the case where the compounds of formula (I) it is desired to obtain contain a radical $R_4 = R_{4''} = CH_3-(CH_2)_n-$ in which at least one of the methylene groups is replaced by a sulphur or oxygen atom,
a compound of formula (IV)

$A-(CH_2)_m-X'-Z$  (IV)

wherein :

- X' and Z — are as defined above,
- A — represents a halogen atom, a mesyloxy group or a tosyloxy group,
- m — is less than or equal to n-1,

and one of the methylene groups may be replaced by an oxygen atom, a sulphur atom, a p-phenylene group, a $-CH(CH_3)-$ group or a $-C(CH_3)_2-$ group,
is condensed with an amine of formula (III) as defined above (in racemic or isomeric form), which amine is protected where appropriate,
to yield a compound of formula (V)

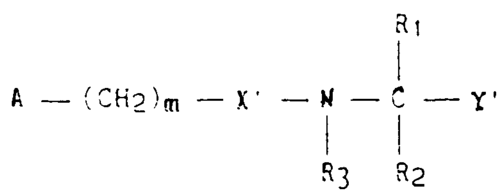

(V)

wherein A, m, X', $R_1$, $R_2$, $R_3$ and Y' are as defined above,
which is reacted with a compound of formula (VI) :

$R_9-B-M$  (VI)

wherein :

- $R_9$ — represents a $CH_3-(CH_2)_p-$ group that is unsubstituted or is substituted at the terminal methyl group by a hydroxy, mercapto, phenyl or ethynyl group and in which one of the methylene groups may be replaced by an oxygen atom, a sulphur atom, a p-phenylene group, a $-CH(CH_3)-$ group or a $-C(CH_3)_2-$ group,
- B — represents an oxygen or sulphur atom,
- M — represents a metal selected from sodium, potassium and caesium,
- p — is such that the sum of m and p is less than or equal to n-1,

to yield, after deprotection where appropriate, a compound of formula (I/b), a particular instance of compounds of formula (I) :

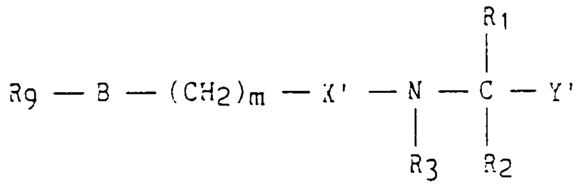

(I/b)

wherein $R_9$, B, m, X', $R_1$, $R_2$, $R_3$ and Y' are as defined above,
which compounds of formulae (I/a) and (I/b), which may be written in a simplified manner :

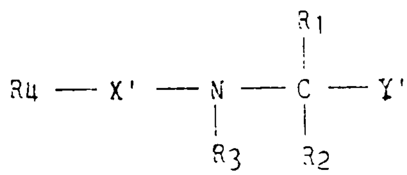

a in the case where X' represents

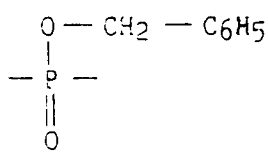

may be converted by catalytic hydrogenation into compounds of formula (I/c), a particular instance of compounds of formula (I) :

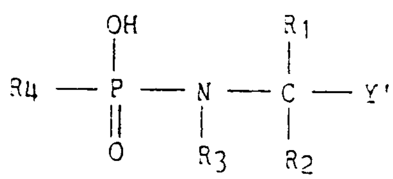

(I/c)

wherein $R_1$, $R_2$, $R_3$, $R_4$ and Y' are as defined above,
b in the case where Y' represents a -CO(alkoxy) or $-PO(alkoxy)_2$ group, may be totally or partially hydrolysed to yield, respectively, compounds of formulae (I/d), (I/e) and (I/f), a particular instance of compounds of formula (I) :

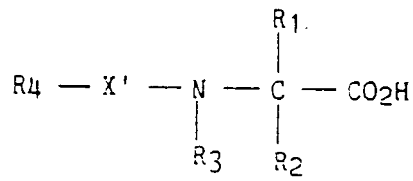
(I/d)

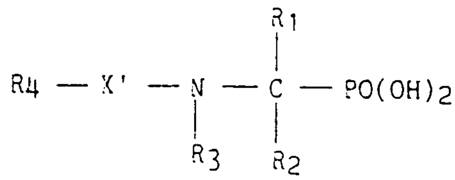
(I/e)

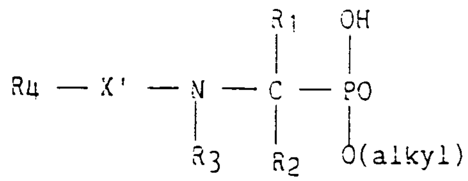
(I/f)

wherein $R_1$, $R_2$, $R_3$, $R_4$ and X' are as defined above,

c in the case where Y' represents a $-CO_2H$ group, may be converted with the aid of chloroacetamide, in the presence of caesium carbonate, into a compound of formula (I/g), a particular instance of compounds of formula (I) :

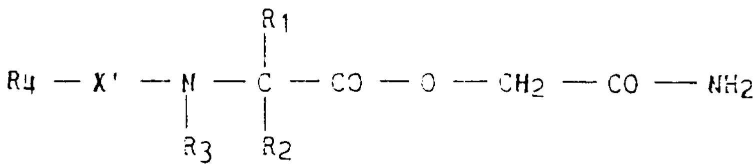
(I/g)

wherein $R_1$, $R_2$, $R_3$, $R_4$ and X' are as defined above,

d in the case where Y' represents a $-CO_2H$ group,

may be esterified with the aid of isopropylidene glycerol to form a compound of formula (I/h), a particular instance of compounds of formula (I)

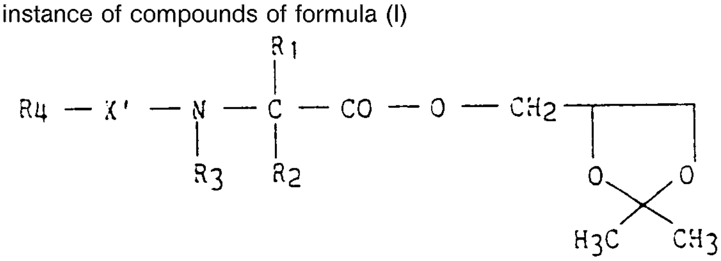
(I/h)

wherein $R_1$, $R_2$, $R_3$ and $R_4$ are as defined above,

which compound of formula (I/h) may be hydrolysed in acidic medium to yield a compound of formula (I/i), a particular instance of compounds of formula (I)

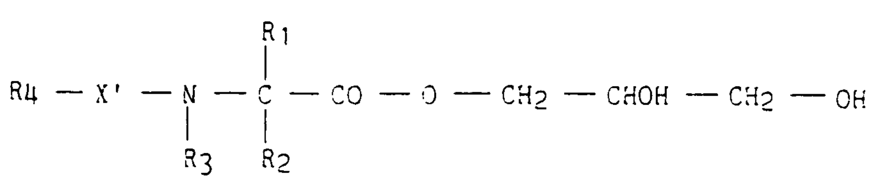
(I/i)

wherein $R_1$, $R_2$, $R_3$ and $R_4$ are as defined above,

which compounds of formulae (I/a) to (I/i) may be purified, where necessary, by a conventional method

of purification, may be separated into their isomers according to a conventional method of separation, and may be converted into their addition salts with a pharmaceutically acceptable acid or base.

7. Pharmaceutical compositions comprising as active ingredient at least one compound according to any one of claims 1 to 5, alone or in combination with one or more inert, non-toxic, pharmaceutically acceptable carriers.

8. Pharmaceutical compositions according to claim 7, for use in the treatment of cancer and/or viral diseases the development of which implicates a myristoylation, such as SIDA, herpes, hepatitis B, influenza, poliomyelitis or leukaemias.

**Claims for the following Contracting State : ES**

1. Process for the preparation of compounds of the general formula (I)

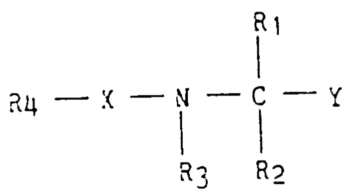

(I)

wherein :

$R_1$ represents
- a hydrogen atom,
- a straight-chain or branched ($C_1$-$C_6$)alkyl group that is unsubstituted or is substituted by one or more groups hydroxy, amino, carboxy, carbamoyl, benzylthio, methylthio, mercapto or phenyl (unsubstituted or substituted by one or more halogen atoms or hydroxy, straight-chain or branched ($C_1$-$C_6$)alkyl, straight-chain or branched ($C_1$-$C_6$)alkoxy or $(CH_3$-$CH_2$-$O)_2PO$-$CH_2$- groups),
- a phenyl group that is unsubstituted or is substituted by one or more halogen atoms or hydroxy or straight-chain or branched ($C_1$-$C_6$)alkyl groups,
- a ($C_3$-$C_7$)cycloalkyl-methyl group,
- an (imidazol-2-yl)methyl group or an (indol-3-yl)methyl group that is unsubstituted or is substituted on the heterocycle by a benzyl, benzhydryl, trityl, benzyloxymethyl, tosyl, straight-chain or branched ($C_1$-$C_6$)alkyl or phenyl group,
- a (1-azaindolizin-2-yl)methyl group of formula :

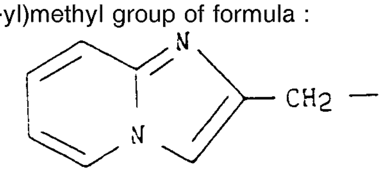

$R_2$ represents a hydrogen atom or a straight-chain or branched ($C_1$-$C_6$)alkyl group,

$R_3$ represents a hydrogen atom or a straight-chain or branched ($C_1$-$C_6$)alkyl group,

or

when $R_1$ represents a hydrogen atom,

$R_2$ and $R_3$ may form, with the carbon and nitrogen atoms to which they are respectively attached, any one of the following heterocycles:

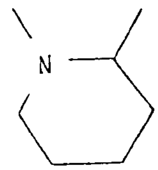 , 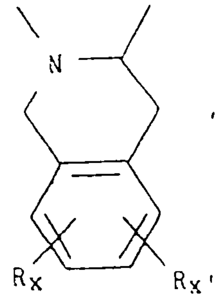 , 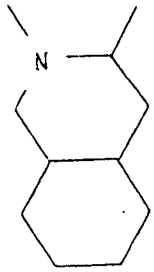 , 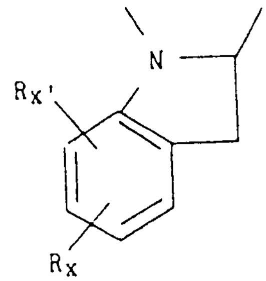 ,

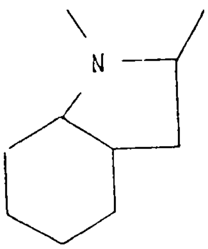 , 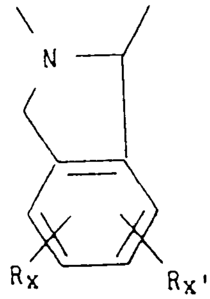 , 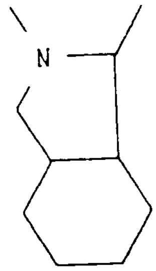 , 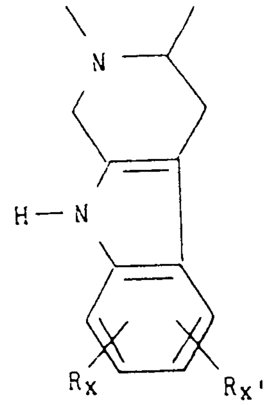

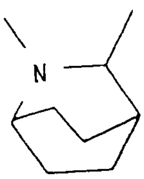 , 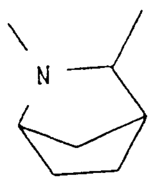 , 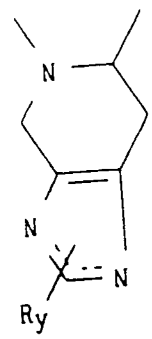

$R_x$ and $R_{x'}$, which are the same or different, each represents a hydrogen atom, a straight-chain or branched ($C_1$-$C_6$)alkyl group, a hydroxy group, a straight-chain or branched ($C_1$-$C_6$)-alkoxy group or a halogen atom,

or $R_x$ and $R_{x'}$, when located at two adjacent carbon atoms, alternatively form a methylenedioxy or ethylenedioxy group,

$R_y$ represents a hydrogen atom, a straight-chain or branched ($C_1$-$C_6$)alkyl group, an aryl group, an aralkyl group, an aroyl group or an arylsulphonyl group,

X represents any one of the following groups :

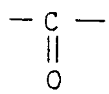 ,

$-SO_2-$ ,

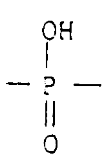 ,

Y represents a group $-CO-R_5$ or

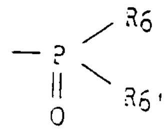

,

$R_5$ represents a hydroxy group, a straight-chain or branched $(C_1-C_6)$alkoxy group, a $H_2N-CO-CH_2-O-$ group, a $HO-CH_2-CHOH-CH_2-O-$ group,

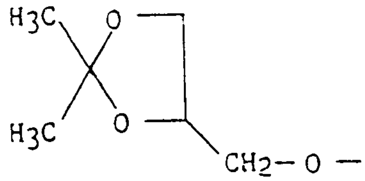

or

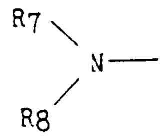

,

$R_7$ and $R_8$, which are the same or different, each represents a hydrogen atom or a straight-chain or branched $(C_1-C_6)$alkyl group, or together with the nitrogen atom to which they are attached form a pyrrolidine, piperidine, morpholine or piperazine ring,

$R_6$ and $R_{6'}$, which are the same or different, each represents a hydrogen atom, a hydroxy group or a straight-chain or branched $(C_1-C_6)$alkoxy group,

$R_4$ represents :

1 a straight-chain or branched alkyl group containing from 6 to 21 carbon atoms that is unsubstituted or is substituted at the terminal methyl group by a hydroxy, mercapto, phenyl or ethynyl group and in which at least one of the methylene groups is replaced by an oxygen atom, a sulphur atom or a p-phenylene nucleus,

in the case where :

$R_1$ represents

- a hydrogen atom,
- a straight-chain or branched $(C_1-C_6)$alkyl group that is unsubstituted or is substituted by one or more groups hydroxy, amino, carboxy, carbamoyl, benzylthio, methylthio, mercapto or phenyl (unsubstituted or substituted by a hydroxy or straight-chain or branched $(C_1-C_6)$alkoxy group),
- an unsubstituted phenyl group,
- an (imidazol-2-yl)methyl group or an (indol-3-yl)methyl group that is unsubstituted or is substituted on the heterocycle by a methyl group

and

$R_2$ = H, $R_3$ = H,

X = 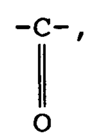

Y = $COR_5$, ($R_5$, = OH, alkoxy),

2 in the other cases :

a straight-chain or branched alkyl group containing from 6 to 21 carbon atoms that is unsubstituted or is substituted at the terminal methyl group by a hydroxy, mercapto, phenyl or ethynyl group and in which one or more methylene groups may be replaced by an oxygen atom, a sulphur atom or a p-phenylene nucleus (with the proviso that when $R_2$ or $R_3$, together with the nitrogen or carbon atoms to which they are attached, form a 1,2,3,4-tetrahydroisoquinoline, indoline or isoindoline ring then $R_4$ is other than the group alk-S-$(CH_2)_n$- in which n is 1, 2, 3, 4 or 5 and alk represents a straight-chain or branched $(C_4-C_{19})$alkyl group),

characterised in that :

1- in the case where the compounds of formula (I) it is desired to obtain contain a radical $R_4 = R_{4'}$ in which no methylene group is replaced by a sulphur or oxygen atom,
a compound of formula (II) :

$R_{4'}$ - X' - Z (II)

wherein :

X' represents a group

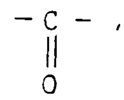

$— SO_2 —$ ,

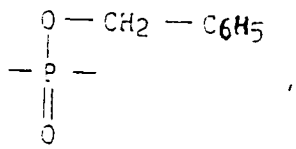

$R_{4'}$ represents a straight-chain or branched alkyl group containing from 6 to 21 carbon atoms that is unsubstituted or is substituted at the terminal methyl group by a hydroxy, mercapto, phenyl or ethynyl group, and in which one or more methylene groups may be replaced by a p-phenylene group,

Z represents a halogen atom, a hydroxy group, a straight-chain or branched $(C_1\text{-}C_6)$alkoxy group, an aralkoxy group or a group

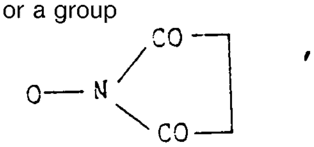

is condensed with an amine of formula (III) (in racemic or isomeric form), which amine is protected where appropriate :

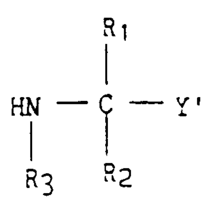

(III)

wherein $R_2$, $R_3$ and $R_4$ are as defined for formula I and Y' represents a group $-CO_2H$, -CO(alkoxy) or $-PO(alkoxy)_2$,
to yield, after deprotection where necessary, a compound of formula (I/a),
a particular instance of compounds of formula (I)

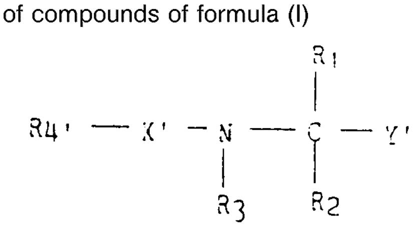

(Ia)

wherein $R_1$, $R_2$, $R_3$, $R_{4'}$, X' and Y' are as defined above,
2- in the case where the compounds of formula (I) it is desired to obtain contain a radical $R_4 = R_{4''} = CH_3\text{-}(CH_2)_n\text{-}$ in which at least one of the methylene groups is replaced by a sulphur or oxygen

atom,
a compound of formula (IV)

$A\text{-}(CH_2)_m\text{-}X'\text{-}Z$ (IV)

wherein :

X' and Z are as defined above,
A represents a halogen atom, a mesyloxy group or a tosyloxy group,
m is less than or equal to n-1,

and one of the methylene groups may be replaced by an oxygen atom, a sulphur atom, a p-phenylene group, a $-CH(CH_3)-$ group or a $-C(CH_3)_2-$ group,
is condensed with an amine of formula (III) as defined above (in racemic or isomeric form), which amine is protected where appropriate,
to yield a compound of formula (V)

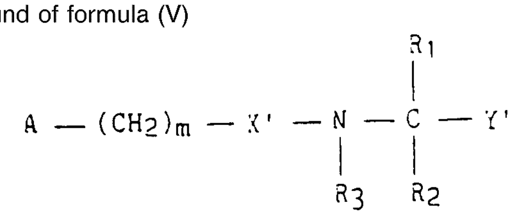

(V)

wherein A, m, X', $R_1$, $R_2$, $R_3$ and Y' are as defined above,
which is reacted with a compound of formula (VI) :

$R_9\text{-}B\text{-}M$ (VI)

wherein :

$R_9$ represents a $CH_3\text{-}(CH_2)_p\text{-}$ group that is unsubstituted or is substituted at the terminal methyl group by a hydroxy, mercapto, phenyl or ethynyl group and in which one of the methylene groups may be replaced by an oxygen atom, a sulphur atom, a p-phenylene group, a $-CH(CH_3)-$ group or a $-C(CH_3)_2-$ group,
B represents an oxygen or sulphur atom,
M represents a metal selected from sodium, potassium and caesium,
p is such that the sum of m and p is less than or equal to n-1,

to yield, after deprotection where appropriate, a compound of formula (I/b), a particular instance of compounds of formula (I) :

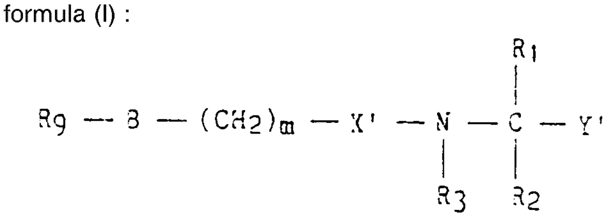

(I/b)

wherein $R_9$, B, m, X', $R_1$, $R_2$, $R_3$ and Y' are as defined above, which compounds of formulae (I/a) and (I/b), which may be written in a simplified manner :

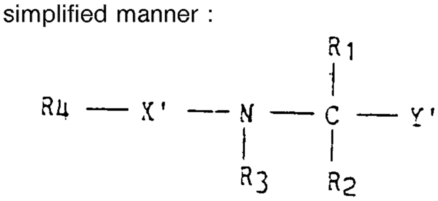

a in the case where X' represents

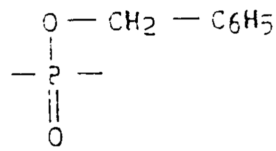

may be converted by catalytic hydrogenation into compounds of formula (I/c), a particular instance of compounds of formula (I) :

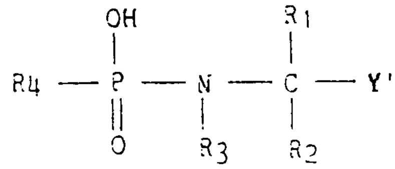

(I/c)

wherein $R_1$, $R_2$, $R_3$, $R_4$ and Y' are as defined above,
b in the case where Y' represents a -CO(alkoxy) or $-PO(alkoxy)_2$ group, may be totally or partially hydrolysed to yield, respectively, compounds of formulae (I/d), (I/e) and (I/f), a particular instance of compounds of formula (I) :

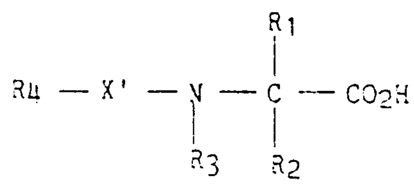

(I/d)

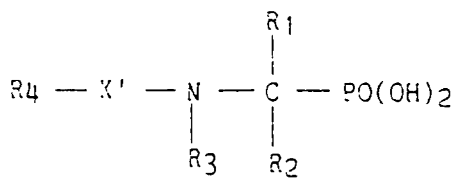

(I/e)

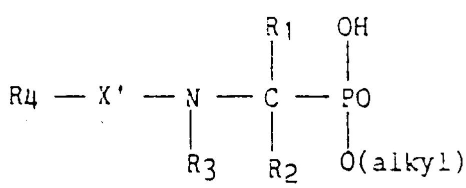

(I/f)

wherein $R_1$, $R_2$, $R_3$, $R_4$ and X' are as defined above,
c in the case where Y' represents a $-CO_2H$ group, may be converted with the aid of chloroacetamide, in the presence of caesium carbonate, into a compound of formula (I/g), a particular instance of compounds of formula (I) :

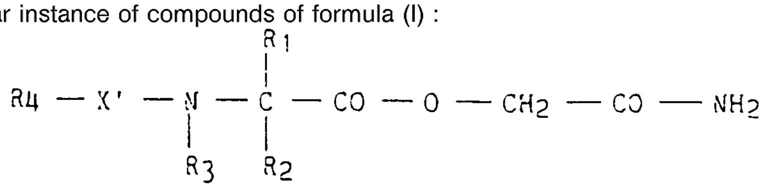

(I/g)

wherein $R_1$, $R_2$, $R_3$, $R_4$ and X' are as defined above,
d in the case where Y' represents a $-CO_2H$ group,
may be esterified with the aid of isopropylidene glycerol to form a compound of formula (I/h), a particular instance of compounds of formula (I)

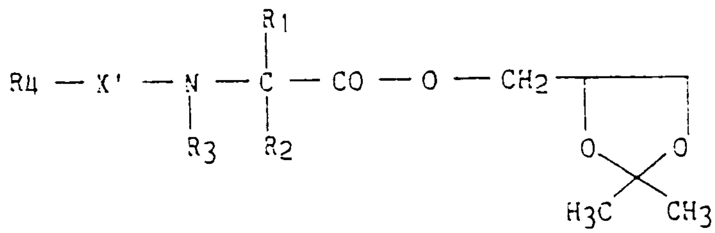

(I/h)

wherein $R_1$, $R_2$, $R_3$ and $R_4$ are as defined above,

which compound of formula (I/h) may be hydrolysed in acidic medium to yield a compound of formula (I/i), a particular instance of compounds of formula (I)

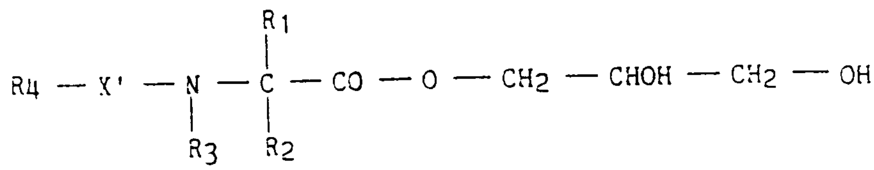

(I/i)

wherein $R_1$, $R_2$, $R_3$ and $R_4$ are as defined above,

which compounds of formulae (I/a) to (I/i) may be purified, where necessary, by a conventional method of purification, may be separated into their isomers according to a conventional method of separation, and may be converted into their addition salts with a pharmaceutically acceptable acid or base.

**2.** Process according to claim 1 for the preparation of N-myristoyl-3-carboxy-(1,2,3,4)-tetrahydroisoquinoline, its enantiomers and also its addition salts with a pharmaceutically acceptable base.

**3.** Process according to claim 1 for the preparation of N-myristoyl-(N$^{\tau}$-benzyl)histidine, its enantiomers and also its addition salts with a pharmaceutically acceptable acid or base.

**4.** Process according to claim 1 for the preparation of ethyl 1-myristoylamino-2-phenylethanehydrogenphosphonate, its enantiomers and also its addition salts with a pharmaceutically acceptable base.

**5.** Compound according to claim 1, which is N-(12-methoxylauroyl)-phenylalanine, its enantiomers and also its addition salts with a pharmaceutically acceptable base.

**Claims for the following Contracting State : GR**

**1.** Process for the preparation of compounds of the general formula (I)

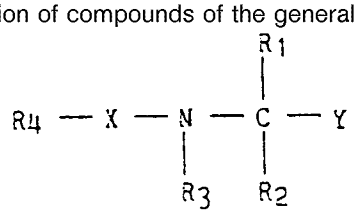

(I)

wherein :

$R_1$ represents

- a hydrogen atom,
- a straight-chain or branched ($C_1$-$C_6$)alkyl group that is unsubstituted or is substituted by one or more groups hydroxy, amino, carboxy, carbamoyl, benzylthio, methylthio, mercapto or phenyl (unsubstituted or substituted by one or more halogen atoms or hydroxy, straight-chain or branched ($C_1$-$C_6$)alkyl, straight-chain or branched ($C_1$-$C_6$)alkoxy or $(CH_3$-$CH_2$-$O)_2PO$-$CH_2$- groups),
- a phenyl group that is unsubstituted or is substituted by one or more halogen atoms or hydroxy or straight-chain or branched ($C_1$-$C_6$)alkyl groups,

- a ($C_3$-$C_7$)cycloalkyl-methyl group,
- an (imidazol-2-yl)methyl group or an (indol-3-yl)methyl group that is unsubstituted or is substituted on the heterocycle by a benzyl, benzhydryl, trityl, benzyloxymethyl, tosyl, straight-chain or branched ($C_1$-$C_6$)alkyl or phenyl group,
- a (1-azaindolizin-2-yl)methyl group of formula :

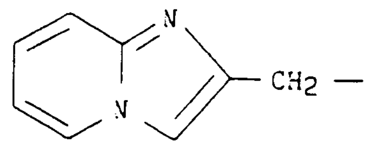

$R_2$ represents a hydrogen atom or a straight-chain or branched ($C_1$-$C_6$)alkyl group,

$R_3$ represents a hydrogen atom or a straight-chain or branched ($C_1$-$C_6$)alkyl group,

or

when $R_1$ represents a hydrogen atom,

$R_2$ and $R_3$ may form, with the carbon and nitrogen atoms to which they are respectively attached, any one of the following heterocycles:

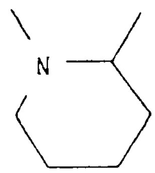 , 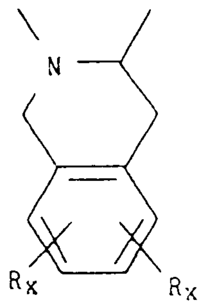 , 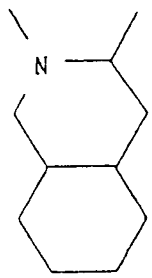 , 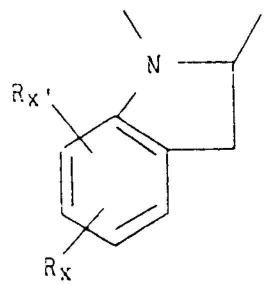 ,

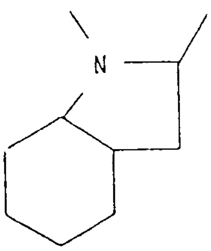 , 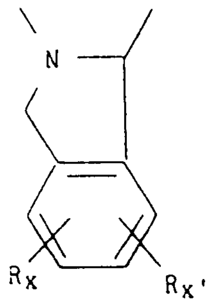 , 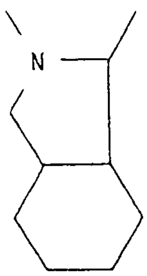 , 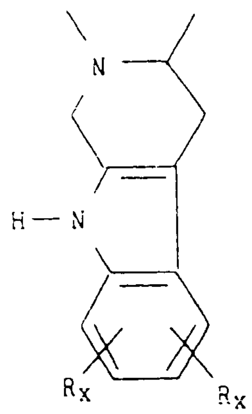

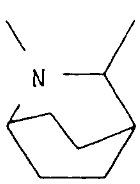 , 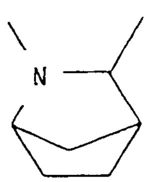 , 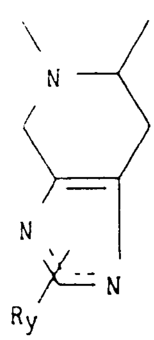

$R_x$ and $R_{x'}$, which are the same or different, each represents a hydrogen atom, a straight-chain or

branched ($C_1$-$C_6$)alkyl group, a hydroxy group, a straight-chain or branched ($C_1$-$C_6$)-alkoxy group or a halogen atom,

or $R_x$ and $R_{x'}$, when located at two adjacent carbon atoms, alternatively form a methylenedioxy or ethylenedioxy group,

$R_y$ represents a hydrogen atom, a straight-chain or branched ($C_1$-$C_6$)alkyl group, an aryl group, an aralkyl group, an aroyl group or an arylsulphonyl group,

X represents any one of the following groups :

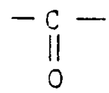 ,

$-SO_2-$ ,

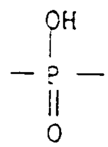 ,

Y represents a group $-CO-R_5$ or

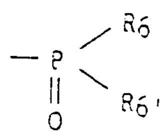 ,

$R_5$ represents a hydroxy group, a straight-chain or branched ($C_1$-$C_6$)alkoxy group, a $H_2N-CO-CH_2-O-$ group, a $HO-CH_2-CHOH-CH_2-O-$ group,

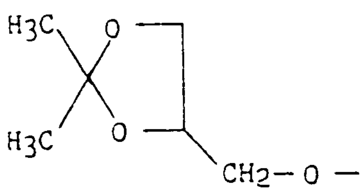 or 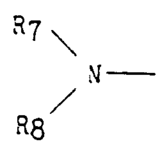 ,

$R_7$ and $R_8$, which are the same or different, each represents a hydrogen atom or a straight-chain or branched ($C_1$-$C_6$)alkyl group, or together with the nitrogen atom to which they are attached form a pyrrolidine, piperidine, morpholine or piperazine ring,

$R_6$ and $R_{6'}$, which are the same or different, each represents a hydrogen atom, a hydroxy group or a straight-chain or branched ($C_1$-$C_6$)alkoxy group,

$R_4$ represents :

1 a straight-chain or branched alkyl group containing from 6 to 21 carbon atoms that is unsubstituted or is substituted at the terminal methyl group by a hydroxy, mercapto, phenyl or ethynyl group and in which at least one of the methylene groups is replaced by an oxygen atom, a sulphur atom or a p-phenylene nucleus,

in the case where :

$R_1$ represents

- a hydrogen atom,
- a straight-chain or branched ($C_1$-$C_6$)alkyl group that is unsubstituted or is substituted by one or more groups hydroxy, amino, carboxy, carbamoyl, benzylthio, methylthio, mercapto or phenyl (unsubstituted or substituted by a hydroxy or straight-chain or branched ($C_1$-$C_6$)alkoxy group), - an unsubstituted phenyl group,
- an (imidazol-2-yl)methyl group or an (indol-3-yl)methyl group that is unsubstituted or is substituted on the heterocycle by a methyl group

and

$R_2$ = H, $R_3$ = H,

X = 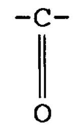 ,

Y = $COR_{5'}$ ($R_{5'}$ = OH, alkoxy),

2 in the other cases :

a straight-chain or branched alkyl group containing from 6 to 21 carbon atoms that is unsubstituted or is substituted at the terminal methyl group by a hydroxy, mercapto, phenyl or ethynyl group and in which one or more methylene groups may be replaced by an oxygen atom, a sulphur atom or a p-phenylene nucleus (with the proviso that when $R_2$ or $R_3$, together with the nitrogen or carbon atoms to which they are attached, form a 1,2,3,4-tetrahydroiso-quinoline, indoline or isoindoline ring then $R_4$ is other than the group alk-$S(CH_2)n$- in which n is 1, 2, 3, 4 or 5 and alk represents a straight-chain or branched ($C_4$-$C_{19}$)alkyl group),

characterised in that :

1- in the case where the compounds of formula (I) it is desired to obtain contain a radical $R_4$ = $R_{4'}$ in which no methylene group is replaced by a sulphur or oxygen atom,

a compound of formula (II) :

$R_{4'}$ - X' - Z (II)

wherein :

X' represents a group

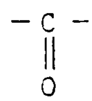 ,

— $SO_2$ — ,

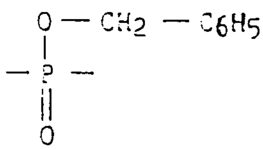 ,

$R_{4'}$ represents a straight-chain or branched alkyl group containing from 6 to 21 carbon atoms that is unsubstituted or is substituted at the terminal methyl group by a hydroxy, mercapto, phenyl or ethynyl group, and in which one or more methylene groups may be replaced by a p-phenylene group,

Z represents a halogen atom, a hydroxy group, a straight-chain or branched ($C_1$-$C_6$)alkoxy group, an aralkoxy group or a group

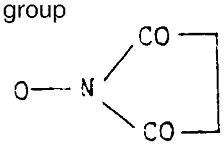 ,

is condensed with an amine of formula (III) (in racemic or isomeric form), which amine is protected where appropriate :

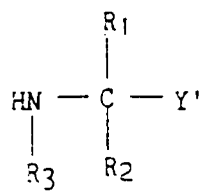

(III)

wherein $R_2$, $R_3$ and $R_4$ are as defined for formula I and Y' represents a group $-CO_2H$, -CO(alkoxy) or $-PO(alkoxy)_2$,
to yield, after deprotection where necessary, a compound of formula (I/a),
a particular instance of compounds of formula (I)

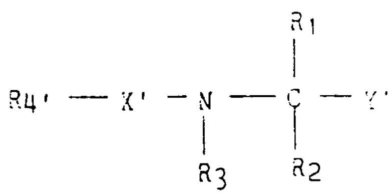

(Ia)

wherein $R_1$, $R_2$, $R_3$, $R_{4'}$, X' and Y' are as defined above,
2- in the case where the compounds of formula (I) it is desired to obtain contain a radical $R_4 = R_{4''} = CH_3-(CH_2)_n-$ in which at least one of the methylene groups is replaced by a sulphur or oxygen atom,
a compound of formula (IV)

$A-(CH_2)_m-X'-Z$ (IV)

wherein :

- X' and Z are as defined above,
- A represents a halogen atom, a mesyloxy group or a tosyloxy group,
- m is less than or equal to n-1,

and one of the methylene groups may be replaced by an oxygen atom, a sulphur atom, a p-phenylene group, a $-CH(CH_3)-$ group or a $-C(CH_3)_2-$ group,
is condensed with an amine of formula (III) as defined above (in racemic or isomeric form), which amine is protected where appropriate,
to yield a compound of formula (V)

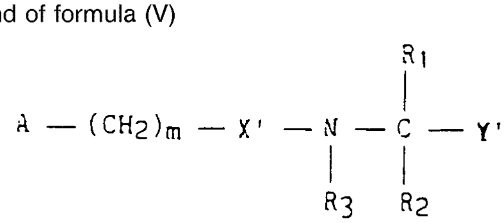

(V)

wherein A, m, X', $R_1$, $R_2$, $R_3$ and Y' are as defined above, which is reacted with a compound of formula (VI) :

$R_9-B-M$ (VI)

wherein :

- $R_9$ represents a $CH_3-(CH_2)_p-$ group that is unsubstituted or is substituted at the terminal methyl group by a hydroxy, mercapto, phenyl or ethynyl group and in which one of the methylene groups may be replaced by an oxygen atom, a sulphur atom, a p-phenylene group, a $-CH(CH_3)-$ group or a $-C(CH_3)_2-$ group,
- B represents an oxygen or sulphur atom,
- M represents a metal selected from sodium, potassium and caesium,
- p is such that the sum of m and p is less than or equal to n-1,

to yield, after deprotection where appropriate, a compound of formula (I/b), a particular instance of compounds of formula (I) :

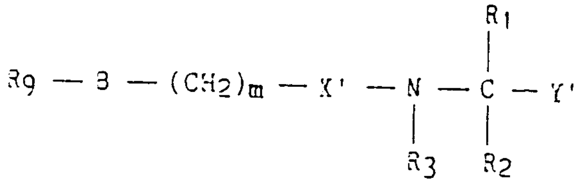

(I/b)

wherein $R_9$, B, m, X', $R_1$, $R_2$, $R_3$ and Y' are as defined above,
which compounds of formulae (I/a) and (I/b), which may be written in a simplified manner :

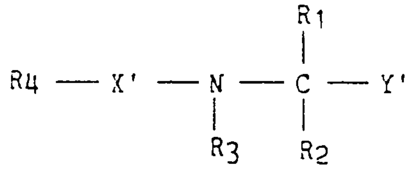

a in the case where X' represents

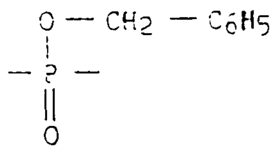

may be converted by catalytic hydrogenation into compounds of formula (I/c), a particular instance of compounds of formula (I) :

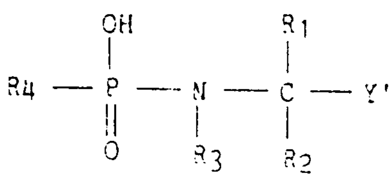

(I/c)

wherein $R_1$, $R_2$, $R_3$, $R_4$ and Y' are as defined above,
b in the case where Y' represents a -CO(alkoxy) or $-PO(alkoxy)_2$ group, may be totally or partially hydrolysed to yield, respectively, compounds of formulae (I/d), (I/e) and (I/f), a particular instance of compounds of formula (I) :

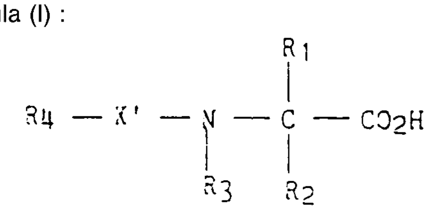

(I/d)

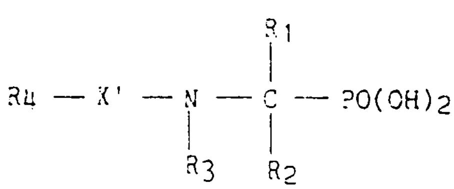

(I/e)

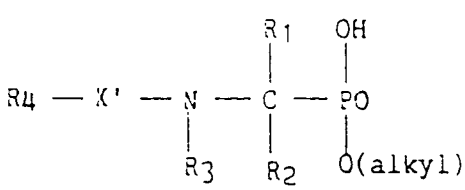

(I/f)

wherein $R_1$, $R_2$, $R_3$, $R_4$ and X' are as defined above,

c in the case where Y' represents a $-CO_2H$ group, may be converted with the aid of chloroacetamide, in the presence of caesium carbonate, into a compound of formula (I/g), a particular instance of compounds of formula (I) :

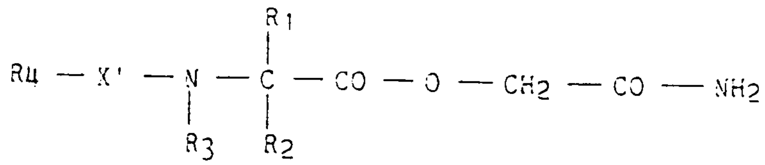
(I/g)

wherein $R_1$, $R_2$, $R_3$, $R_4$ and X' are as defined above,

d in the case where Y' represents a $-CO_2H$ group,

may be esterified with the aid of isopropylidene glycerol to form a compound of formula (I/h), a particular instance of compounds of formula (I)

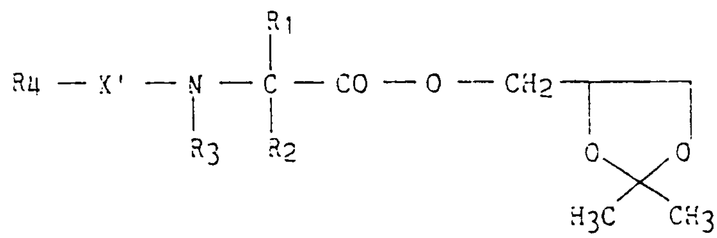
(I/h)

wherein $R_1$, $R_2$, $R_3$ and $R_4$ are as defined above,

which compound of formula (I/h) may be hydrolysed in acidic medium to yield a compound of formula (I/i), a particular instance of compounds of formula (I)

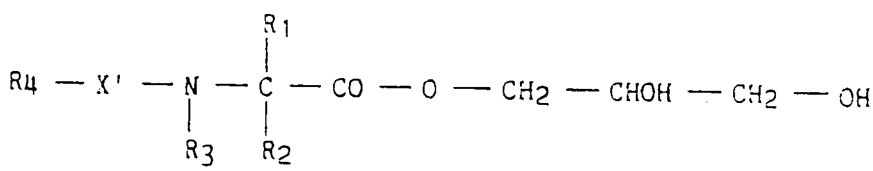
(I/i)

wherein $R_1$, $R_2$, $R_3$ and $R_4$ are as defined above,

which compounds of formulae (I/a) to (I/i) may be purified, where necessary, by a conventional method of purification, may be separated into their isomers according to a conventional method of separation, and may be converted into their addition salts with a pharmaceutically acceptable acid or base.

2. Process according to claim 1 for the preparation of N-myristoyl-3-carboxy-(1,2,3,4)-tetrahydroisoquinoline, its enantiomers and also its addition salts with a pharmaceutically acceptable base.

3. Process according to claim 1 for the preparation of N-myristoyl-(N$^{\tau}$-benzyl)histidine, its enantiomers and also its addition salts with a pharmaceutically acceptable acid or base.

4. Process according to claim 1 for the preparation of ethyl 1-myristoylamino-2-phenylethanehydrogenphosphonate, its enantiomers and also its addition salts with a pharmaceutically acceptable base.

5. Compound according to claim 1, which is N-(12-methoxylauroyl)-phenylalanine, its enantiomers and also its addition salts with a pharmaceutically acceptable base.

**Patentansprüche**

**Patentansprüche für folgende Vertragsstaaten : AT, BE, CH, LI, DE, DK, FR, GB, IT, LU, NL, PT, SE**

1. Verbindungen der allgemeinen Formel (I)

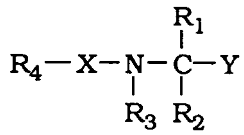

(I)

in der

$R_1$

- ein Wasserstoffatom,
- eine geradkettige oder verzweigte ($C_1$-$C_6$)-Alkylgruppe, die nichtsubstituiert oder durch eine oder mehrere Hydroxyl-, Amino-, Carboxy-, Carbamoyl-, Benzylthio-, Methylthio-, Mercapto- oder Phenyl-gruppen (welche nichtsubstituiert oder durch eines oder mehrere Halogenatome oder Hydroxylgruppen, geradkettige oder verzweigte ($C_1$-$C_6$)-Alkylgruppen, geradkettige oder verzweigte ($C_1$-$C_6$)-Alkoxygruppen oder ($CH_3$-$CH_2$-O)$_2$-PO-$CH_2$-) substituiert ist),
- eine Phenylgruppe, die nichtsubstituiert oder durch eines oder mehrere Halogenatome oder Hydroxylgruppen oder geradkettige oder verzweigte ($C_1$-$C_6$)-Alkylgruppen substituiert ist,
- eine ($C_3$-$C_7$)-Cycloalkyl-methylgruppe,
- eine (Imidazol-2-yl)-methylgruppe oder eine (Indol-3-yl)-methylgruppe, die nichtsubstituiert oder am Heterocyclus durch eine Benzyl-, Benzhydryl-, Trityl-, Benzyloxymethyl-, Tosyl-, geradkettige oder verzweigte ($C_1$-$C_6$)-Alkylgruppe oder Phenylgruppe substituiert ist,
- eine (1-Azaindolizin-2-yl)-methylgruppe der Formel:

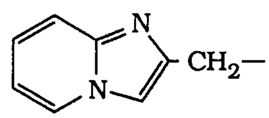

$R_2$ ein Wasserstoffatom oder eine geradkettige oder verzweigte ($C_1$-$C_6$)-Alkylgruppe,

$R_3$ ein Wasserstoffatom oder eine geradkettige oder verzweigte ($C_1$-$C_6$)-Alkylgruppe,

oder

wenn $R_1$ ein Wasserstoffatom darstellt.

$R_2$ und $R_3$ gemeinsam mit den Kohlenstoff- und Stickstoffatomen, an die sie gebunden sind, einen der folgenden Heterocyclen bilden können:

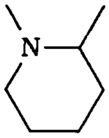 , 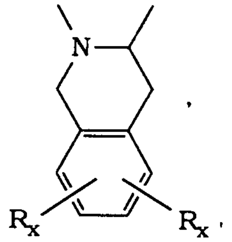 , 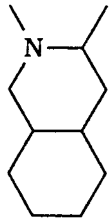 , 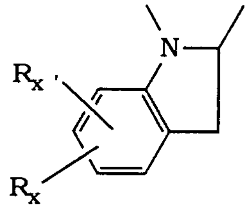 ,

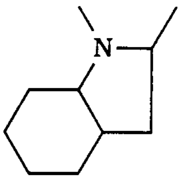 , 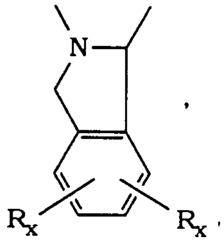 , 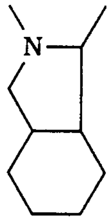 , 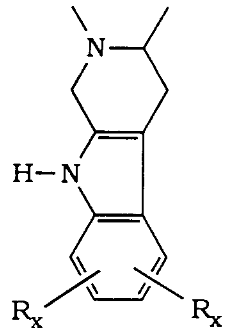 ,

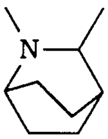 , 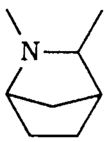 , 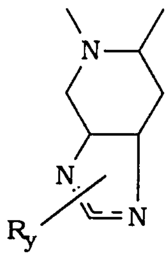

$R_x$ und $R_{x'}$, die gleichartig oder verschieden sein können, ein Wasserstoffatom, eine geradkettige oder verzweigte ($C_1$-$C_6$)-Alkylgruppe, eine Hydroxylgruppe, eine geradkettige oder verzweigte ($C_1$-$C_6$)-Alkoxygruppe oder ein Halogenatom,

oder $R_x$ und $R_{x'}$, wenn sie an zwei benachbarten Kohlenstoffatomen angeordnet sind, gemeinsam eine Methylendioxy- oder Ethylendioxy-gruppe bilden,

Ry ein Wasserstoffatom, eine geradkettige oder verzweigte ($C_1$-$C_6$)-Alkylgruppe, eine Arylgruppe, eine Aralkylgruppe, eine Aroylgruppe oder eine Arylsulfonylgruppe,

X eine der folgenden Gruppen:

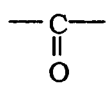 ,

$-SO_2-$ ,

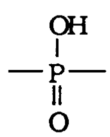 ,

Y eine Gruppe $-CO-R_5$ oder

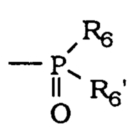

$R_5$ eine Hydroxylgruppe, eine geradkettige oder verzweigte ($C_1$-$C_6$)-Alkoxygruppe,$H_2N$-$CO$-$CH_2$-$O$-, $HO$-$CH_2$-$CHOH$-$CH_2$-$O$-,

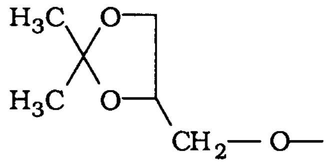 , 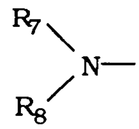

$R_7$ und $R_8$, die gleichartig oder verschieden sein können, ein Wasserstoffatom, eine geradkettige oder verzweigte ($C_1$-$C_6$)-Alkylgruppe oder gemeinsam mit dem Stickstoffatom, an das sie gebunden sind, einen Pyrrolidin-, Piperidin-, Morpholin- oder Piperazin-Ring bilden,

$R_6$ und $R_{6'}$, die gleichartig oder verschieden sein können, ein Wasserstoffatom, eine Hydroxylgruppe oder eine geradkettige oder verzweigte ($C_1$-$C_6$)-Alkoxygruppe,

$R_4$

1 eine geradkettige oder verzweigte Alkylgruppe mit 6 bis 21 Kohlenstoffatomen, die nicht substituiert oder an der endständigen Methylgruppe durch eine Hydroxyl-, Mercapto-, Phenyl- oder Ethinyl-gruppe substituiert ist und worin mindestens eine der Methylengruppen durch ein Sauerstoffatom, ein Schwefelatom oder einen p-Phenylenkern ersetzt ist,

dann, wenn:

$R_1$

- ein Wasserstoffatom,
- eine geradkettige oder verzweigte ($C_1$-$C_6$)-Alkylgruppe, die nicht substituiert oder durch eine oder mehrere Hydroxyl-, Amino-, Carboxy-, Carbamoyl-, Benzylthio-, Methylthio-, Mercapto- oder Phenylgruppen (die nichtsubstituiert oder durch eine Hydroxylgruppe oder eine geradkettige oder verzweigte ($C_1$-$C_6$)-Alkoxygruppe substituiert ist),
- eine nichtsubstituierte Phenylgruppe,
- eine (Imidazol-2-yl)-methylgruppe oder eine (Indol-3-yl)-methylgruppe, die nichtsubstituiert oder am Heterocyclus durch eine Methylgruppe substituiert ist, und

$R_2 = H$, $R_3 = H$,

X = 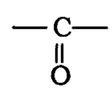 ,

$Y = COR_5'$ ($R_5'$ = OH,Alkoxy)

2 und in den anderen Fällen:

eine geradkettige oder verzweigte Alkylgruppe mit 6 bis 21 Kohlenstoffatomen, die nichtsubstituiert oder an der endständigen Methylgruppe durch eine Hydroxyl-, Mercapto-, Phenyl- oder Ethinyl-gruppe substituiert ist und worin eine oder mehrere Methylengruppen durch ein Sauerstoffatom, ein Schwefelatom oder eine p-Phenylengruppe ersetzt sein können (mit der Maßgabe, daß, wenn $R_2$ oder $R_3$ mit den Stickstoff- oder Kohlenstoffatomen, an die sie gebunden sind, einen 1,2,3,4-Tetrahydroisochinolin-, Indolin-oder Isoindolin-Ring bilden, $R_4$ von der Gruppe Alk-S-$(CH_2)_n$- verschieden ist, worin n einen Wert von 1, 2, 3, 4 oder 5 und Alk eine geradkettige oder verzweigte ($C_4$-$C_{19}$)-Alkylgruppe darstellen),

bedeuten,

deren Isomere, Diastereoisomere und Epimere sowie deren Additionssalze mit einer pharmazeutisch annehmbaren Säure oder Base.

2. Verbindung nach Anspruch 1, nämlich N-Myristoyl-3-carboxy-(1,2,3,4)-tetrahydroisochinolin, dessen Enantiomere sowie dessen Additionssalze mit einer pharmazeutisch annehmbaren Base.

3. Verbindung nach Anspruch 1, nämlich N-Myristoyl-(N'τ-benzyl)-histidin, dessen Enantiomere sowie dessen Additionssalze mit einer pharmazeutisch annehmbaren Säure oder Base.

**4.** Verbindung nach Anspruch 1, nämlich 1-Myristoylamino-2-phenylethanhydrogenphosphonsäureethylester, dessen Enantiomere sowie dessen Additionssalze mit einer pharmazeutisch annehmbaren Base.

**5.** Verbindung nach Anspruch 1, nämlich N-(12-Methoxylauroyl)-phenylalanin, dessen Enantiomere sowie dessen Additionssalze mit einer pharmazeutisch annehmbaren Base.

**6.** Verfahren zur Herstellung der Derivate der Formel (I) nach Anspruch 1, **dadurch gekennzeichnet**, daß man:

1- dann, wenn die herzustellenden Derivate der Formel (I) eine Gruppe $R_4 = R_{4'}$ aufweisen. worin keine Methylengruppe durch ein Schwefelatom oder ein Sauerstoffatom ersetzt ist.
man eine Verbindung der Formel (II):

$R_{4'}$ - X'- Z (II)

in der:

X' eine Gruppe

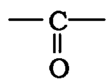
,

$-SO_2-$ ,

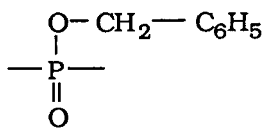
,

$R_{4'}$ eine geradkettige oder verzweigte Alkylgruppe mit 6 bis 21 Kohlenstoffatomen, die nichtsubstituiert oder an der endständigen Methylgruppe durch eine Hydroxyl-, Mercapto-, Phenyl- oder Ethinylgruppe substituiert ist, und worin eine oder mehrere Methylengruppen durch eine p-Phenylengruppe ersetzt sein können,

Z ein Halogenatom, eine Hydroxylgruppe, eine geradkettige oder verzweigte ($C_1$-$C_6$)-Alkoxygruppe, eine Aralkoxygruppe oder eine Gruppe

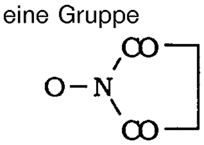

bedeuten,

mit einem gegebenenfalls geschützen (in Form des Racemats oder der Isomeren vorliegenden) Amin der Formel (III)

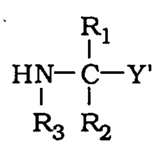
(III)

in der $R_2$, $R_3$ und $R_4$ die bezüglich der Formel I angegebenen Bedeutungen besitzen und Y' eine Gruppe der Formeln $-CO_2H$, -CO(Alkoxy) oder $-PO(Alkoxy)_2$ darstellen, kondensiert,
so daß man nach der eventuellen Abspaltung der Schutzgruppe eine Verbindung der Formel (I/a) erhält, einem Sonderfall der Verbindungen der Formel (I):

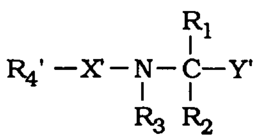
(I/a)

in der $R_1$, $R_2$, $R_3$, $R_{4'}$, X' und Y' die oben angegebenen Bedeutungen besitzen,
2- dann, wenn die herzustellenden Derivate der Formel (I) eine Gruppe $R_4 = R_{4''} = CH_3\text{-}(CH_2)_n\text{-}$, worin mindestens eine der Methylengruppen durch ein Schwefelatom oder ein Sauerstoffatom ersetzt ist, aufweisen,
man:
eine Verbindung der Formel (IV)

$A - (CH_2)_m - X' - Z$ (IV)

in der:
X' und Z die oben angegebenen Bedeutungen besitzen,
A ein Halogenatom, eine Mesyloxygruppe oder eine Tosyloxygruppe und
m kleiner oder gleich n - 1 ist, bedeuten,
und eine der Methylengruppen durch ein Sauerstoffatom, ein Schwefelatom, eine p-Phenylengruppe, eine Gruppe $-CH(CH_3)-$ oder eine Gruppe $-C(CH_3)_2-$ersetzt sein kann,
mit einem gegebenenfalls geschützten (in Form des Racemats oder der Isomeren vorliegenden) Amin der Formel (III), wie es oben definiert worden ist,
kondensiert zur Bildung einer Verbindung der Formel (V)

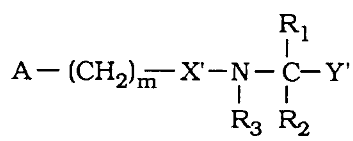
(V)

In der A, m, X', $R_1$, $R_2$, $R_3$ und Y' die oben angegebenen Bedeutungen besitzen, welche man mit einem Derivat der Formel (VI):

$R_9 - B - M$ (VI)

in der:
$R_9$ eine Gruppe der Formel $CH_3\text{-}(CH_2)_p\text{-}$, die nichtsubstituiert oder an der endständigen Methylgruppe durch eine Hydroxyl-, Mercapto-, Phenyl- oder Ethinylgruppe substituiert ist und bei der eine der Methylengruppen durch ein Sauerstoffatom, ein Schwefelatom, eine p-Phenylengruppe, eine Gruppe $-CH(CH_3)-$ oder eine Gruppe $-C(CH_3)_2-$ ersetzt sein kann,
B ein Sauerstoffatom oder ein Schwefelatom,
M ein aus Natrium, Kalium oder Caesium ausgewähltes Metall und
p einen solchen Wert besitzt, daß $m + p$ kleiner oder gleich n - 1 ist,
bedeuten, umsetzt, so daß man nach der eventuellen Abspaltung der Schutzgruppe eine Verbindung der Formel (I/b) erhält, einem Sonderfall der Verbindungen der Formel (I):

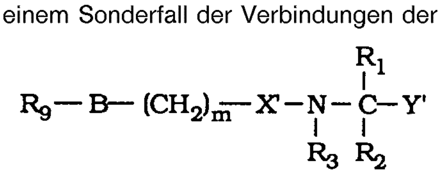
(I/b)

in der $R_9$, B, m, X', $R_1$, $R_2$, $R_3$ und Y' die oben angegebenen Bedeutungen besitzen,
welche Derivate der Formeln (I/a) und (I/b) man auch vereinfacht wie folgt schreiben kann:

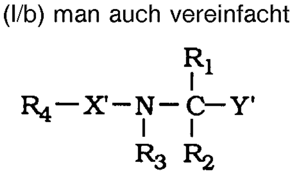

welche
a dann, wenn X' eine Gruppe der Formel

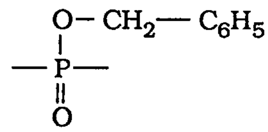

darstellt, durch katalytische Hydrierung in die Verbindungen der Formel (I/c) umgewandelt werden können, einem Sonderfall der Verbindungen der Formel (I):

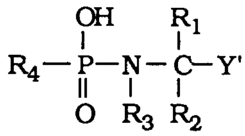

(I/c)

in der $R_1$, $R_2$, $R_3$, $R_4$ und Y' die oben angegebenen Bedeutungen besitzen,

b dann, wenn Y' eine Gruppe -CO(Alkoxy) oder $-PO(Alkoxy)_2$ darstellt, vollständig oder teilweise verseift werden können, so daß man die Verbindungen der Formeln (I/d), (I/e) bzw. (I/f) erhält, Sonderfälle der Verbindungen der Formel (I):

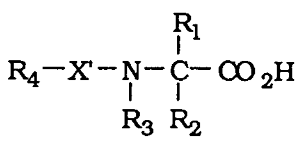

(I/d)

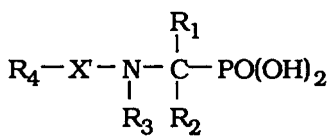

(I/e)

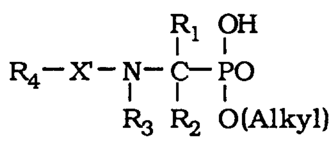

(I/f)

worin $R_1$, $R_2$, $R_3$, $R_4$ und X' die oben angegebenen Bedeutungen besitzen,

c dann, wenn Y' eine Gruppe $-CO_2H$ darstellt, in Gegenwart von Caesiumcarbonat mit Hilfe von Chloracetamid in die Verbindung der Formel (I/g) umgewandelt werden können, einem Sonderfall der Verbindungen der Formel (I):

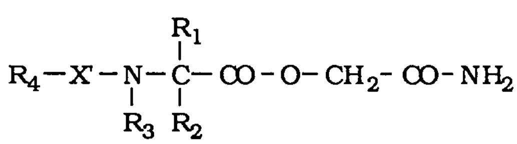

(I/g)

in der $R_1$, $R_2$, $R_3$, $R_4$ und X' die oben angegebenen Bedeutungen besitzen,

d dann, wenn Y' eine Gruppe $-CO_2H$ darstellt,

mit Hilfe von Isopropylidenglycerol zu einer Verbindung der Formel (I/h) verestert werden können, einem Sonderfall der Verbindungen der Formel (I):

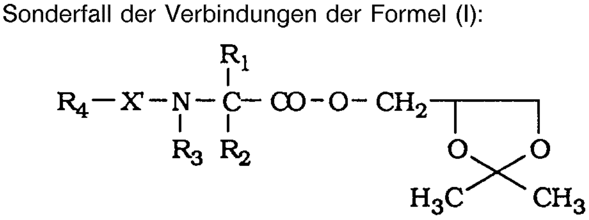

(I/h)

in der $R_1$, $R_2$, $R_3$ und $R_4$ die oben angegebenen Bedeutungen besitzen, welches Derivat der Formel (I/h) in saurem Medium hydrolysiert werden kann zur Bildung der Verbindung der Formel (I/i), einem Sonderfall der Verbindungen der Formel (I):

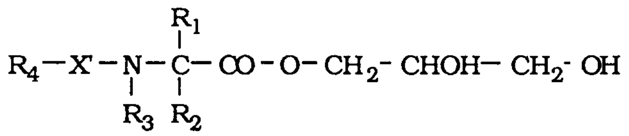
(I/i)

in der $R_1$, $R_2$, $R_3$ und $R_4$ die oben angegebenen Bedeutungen besitzen,
welche Verbindungen der Formeln (I/a) bis (I/i) gegebenenfalls mit Hilfe einer klassischen Reinigungsmethode gereinigt werden können, dessen Isomeren mit Hilfe einer klassischen Trennmethode getrennt werden können und welche man mit einer pharmazeutisch annehmbaren Säure oder Base in ihre Säureadditionssalze umwandeln kann.

**7.** Pharmazeutische Zubereitungen enthaltend als Wirkstoff mindestens eine Verbindung nach einem der Ansprüche 1 bis 5 allein oder in Kombination mit einem oder mehreren inerten, nichttoxischen, pharmazeutisch annehmbaren Trägermaterialien.

**8.** Pharmazeutische Zubereitungen nach Anspruch 7 für die Behandlung von Krebs und/oder Virenerkrankungen, deren Ausbrechen eine Myristoylierung impliziert, wie AIDS, Herpes, Hepatitis B, Influenza, Poliomyelitis oder Leukämie.

**Patentansprüche für folgenden Vertragsstaat : ES**

**1.** Verfahren zur Herstellung der Verbindungen der allgemeinen Formel (I)

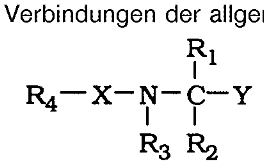
(I)

in der

$R_1$

- ein Wasserstoffatom,
- eine geradkettige oderverzweigte ($C_1$-$C_6$)-Alkylgruppe, die nichtsubstituiert oder durch eine oder mehrere Hydroxyl-, Amino-, Carboxy-, Carbamoyl-` Benzylthio-, Methylthio-, Mercapto- oder Phenyl-gruppen (welche nichtsubstituiert oder durch eines oder mehrere Halogenatome oder Hydroxylgruppen, geradkettige oder verzweigte ($C_1$-$C_6$)-Alkylgruppen, geradkettige oder verzweigte ($C_1$-$C_6$)-Alkoxygruppen oder $(CH_3\text{-}CH_2\text{-}O)_2\text{-}PO\text{-}CH_2\text{-}$) substituiert ist),
- eine Phenylgruppe, die nichtsubstituiert oder durch eines oder mehrere Halogenatome oder Hydroxylgruppen oder geradkettige oder verzweigte ($C_1$-$C_6$)-Alkylgruppen substituiert ist,
- eine ($C_3$-$C_7$)-Cycloalkyl-methylgruppe,
- eine (Imidazol-2-yl)-methylgruppe oder eine (Indol-3-yl)-methylgruppe, die nichtsubstituiert oder am Heterocyclus durch eine Benzyl-, Benzhydryl-, Trityl-, Benzyloxymethyl-, Tosyl-, geradkettige oder verzweigte ($C_1$-$C_6$)-Alkylgruppe oder Phenylgruppe substituiert ist,
- eine (1-Azaindolizin-2-yl)-methylgruppe der Formel:

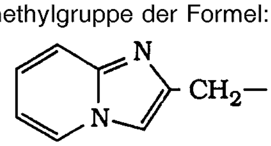

$R_2$ ein Wasserstoffatom oder eine geradkettige oder verzweigte ($C_1$-$C_6$)-Alkylgruppe,

R3 ein Wasserstoffatom oder eine geradkettige oder verzweigte ($C_1$-$C_6$)-Alkylgruppe,

<u>oder</u>

wenn $R_1$ ein Wasserstoffatom darstellt,

$R_2$ und $R_3$ gemeinsam mit den Kohlenstoff- und Stickstoffatomen, an die sie gebunden sind, einen der folgenden Heterocyclen bilden können:

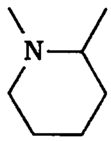 , 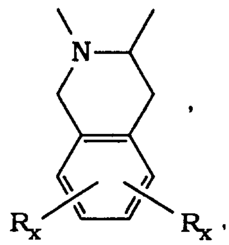 , 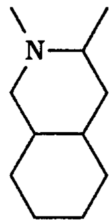 , 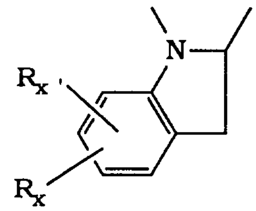 ,

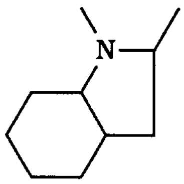 , 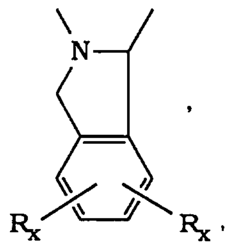 , 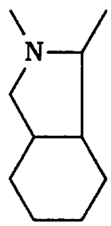 , 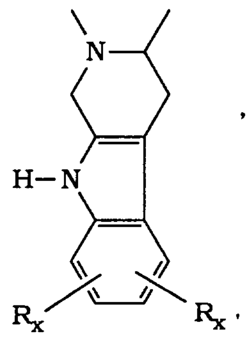 ,

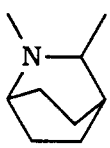 , 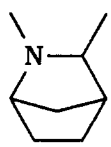 , 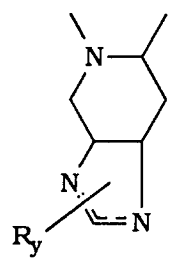

$R_x$ und $R_{x'}$, die gleichartig oder verschieden sein können, ein Wasserstoffatom, eine geradkettige oder verzweigte ($C_1$-$C_6$)-Alkylgruppe, eine Hydroxylgruppe, eine geradkettige oder verzweigte ($C_1$-$C_6$)-Alkoxygruppe oder ein Halogenatom,

oder $R_x$ und $R_{x'}$, wenn sie an zwei benachbarten Kohlenstoffatomen angeordnet sind, gemeinsam eine Methylendioxy- oder Ethylendioxy-gruppe bilden,

Ry ein Wasserstoffatom, eine geradkettige oder verzweigte ($C_1$-$C_6$)-Alkylgruppe, eine Arylgruppe, eine Aralkylgruppe, eine Aroylgruppe oder eine Arylsulfonylgruppe,

X eine der folgenden Gruppen:

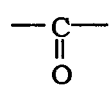 ,

$-SO_2-$ ,

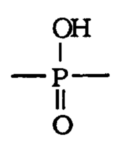 ,

Y eine Gruppe -CO-$R_5$ oder

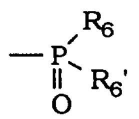

$R_5$ eine Hydroxylgruppe, eine geradkettige oder verzweigte $(C_1\text{-}C_6)$-Alkoxygruppe,$H_2N$-$CO$-$CH_2$-O-, HO-$CH_2$-CHOH-$CH_2$-O-,

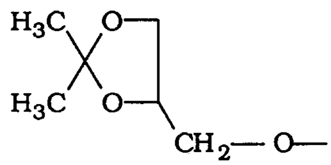
,
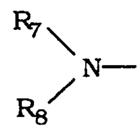

$R_7$ und $R_8$, die gleichartig oder verschieden sein können, ein Wasserstoffatom, eine geradkettige oder verzweigte $(C_1\text{-}C_6)$-Alkylgruppe oder gemeinsam mit dem Stickstoffatom, an das sie gebunden sind, einen Pyrrolidin-, Piperidin-, Morpholin- oder Piperazin-Ring bilden,

$R_6$ und $R_{6'}$, die gleichartig oder verschieden sein können, ein Wasserstoffatom, eine Hydroxylgruppe oder eine geradkettige oder verzweigte $(C_1\text{-}C_6)$-Alkoxygruppe,

$R_4$

1 eine geradkettige oder verzweigte Alkylgruppe mit 6 bis 21 Kohlenstoffatomen, die nicht substituiert oder an der endständigen Methylgruppe durch eine Hydroxyl-, Mercapto-, Phenyl- oder Ethinyl-gruppe substituiert ist und worin mindestens eine der Methylengruppen durch ein Sauerstoffatom, ein Schwefelatom oder einen p-Phenylenkern ersetzt ist,

dann, wenn:

$R_1$

- ein Wasserstoffatom,
- eine geradkettige oder verzweigte $(C_1\text{-}C_6)$-Alkylgruppe, die nicht substituiert oder durch eine oder mehrere Hydroxyl-, Amino-, Carboxy-, Carbamoyl-, Benzylthio-, Methylthio-, Mercapto- oder Phenyl-gruppen (die nichtsubstituiert oder durch eine Hydroxylgruppe oder eine geradkettige oder verzweigte $(C_1\text{-}C_6)$-Alkoxygruppe substituiert ist),
- eine nichtsubstituierte Phenylgruppe,
- eine (Imidazol-2-yl)-methylgruppe oder eine (Indol-3-yl)-methylgruppe, die nichtsubstituiert oder am Heterocyclus durch eine Methylgruppe substituiert ist, und

$R_2 = H$, $R_3 = H$,

X= 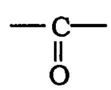 ,

$Y = COR_5'$ ($R_5' = OH$, Alkoxy)

2 und in den anderen Fällen:

eine geradkettige oder verzweigte Alkylgruppe mit 6 bis 21 Kohlenstoffatomen, die nichtsubstituiert oder an der endständigen Methylgruppe durch eine Hydroxyl-, Mercapto-, Phenyl- oder Ethinyl-gruppe substituiert ist und worin eine oder mehrere Methylengruppen durch ein Sauerstoffatom, ein Schwefelatom oder eine p-Phenylengruppe ersetzt sein können (mit der Maßgabe, daß, wenn $R_2$ oder $R_3$ mit den Stickstoff- oder Kohlenstoffatomen, an die sie gebunden sind, einen 1,2,3,4-Tetrahydroisochinolin-, Indolin- oder Isoindolin-Ring bilden, $R_4$ von der Gruppe Alk-S-$(CH_2)_n$- verschieden ist, worin n einen Wert von 1, 2, 3, 4 oder 5 und Alk eine geradkettige oder verzweigte $(C_4\text{-}C_{19})$-Alkylgruppe darstellen),

bedeuten,

**dadurch gekennzeichnet**, daß man:

1- dann, wenn die herzustellenden Derivate der Formel (I) eine Gruppe $R_4 = R_{4'}$ aufweisen, worin keine Methylengruppe durch ein Schwefelatom oder ein Sauerstoffatom ersetzt ist,
man eine Verbindung der Formel (II):

$R_{4'}$ - X'- Z (II)

in der:

X' eine Gruppe

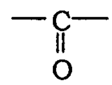
,

$-SO_2-$ ,

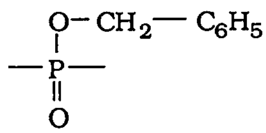
,

$R_{4'}$ eine geradkettige oder verzweigte Alkylgruppe mit 6 bis 21 Kohlenstoffatomen, die nichtsubstituiert oder an der endständigen Methylgruppe durch eine Hydroxyl-, Mercapto-, Phenyl- oder Ethinylgruppe substituiert ist, und worin eine oder mehrere Methylengruppen durch eine p-Phenylengruppe ersetzt sein können,

Z ein Halogenatom, eine Hydroxylgruppe, eine geradkettige oder verzweigte ($C_1$-$C_6$)-Alkoxygruppe, eine Aralkoxygruppe oder eine Gruppe

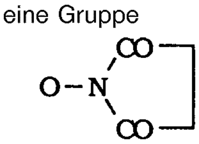

bedeuten,
mit einem gegebenenfalls geschützen (in Form des Racemats oder der Isomeren vorliegenden) Amin der Formel (III)

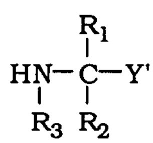
(III)

in der $R_2$, $R_3$ und $R_4$ die bezüglich der Formel I angegebenen Bedeutungen besitzen und Y' eine Gruppe der Formeln $-CO_2H$, -CO(Alkoxy) oder $-PO(Alkoxy)_2$ darstellen, kondensiert,
so daß man nach der eventuellen Abspaltung der Schutzgruppe eine Verbindung der Formel (I/a) erhält, einem Sonderfall der Verbindungen der Formel (I):

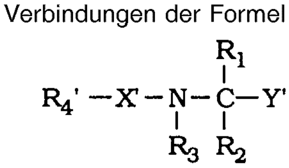
(I/a)

in der $R_1$, $R_2$, $R_3$, $R_{4'}$, X' und Y' die oben angegebenen Bedeutungen besitzen,
2- dann, wenn die herzustellenden Derivate der Formel (I) eine Gruppe $R_4 = R_{4''} = CH_3-(CH_2)_n-$, worin mindestens eine der Methylengruppen durch ein Schwefelatom oder ein Sauerstoffatom ersetzt ist, aufweisen,
man:
eine Verbindung der Formel (IV)

A - $(CH_2)_m$ - X'- Z (IV)

in der:

- X' und Z die oben angegebenen Bedeutungen besitzen,
- A ein Halogenatom, eine Mesyloxygruppe oder eine Tosyloxygruppe und
- m kleiner oder gleich n - 1 ist.

bedeuten,

und eine der Methylengruppen durch ein Sauerstoffatom, ein Schwefelatom, eine p-Phenylengruppe, eine Gruppe $-CH(CH_3)-$ oder eine Gruppe $-C(CH_3)_2$-ersetzt sein kann,

mit einem gegebenenfalls geschützten (in Form des Racemats oder der Isomeren vorliegenden) Amin der Formel (III), wie es oben definiert worden ist,

kondensiert zur Bildung einer Verbindung der Formel (V)

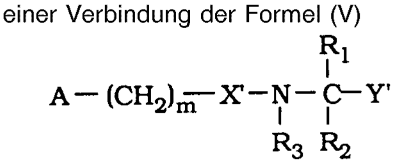

(V)

in der A, m, X', $R_1$, $R_2$, $R_3$ und Y' die oben angegebenen Bedeutungen besitzen, welche man mit einem Derivat der Formel (VI):

$R_9$ - B - M (VI)

in der:

- $R_9$ eine Gruppe der Formel $CH_3$-$(CH_2)_p$-, die nichtsubstituiert oder an der endständigen Methylgruppe durch eine Hydroxyl-, Mercapto-, Phenyl- oder Ethinylgruppe substituiert ist und bei der eine der Methylengruppen durch ein Sauerstoffatom, ein Schwefelatom, eine p-Phenylengruppe, eine Gruppe $-CH(CH_3)-$ oder eine Gruppe $-C(CH_3)_2-$ ersetzt sein kann,
- B ein Sauerstoffatom oder ein Schwefelatom,
- M ein aus Natrium, Kalium oder Caesium ausgewähltes Metall und
- p einen solchen Wert besitzt, daß m + p kleiner oder gleich n - 1 ist,

bedeuten,

umsetzt, so daß man nach der eventuellen Abspaltung der Schutzgruppe eine Verbindung der Formel (I/b) erhält, einem Sonderfall der Verbindungen der Formel (I):

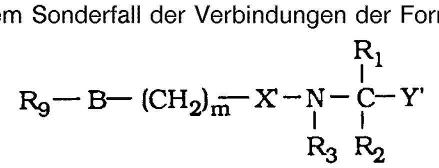

(I/b)

in der $R_9$, B, m, X', $R_1$, $R_2$, $R_3$ und Y' die oben angegebenen Bedeutungen besitzen,

welche Derivate der Formeln (I/a) und (I/b) man auch vereinfacht wie folgt schreiben kann:

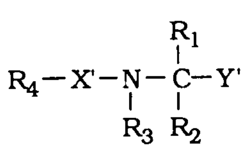

welche

a dann, wenn X' eine Gruppe der Formel

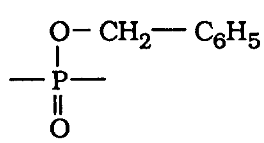

darstellt, durch katalytische Hydrierung in die Verbindungen der Formel (I/c) umgewandelt werden können, einem Sonderfall der Verbindungen der Formel (I):

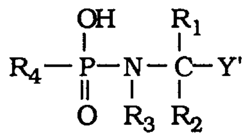

(I/c)

in der $R_1$, $R_2$, $R_3$, $R_4$ und Y' die oben angegebenen Bedeutungen besitzen,

b dann, wenn Y' eine Gruppe -CO(Alkoxy) oder -PO(Alkoxy)$_2$ darstellt, vollständig oder teilweise verseift werden können, so daß mandie Verbindungen der Formeln (I/d), (I/e) bzw. (I/f) erhält, Sonderfälle der Verbindungen der Formel (I):

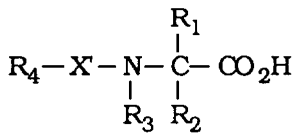

(I/d)

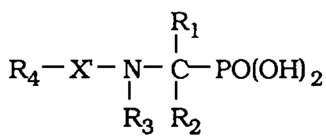

(I/e)

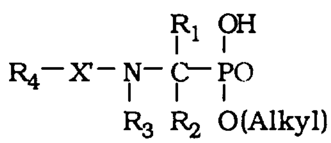

(I/f)

worin $R_1$, $R_2$, $R_3$, $R_4$ und X' die oben angegebenen Bedeutungen besitzen,

c dann wenn Y' eine Gruppe -$CO_2$H darstellt, in Gegenwart von Caesiumcarbonat mit Hilfe von Chloracetamid in die Verbindung der Formel (I/g) umgewandelt werden können, einem Sonderfall der Verbindungen der Formel (I):

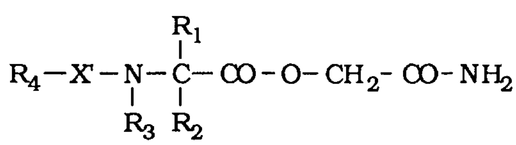

(I/g)

in der $R_1$, $R_2$, $R_3$, $R_4$ und X' die oben angegebenen Bedeutungen besitzen,

d dann, wenn Y' eine Gruppe -$CO_2$H darstellt,

mit Hilfe von Isopropylidenglycerol zu einer Verbindung der Formel (I/h) verestert werden können, einem Sonderfall der Verbindungen der Formel (I):

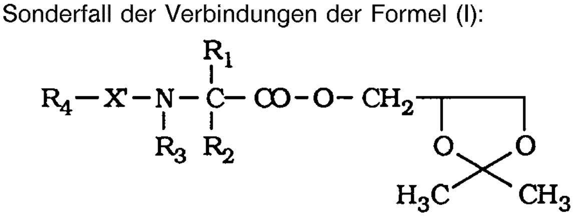

(I/h)

in der $R_1$, $R_2$, $R_3$ und $R_4$ die oben angegebenen Bedeutungen besitzen, welches Derivat der Formel (I/h) in saurem Medium hydrolysiert werden kann zur Bildung der Verbindung der Formel (I/i), einem Sonderfall der Verbindungen der Formel (I):

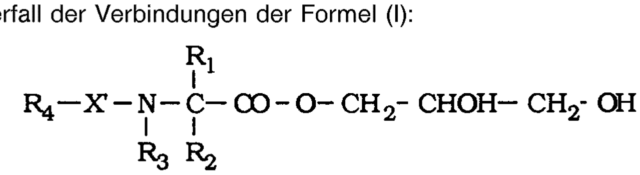

(I/i)

in der $R_1$, $R_2$, $R_3$ und $R_4$ die oben angegebenen Bedeutungen besitzen,
welche Verbindungen der Formeln (I/a] bis (I/i) gegebenenfalls mit Hilfe einer klassischen Reinigungsmethode gereinigt werden können, dessen Isomeren mit Hilfe einer klassischen Trennmethode getrennt werden können und welche man mit einer pharmazeutisch annehmbaren Säure oder Base in ihre Säureadditionssalze umwandeln kann.

2. Verfahren nach Anspruch 1 zur Herstellung von N-Myristoyl-3-carboxy-(1,2,3,4)-tetrahydroisochinolin, von dessen Enantiomeren sowie von dessen Additionssalzen mit einer pharmazeutisch annehmbaren Base.

3. Verfahren nach Anspruch 1 zur Herstellung von N-Myristoyl-(N$^{'\tau}$-benzyl)histin, von dessen Enantiomeren sowie von dessen Additionssalzen mit einer pharmazeutisch annehmbaren Säure oder Base.

4. Verfahren nach Anspruch 1 zur Herstellung von 1-Myristoylamino-2-phenylethanhydrogenphosphonsäureethylester, von dessen Enantiomeren sowie von dessen Additionssalzen mit einer pharmazeutisch annehmbaren Base.

5. Verfahren nach Anspruch 1 zur Herstellung von N-(12-Methoxylauroyl)-phenylalanin, von dessen Enantiomeren sowie von dessen Additionssalzen mit einer pharmazeutisch annehmbaren Base.

**Patentansprüche für folgenden Vertragsstaat : GR**

1. Verfahren zur Herstellung der Verbindungen der allgemeinen Formel (I)

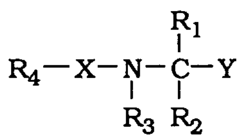

(I)

in der
$R_1$

- ein Wasserstoffatom,
- eine geradkettige oderverzweigte ($C_1$-$C_6$)-Alkylgruppe, die nichtsubstituiert oder durch eine oder mehrere Hydroxyl-, Amino-, Carboxy-, Carbamoyl-, Benzylthio-, Methylthio-, Mercapto- oder Phenyl-gruppen (welche nichtsubstituiert oder durch eines oder mehrere Halogenatome oder Hydroxylgruppen, geradkettige oder verzweigte ($C_1$-$C_6$)-Alkylgruppen, geradkettige oder verzweigte ($C_1$-$C_6$)-Alkoxygruppen oder ($(CH_3$-$CH_2$-$O)_2$-PO-$CH_2$-) substituiert ist)`
- eine Phenylgruppe. die nichtsubstituiert oder durch eines oder mehrere Halogenatome oder Hydroxylgruppen oder geradkettige oder verzweigte ($C_1$-$C_6$)-Alkylgruppen substituiert ist,
- eine ($C_3$-$C_7$)-Cycloalkyl-methylgruppe,
- eine (Imidazol-2-yl)-methylgruppe oder eine (Indol-3-yl)-methylgruppe, die nichtsubstituiert oder am Heterocyclus durch eine Benzyl-, Benzhydryl-, Trityl-, Benzyloxymethyl-, Tosyl-, geradkettige oder verzweigte ($C_1$-$C_6$)-Alkylgruppe oder Phenylgruppe substituiert ist,
- eine (1-Azaindolizin-2-yl)-methylgruppe der Formel:

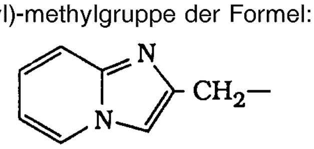

$R_2$ ein Wasserstoffatom oder eine geradkettige oder verzweigte ($C_1$-$C_6$)-Alkylgruppe,
$R_3$ ein Wasserstoffatom oder eine geradkettige oder verzweigte ($C_1$-$C_6$)-Alkylgruppe,
oder
wenn $R_1$ ein Wasserstoffatom darstellt,

$R_2$ und $R_3$ gemeinsam mit den Kohlenstoff- und Stickstoffatomen, an die sie gebunden sind, einen der folgenden Heterocyclen bilden können:

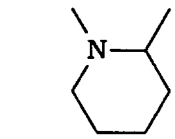 , 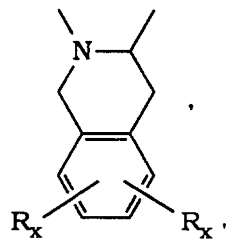 , 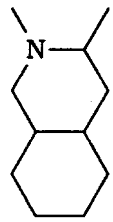 , 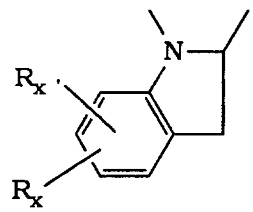 ,

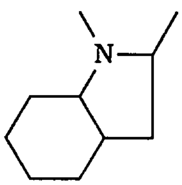 , 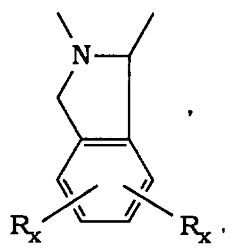 , 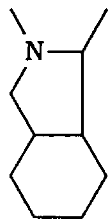 , 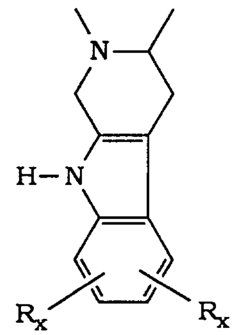 ,

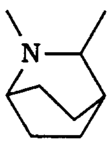 , 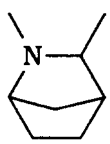 , 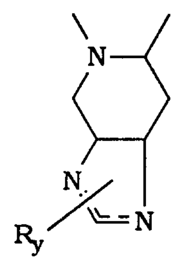

$R_x$ und $R_{x'}$, die gleichartig oder verschieden sein können. ein Wasserstoffatom, eine geradkettige oder verzweigte ($C_1$-$C_6$)-Alkylgruppe, eine Hydroxylgruppe, eine geradkettige oder verzweigte ($C_1$-$C_6$)-Alkoxygruppe oder ein Halogenatom,

oder $R_x$ und $R_{x'}$, wenn sie an zwei benachbarten Kohlenstoffatomen angeordnet sind, gemeinsam eine Methylendioxy- oder Ethylendioxy-gruppe bilden,

Ry ein Wasserstoffatom eine geradkettige oder verzweigte ($C_1$-$C_6$)-Alkylgruppe, eine Arylgruppe, eine Aralkylgruppe, eine Aroylgruppe oder eine Arylsulfonylgruppe,

X eine der folgenden Gruppen:

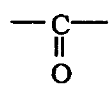 ,

$-SO_2-$ ,

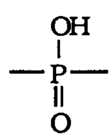 ,

Y eine Gruppe - CO-$R_5$ oder

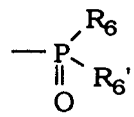

$R_5$ eine Hydroxylgruppe, eine geradkettige oder verzweigte ($C_1$-$C_6$)-Alkoxygruppe,$H_2N$-$CO$-$CH_2$-O-, HO-$CH_2$-CHOH-$CH_2$-O-,

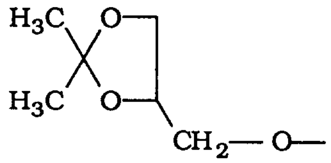 , 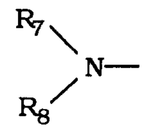

$R_7$ und $R_8$, die gleichartig oder verschieden sein können, ein Wasserstoffatom, eine geradkettige oder verzweigte ($C_1$-$C_6$)-Alkylgruppe oder gemeinsam mit dem Stickstoffatom, an das sie gebunden sind, einen Pyrrolidin-, Piperidin-, Morpholin- oder Piperazin-Ring bilden,

$R_6$ und $R_{6'}$ , die gleichartig oder verschieden sein können, ein Wasserstoffatom, eine Hydroxylgruppe oder eine geradkettige oder verzweigte ($C_1$-$C_6$)-Alkoxygruppe,

$R_4$

1 eine geradkettige oder verzweigte Alkylgruppe mit 6 bis 21 Kohlenstoffatomen, die nicht substituiert oder an der endständigen Methylgruppe durch eine Hydroxyl-, Mercapto-, Phenyl- oder Ethinyl-gruppe substituiert ist und worin mindestens eine der Methylengruppen durch ein Sauerstoffatom, ein Schwefelatom oder einen p-Phenylenkern ersetzt ist,

dann, wenn:

$R_1$

- ein Wasserstoffatom,
- eine geradkettige oder verzweigte ($C_1$-$C_6$)-Alkylgruppe, die nicht substituiert oder durch eine oder mehrere Hydroxyl-, Amino-, Carboxy-, Carbamoyl-, Benzylthio-, Methylthio-, Mercapto- oder Phenyl-gruppen (die nichtsubstituiert oder durch eine Hydroxylgruppe oder eine geradkettige oder verzweigte ($C_1$-$C_6$)-Alkoxygruppe substituiert ist),
- eine nichtsubstituierte Phenylgruppe,
- eine (Imidazol-2-yl)-methylgruppe oder eine (Indol-3-yl)-methylgruppe, die nichtsubstituiert oder am Heterocyclus durch eine Methylgruppe substituiert ist, und

$R_2 = H$, $R_3 = H$,

X= 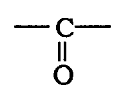 ,

Y = $COR_5'$ ($R_5'$ = OH, Alkoxy)

2 und in den anderen Fällen:

eine geradkettige oder verzweigte Alkylgruppe mit 6 bis 21 Kohlenstoffatomen, die nichtsubstituiert oder an der endständigen Methylgruppe durch eine Hydroxyl-, Mercapto-, Phenyl- oder Ethinyl-gruppe substituiert ist und worin eine oder mehrere Methylengruppen durch ein Sauerstoffatom, ein Schwefelatom oder eine p-Phenylengruppe ersetzt sein können (mit der Maßgabe, daß, wenn $R_2$ oder $R_3$ mit den Stickstoff- oder Kohlenstoffatomen, an die sie gebunden sind, einen 1,2,3,4-Tetrahydroisochinolin-, Indolin- oder Isoindolin-Ring bilden, $R_4$ von der Gruppe Alk-S-$(CH_2)_n$- verschieden ist, worin n einen Wert von 1,2,3,4 oder 5 und Alk eine geradkettige oder verzweigte ($C_4$-$C_{19}$)-Alkylgruppe darstellen),

bedeuten,

**dadurch gekennzeichnet**, daß man:

1- dann, wenn die herzustellenden Derivate der Formel (I) eine Gruppe $R_4 = R_{4'}$ aufweisen, worin keine Methylengruppe durch ein Schwefelatom oder ein Sauerstoffatom ersetzt ist.
man eine Verbindung der Formel (II):

$R_{4'}$ - X' - Z (II)

in der:

X' eine Gruppe

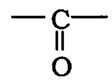 ,

$-SO_2-$ ,

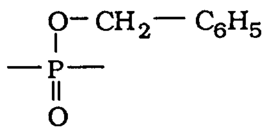 ,

$R_{4'}$ eine geradkettige oder verzweigte Alkylgruppe mit 6 bis 21 Kohlenstoffatomen, die nichtsubstituiert oder an der endständigen Methylgruppe durch eine Hydroxyl-, Mercapto-, Phenyl- oder Ethinylgruppe substituiert ist, und worin eine oder mehrere Methylengruppen durch eine p-Phenylengruppe ersetzt sein können,

Z ein Halogenatom, eine Hydroxylgruppe, eine geradkettige oder verzweigte ($C_1$-$C_6$)-Alkoxygruppe, eine Aralkoxygruppe oder eine Gruppe

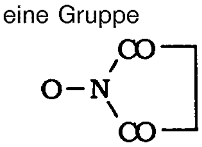

bedeuten,

mit einem gegebenenfalls geschützen (in Form des Racemats oder der Isomeren vorliegenden) Amin der Formel (III)

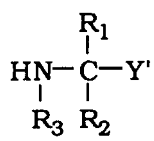 (III)

in der $R_2$, $R_3$ und $R_4$ die bezüglich der Formel I angegebenen Bedeutungen besitzen und Y' eine Gruppe der Formeln $-CO_2H$, -CO(Alkoxy) oder $-PO(Alkoxy)_2$ darstellen, kondensiert,
so daß man nach der eventuellen Abspaltung der Schutzgruppe eine Verbindung der Formel (I/a) erhält, einem Sonderfall der Verbindungen der Formel (I):

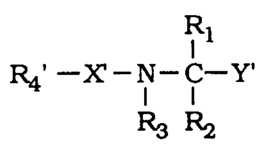 (I/a)

in der $R_1$, $R_2$, $R_3$, $R_{4'}$, X' und Y' die oben angegebenen Bedeutungen besitzen,
2- dann, wenn die herzustellenden Derivate der Formel (I) eine Gruppe $R_4 = R_{4''} = CH_3-(CH_2)_n-$, worin mindestens eine der Methylengruppen durch ein Schwefelatom oder ein Sauerstoffatom ersetzt ist, aufweisen,
man:
eine Verbindung der Formel (IV)

A - $(CH_2)_m$ - X' - Z (IV)

in der:

- X' und Z die oben angegebenen Bedeutungen besitzen,
- A ein Halogenatom, eine Mesyloxygruppe oder eine Tosyloxygruppe und
- m kleiner oder gleich n - 1 ist,

bedeuten,
und eine der Methylengruppen durch ein Sauerstoffatom, ein Schwefelatom, eine p-Phenylengruppe, eine Gruppe $-CH(CH_3)-$ oder eine Gruppe $-C(CH_3)_2-$ersetzt sein kann,
mit einem gegebenenfalls geschützten (in Form des Racemats oder der Isomeren vorliegenden) Amin der Formel (III), wie es oben definiert worden ist,
kondensiert zur Bildung einer Verbindung der Formel (V)

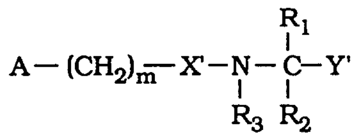

(V)

in der A, m, X', $R_1$, $R_2$, $R_3$ und Y' die oben angegebenen Bedeutungen besitzen, welche man mit einem Derivat der Formel (VI):

$R_9$ - B - M (VI)

in der:

- $R_9$ eine Gruppe der Formel $CH_3-(CH_2)_p-$, die nichtsubstituiert oder an der endständigen Methylgruppe durch eine Hydroxyl-, Mercapto-, Phenyl- oder Ethinylgruppe substituiert ist und bei der eine der Methylengruppen durch ein Sauerstoffatom, ein Schwefelatom, eine p-Phenylengruppe, eine Gruppe $-CH(CH_3)-$ oder eine Gruppe $-C(CH_3)_2-$ ersetzt sein kann,
- B ein Sauerstoffatom oder ein Schwefelatom,
- M ein aus Natrium, Kalium oder Caesium ausgewähltes Metall und
- p einen solchen Wert besitzt, daß m + p kleiner oder gleich n - 1 ist,

bedeuten,
umsetzt, so daß man nach der eventuellen Abspaltung der Schutzgruppe eine Verbindung der Formel (I/b) erhält, einem Sonderfall der Verbindungen der Formel (I):

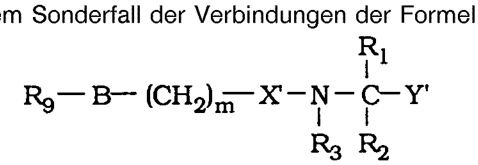

(I/b)

in der $R_9$, B, m, X', $R_1$, $R_2$, $R_3$und Y' die oben angegebenen Bedeutungen besitzen,
welche Derivate der Formeln (I/a) und (I/b) man auch vereinfacht wie folgt schreiben kann:

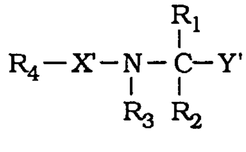

welche man
a dann, wenn X' eine Gruppe der Formel

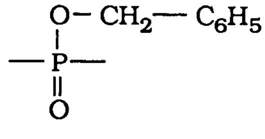

darstellt, durch katalytische Hydrierung in die Verbindungen der Formel (I/c) umgewandelt werden können, einem Sonderfall der Verbindungen der Formel (I):

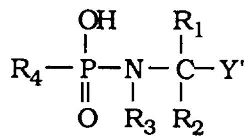

(I/c)

in der $R_1$, $R_2$, $R_3$, $R_4$ und Y' die oben angegebenen Bedeutungen besitzen,

b dann wenn Y' eine Gruppe -CO(Alkoxy) oder $-PO(Alkoxy)_2$ darstellt, vollständig oder teilweise verseift werden können, so daß mandie Verbindungen der Formeln (I/d), (I/e) bzw. (I/f) erthält, Sonderfälle der Verbindungen der Formel (I):

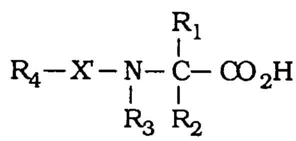

(I/d)

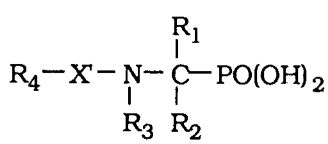

(I/e)

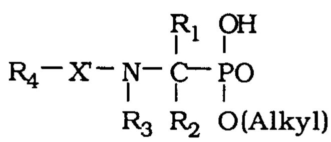

(I/f)

worin $R_1$, $R_2$, $R_3$, $R_4$ und X' die oben angegebenen Bedeutungen besitzen,

c dann, wenn Y' eine Gruppe $-CO_2H$ darstellt, in Gegenwart von Caesiumcarbonat mit Hilfe von Chloracetamid in die Verbindung der Formel (I/g) umgewandelt werden können, einem Sonderfall der Verbindungen der Formel (I):

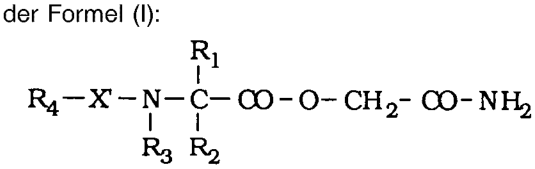

(I/g)

in der $R_1$, $R_2$, $R_3$, $R_4$ und X' die oben angegebenen Bedeutungen besitzen,

d dann, wenn Y' eine Gruppe $-CO_2H$ darstellt,

mit Hilfe von Isopropylidenglycerol zu einer Verbindung der Formel (I/h) verestert werden können, einem Sonderfall der Verbindungen der Formel (I):

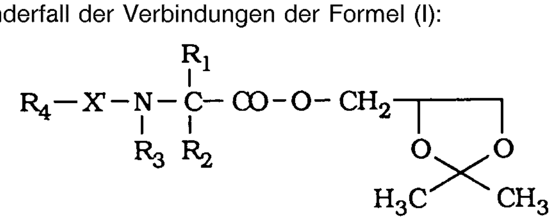

(I/h)

In der $R_1$, $R_2$, $R_3$ und $R_4$ die oben angegebenen Bedeutungen besitzen, welches Derivat der Formel (I/h) in saurem Medium hydrolysiert werden kann zur Bildung der Verbindung der Formel (I/i), einem Sonderfall der Verbindungen der Formel (I):

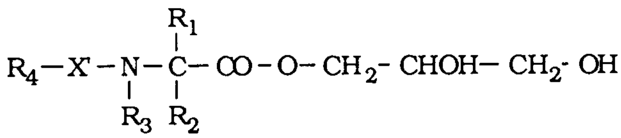 (I/i)

in der $R_1$, $R_2$, $R_3$ und $R_4$ die oben angegebenen Bedeutungen besitzen,
welche Verbindungen der Formeln (I/a) bis (I/i) gegebenenfalls mit Hilfe einer klassischen Reinigungsmethode gereinigt werden können, dessen Isomeren mit Hilfe einer klassischen Trennmethode getrennt werden können und welche man mit einer pharmazeutisch annehmbaren Säure oder Base in ihre Säureadditionssalze umwandeln kann.

**2.** Verfahren nach Anspruch 1 zur Herstellung von N-Myristoyl-3-carboxy-(1,2,3,4)-tetrahydroisochinolin, von dessen Enantiomeren sowie von dessen Additionssalzen mit einer pharmazeutisch annehmbaren Base.

**3.** Verfahren nach Anspruch 1 zur Herstellung von N-Myristoyl-($N'^{\tau}$-benzyl)-histin, von dessen Enantiomeren sowie von dessen Additionssalzen mit einer pharmazeutisch annehmbaren Säure oder Base.

**4.** Verfahren nach Anspruch 1 zur Herstellung von 1-Myristoylamino-2-phenylethanhydrogenphosphonsäureethylester, von dessen Enantiomeren sowie von dessen Additionssalzen mit einer pharmazeutisch annehmbaren Base.

**5.** Verfahren nach Anspruch 1 zur Herstellung von N-(12-Methoxylauroyl)-phenylalanin, von dessen Enantiomeren sowie von dessen Additionssalzen mit einer pharmazeutisch annehmbaren Base.